# EUROPÄISCHE PATENTSCHRIFT

(11) **EP 2 435 420 B1**
(45) Veröffentlichungstag und Bekanntmachung des Hinweises auf die Patenterteilung: **03.04.2013**
(21) Anmeldenummer: 10721330.8
(22) Anmeldetag: 19.05.2010
(51) Int. Cl.: C07D 401/14, C07D 403/04, C07D 405/14, A01N 43/60, A61K 31/497, A61P 33/00

(54) **Pyrazinylpyrazole**
Pyrazinylpyrazoles
Pyrazinylpyrazoles

(30) Priorität: 29.05.2009 EP 09161568
(43) Veröffentlichungstag der Anmeldung: 04.04.2012
(73) Patentinhaber: Bayer CropScience AG, 40789 Monheim (DE)
(72) Erfinder: SCHWARZ, Hans-Georg, 46282 Dorsten (DE); FRACKENPOHL, Jens, 60322 Frankfurt (DE); HENSE, Achim, 51379 Leverkusen (DE); MAECHLING, Simon, 50674 Köln (DE); SCHNATTERER, Stefan, 65795 Hattersheim (DE); VELTEN, Robert, 40764 Langenfeld (DE); WERNER, Stefan, 40789 Monheim (DE); BECKER, Angela, 40235 Düsseldorf (DE); MALSAM, Olga, 51503 Rösrath (DE); FRANKEN, Eva-Maria, 51381 Leverkusen (DE); VOERSTE, Arnd, 50674 Köln (DE); GÖRGENS, Ulrich, 40882 Ratingen (DE); LÜMMEN, Peter, 65510 Idstein (DE)
(86) Internationale Anmeldenummer: PCT/EP2010/003060
(87) Internationale Veröffentlichungsnummer: WO 2010/136145

(56) Entgegenhaltungen:
- WO-A-01/07413
- WO-A-2007/048733
- WO-A-2008/077483
- DE-A1- 19 544 799

## Beschreibung

Die vorliegende Erfindung betrifft Pyrazin-2-yl-pyrazole und ihre Verwendung als Insektizide und/oder Parasitizide.

Weiterer Gegenstand der vorliegenden Erfindung sind Verfahren zu ihrer Herstellung und Mittel, die solche Pyrazin-2-yl-pyrazole enthalten.

Die WO 2007/048733 A beschreibt die Verwendung von Aminopyrazolen zur Bekämpfung pflanzenpathogener Schadpilze, wobei generisch auch Pyrazin-2-yl-pyrazole umfasst werden. Die Pyrazin-2-yl-pyrazole tragen in 3-Position nur Wasserstoff als Substituent.

Die WO 2007/027842 A offenbart Anilinopyrazole, die in 1-Position der Pyrazol-Einheit durch 2-Pyrazine substituiert sein können. Diese internationale Anmeldung bezieht sich auf pharmazeutische Anwendungen, insbesondere auf die Behandlung von Diabetes; eine arthropodizide Wirkung wird nicht beschrieben.

Die gemäß den oben genannten Schriften bereits bekannten Wirkstoffe weisen bei ihrer Anwendung Nachteile auf, insbesondere dass sie keine oder aber eine nur unzureichende insektizide Wirkung besitzen. Daher besteht Bedarf nach weiteren Insektiziden und/oder Parasitiziden. Insektizide Pyrimiding epyrazole sind in WO 2008/077483 A bekannt.

Aufgabe der vorliegenden Erfindung ist somit die Bereitstellung von alternativen Insektiziden und/oder Parasitiziden, die gegenüber den aus dem Stand der Technik bekannten Wirkstoffen eine verbesserte Wirkung bzw. ein verbreitertes Wirkspektrum zeigen.

Gelöst wird diese Aufgabe durch Pyrazinylpyrazole der allgemeinen Formel (I) 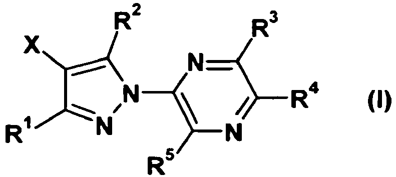 in welcher
- X: für Phenyl, 2-Pyridyl oder 3-Pyridyl steht, welche jeweils substituiert sind durch einen oder mehrere Substituenten, ausgewählt aus der Gruppe, bestehend aus Halogen, Alkyl, Haloalkyl, Alkoxy, Alkoxyalkyl, Alkoxyalkoxy, Cycloalkyl, Alkenyloxy, Alkinyloxy, Benzyloxy, Cycloalkylalkoxy, Haloalkoxy, Haloalkoxyalkyl, Alkylsulfanyl, Haloalkylsulfanyl, Alkylsulfinyl, Haloalkylsulfinyl, Alkylsulfonyl, Haloalkylsulfonyl, Cyano, Nitro, Alkylcarbonyl, Alkoxycarbonyl, Alkoxycarbonylalkyl, Carboxyl, Carboxamid, Dialkylcarboxamid, Trialkylsilyl, Amino, Alkylamino, Dialkylamino, Alkylsulfonylamino, Dialkylsulfonylamino, Formyl, -CH=NO-H, -CH=NO-Alkyl, -CH=NO-Halolkyl, -C(CH₃)=NO-H, -C(CH₃)=NO-Alkyl, -C(CH₃)=NO-Haloalkyl; oder für gegebenenfalls mit einem oder mehreren Halogenatomen, Cyano, Nitro, Alkyl, Alkoxy oder Haloalkyl substituiertes Phenyl, 2-Pyridyl und 3-Pryidyl steht, wobei vicinale Alkyl-, Haloalkyl-, Alkoxy- und/oder Haloalkoxygruppen an dem Phenylsubstituenten, 2-Pyridylsubstituenten oder 3-Pyridylsubstituenten zusammen mit den Kohlenstoffatomen, an die sie gebunden sind, ein fünf- bis siebengliedriges cyclisches System bilden können, das 0 bis 2 Sauerstoff- oder Stickstoffatome enthält, wobei zwei Sauerstoffatome nicht direkt miteinander verbunden sind, und dessen Alkylanteil gegebenenfalls mit einem oder mehreren Halogenatomen und/oder weiteren Alkylresten substituiert sein kann;
- R¹: für Wasserstoff, Alkyl, welches gegebenenfalls einfach oder unabhängig voneinander mehrfach mit Alkoxy, Haloalkoxy, Alkylsulfanyl, Haloalkylsulfanyl, Alkylsulfinyl, Haloalkylsulfinyl, Alkylsulfonyl, Haloalkylsulfonyl, Alkylcarbonyl, Alkoxycarbonyl, Hydroxy und/oder Cycloalkyl substituiert ist; Alkenyl, welches gegebenenfalls einfach oder unabhängig voneinander mehrfach mit Halogen, Alkoxy, Haloalkoxy, Alkylsulfanyl, Haloalkylsulfanyl, Alkylsulfinyl, Haloalkylsulfinyl, Alkylsulfonyl, Haloalkylsulfonyl, Alkylcarbonyl, Alkoxycarbonyl, und/oder Cycloalkyl substituiert ist; Cycloalkyl, welches gegebenenfalls einfach oder unabhängig voneinander mehrfach mit Alkyl, Haloalkyl und/oder Halogen substituiert ist; Haloalkyl, welches gegebenenfalls einfach oder unabhängig voneinander mehrfach mit Alkoxy, Alkylsulfanyl, Haloalkylsulfanyl, Alkylsulfinyl, Haloalkylsulfinyl, Alkylsulfonyl, Haloalkylsulfonyl und/oder Phenyl, gegebenenfalls einfach oder unabhängig voneinander mehrfach mit Halogen, Alkyl, Haloalkyl und/oder Alkoxy substituiert, substituiert ist; Phenyl, welches gegebenenfalls einfach oder unabhängig voneinander mehrfach mit Halogen, Alkyl, Haloalkyl und/oder Alkoxy substituiert ist; Benzyl, welches gegebenenfalls einfach oder unabhängig voneinander mehrfach mit Halogen, Alkyl, Haloalkyl und/oder Alkoxy substituiert ist; Cyano, Formyl, Alkylcarbonyl, -CH=NO-H, - CH=NO-Alkyl, -CH=NO-Haloalkyl, -C(CH₃)=NO-H, -C(CH₃)=NO-Alkyl oder -C(CH₃)=NO-Haloalkyl; steht;
- R²: für gegebenenfalls substituiertes Amino steht, wobei Amino einfach oder unabhängig voneinander zweifach substituiert sein kann mit Alkyl, Haloalkyl, Alkoxyalkyl, Alkylsulfanylalkyl, Alkylsulfinylalkyl, Alkylsulfonylalkyl, Alkylcarbonyl, Cycloalkyl, Cycloalkylalkyl, Alkenyl, wobei die vorstehenden Reste gegebenenfalls substituiert sind durch Halogen, Cyano, Alkoxy, Alkoxycarbonyl oder Phenyl, wobei der Phenylring gegebenenfalls einfach oder mehrfach substituiert ist mit einem oder mehreren Substituenten unabhängig voneinander ausgewählt aus Halogen, Alkyl, Haloalkyl und Alkoxy; Alkinyl, Alkoxycarbonyl, Alkenyloxycarbonyl, Alkinyloxycarbonyl, Alkoxycarbonylalkyl, Alkoxycarbonylcarbonyl, Heterocyclyl, Heteroaryl, Heterocyclylalkyl oder Heteroarylalkyl, wobei der heterocyclische oder heteroaromatische Ring gegebenenfalls einfach oder mehrfach substituiert sein kann mit einem oder mehreren Substituenten unabhängig voneinander ausgewählt aus Halogen, Alkyl, Haloalkyl und Alkoxy; Benzyl oder Phenylcarbonyl, wobei der Phenylring im Benzyl und Phenylcarbonyl gegebenenfalls einfach oder mehrfach substituiert ist mit einem oder mehreren Substituenten unabhängig voneinander ausgewählt aus Halogen, Alkyl, Haloalkyl und Alkoxy; und
- R³, R⁴: unabhängig voneinander für Wasserstoff, Halogen, Alkyl, Cycloalkyl, Haloalkyl, Cyano, Hydroxy, Formyl, Alkylcarbonyl, -CH=NO-H, -CH=NO-Alkyl, -CH=NO-Haloalkyl, - C(CH₃)=NO-H, -C(CH₃)=NO-Alkyl, -C(CH₃)=NO-Haloalkyl, Nitro, Hydroxy, SH, Alkoxy, Alkylsulfanyl, Haloalkylsulfanyl, Alkylsulfinyl, Haloalkylsulfinyl, Alkylsulfonyl oder Haloalkylsulfonyl stehen;
- R⁵: für Halogen, Alkyl, Haloalkyl, Hydroxy, Alkoxy, Alkoxyalkyl, Alkoxyalkoxy, Cycloalkyl, Alkenyloxy, Alkinyloxy, Benzyloxy, Cycloalkylalkoxy, Haloalkoxy, Haloalkoxyalkyl, - SH, Alkylsulfanyl, Haloalkylsulfanyl, Alkylsulfinyl, Haloalkylsulfinyl, Alkylsulfonyl, Haloalkylsulfonyl, Cyano, Nitro, Alkylcarbonyl, Alkoxycarbonyl, Alkoxycarbonylalkyl, Carboxyl, Carboxamid, Dialkylcarboxamid, Trialkylsilyl, Nitro, Amino, Alkylamino, Dialkylamino, Alkylsulfonylamino, Dialkylsulfonylamino, Formyl, -CH=NO-H, -CH=NO-Alkyl, -CH=NO-Halolkyl, -C(CH₃)=NO-H, -C(CH₃)=NO-Alkyl, -C(CH₃)=NO-Haloalkyl oder Heteroaryl, wobei der heteroaromatische Ring gegebenenfalls einfach oder mehrfach substituiert sein kann mit einem oder mehreren Substituenten unabhängig voneinander ausgewählt aus Halogen, Alkyl, Haloalkyl und Alkoxy; steht;
sowie die N-Oxide und Salze der Verbindungen der allgemeinen Formel (I).

Erfindungsgemäß wurde gefunden, dass die Verbindungen der allgemeinen Formel (I) sowie deren N-Oxide und Salze gute insektizide und parasitizide Eigenschaften besitzen und sich im Pflanzenschutz, im Bereich Tiergesundheit und im Materialschutz, insbesondere zum Schutz technischer Materialien zur Bekämpfung unerwünschter Schädlinge, wie Insekten, Spinnmilben, Endo- oder Ektoparasiten, verwenden lassen.

Im Folgenden werden bevorzugte Ausführungsformen der Verbindungen der allgemeinen Formel (I) beschrieben:

In einer ersten Ausführungsform der vorliegenden Erfindung sind Verbindungen der allgemeinen Formel (I)
(a) bevorzugt, in welchen der Rest X für Phenyl, 2-Pyridyl oder 3-Pyridyl steht, welche jeweils substituiert sind durch einen oder mehrere Substituenten, ausgewählt aus der Gruppe, bestehend aus Halogen, Alkyl, Haloalkyl, Alkoxy, Haloalkoxy, Alkylsulfanyl, Haloalkylsulfanyl, Alkylsulfinyl, Haloalkylsulfinyl, Alkylsulfonyl, Haloalkylsulfonyl, Cyano, Nitro und Dialkylamino; oder für gegebenenfalls mit einem oder mehreren Halogenatomen, Cyano, Nitro, Alkyl, Alkoxy oder Haloalkyl substituiertes Phenyl, 2-Pyridyl und 3-Pyridyl steht, wobei vicinale Alkyl-, Haloalkyl- und/oder Alkoxygruppen an dem Phenylsubstituenten, 2-Pyridylsubstituenten oder 3-Pyridylsubstituenten zusammen mit den Kohlenstoffatomen, an die sie gebunden sind, ein fünf- bis siebengliedriges cyclisches System bilden können, das 0 bis 2 Sauerstoff- oder Stickstoffatome enthält, wobei zwei Sauerstoffatome nicht direkt miteinander verbunden sind, und dessen Alkylanteil gegebenenfalls mit einem oder mehreren Halogenatomen und/oder weiteren Alkylresten substituiert sein kann;
(b) weiter bevorzugt, in welchen der Rest X für Phenyl, 2-Pyridyl oder 3-Pyridyl steht, welche jeweils substituiert sind durch einen oder mehrere Substituenten, ausgewählt aus der Gruppe, bestehend aus Halogen, Alkyl, Haloalkyl, Alkoxy, Haloalkoxy, Alkylsulfanyl, Haloalkylsulfanyl, Alkylsulfinyl, Haloalkylsulfinyl, Alkylsulfonyl, Haloalkylsulfonyl, Cyano und Dialkylamino; oder für gegebenenfalls mit einem oder mehreren Halogenatomen, Cyano, Nitro, Alkyl, Alkoxy oder Haloalkyl substituiertes Phenyl, 2-Pyridyl und 3-Pyridyl steht, wobei vicinale Alkyl-, Haloalkyl- und/oder Alkoxygruppen an dem Phenylsubstituenten, 2-Pyridylsubstituenten oder 3-Pyridylsubstituenten zusammen mit den Kohlenstoffatomen, an die sie gebunden sind, ein fünf- bis siebengliedriges cyclisches System bilden können, das 1 oder 2 Sauerstoffatome enthält, wobei zwei Sauerstoffatome nicht direkt miteinander verbunden sind, und dessen Alkylanteil gegebenenfalls mit einem oder mehreren Halogenatomen und/oder weiteren Alkylresten substituiert sein kann;
(c) besonders bevorzugt, in welchen der Rest X für Phenyl, 2-Pyridyl oder 3-Pyridyl steht, welche jeweils substituiert sind durch einen oder mehrere Substituenten, ausgewählt aus der Gruppe, bestehend aus Fluor, Chlor, Brom, Iod, CF₃, Methoxy, Ethoxy, Propoxy, Trifluorethoxy, Methylsulfanyl, 2,2,2-Trifluorethylsulfanyl, Methylsulfinyl, 2,2,2-Trifluorethylsulfinyl, Methylsulfonyl, 2,2,2-Trifluorethylsulfonyl, Cyano und Dimethylamino; oder für gegebenenfalls mit einem oder mehreren Halogenatomen, Cyano, Nitro, Methyl, Methoxy oder CF₃ substituiertes Phenyl steht, wobei vicinale Alkyl- oder Alkoxygruppen an dem Phenylsubstituenten zusammen mit den Kohlenstoffatomen, an die sie gebunden sind, ein fünf - bis siebengliedriges cyclisches System bilden können, das 1 oder 2 Sauerstoffatome enthält, wobei zwei Sauerstoffatome nicht direkt miteinander verbunden sind, und dessen Alkylanteil mit einem oder mehreren weiteren Alkylresten substituiert sein kann; steht.

In einer **zweiten** Ausführungsform der vorliegenden Erfindung sind Verbindungen der allgemeinen Formel (I)
(a) bevorzugt, in welchen der Rest R¹ für Wasserstoff, Alkyl, welches gegebenenfalls einfach oder unabhängig voneinander mehrfach mit Alkoxy, Haloalkoxy, Alkylsulfanyl, Haloalkylsulfanyl, Alkylsulfinyl, Haloalkylsulfinyl, Alkylsulfonyl, Haloalkylsulfonyl, Alkylcarbonyl, Alkoxycarbonyl, Hydroxy und/oder Cycloalkyl substituiert ist; Alkenyl, welches gegebenenfalls einfach oder unabhängig voneinander mehrfach mit Halogen, Alkoxy, Haloalkoxy, Alkylsulfanyl, Haloalkylsulfanyl, Alkylsulfinyl, Haloalkylsulfinyl, Alkylsulfonyl, Haloalkylsulfonyl, Alkylcarbonyl, Alkoxycarbonyl, und/oder Cycloalkyl substituiert ist; Cycloalkyl, welches gegebenenfalls einfach oder unabhängig voneinander mehrfach mit Alkyl, Haloalkyl und/oder Halogen substituiert ist; Haloalkyl, welches gegebenenfalls einfach oder unabhängig voneinander mehrfach mit Alkoxy, Alkylsulfanyl, Haloalkylsulfanyl, Alkylsulfinyl, Haloalkylsulfinyl, Alkylsulfonyl, Haloalkylsulfonyl und/oder Phenyl substituiert ist; CH=NOH, CH=NOCH₃oder CN steht;
(b) weiter bevorzugt, in welchen der Rest R¹ für Alkyl, welches gegebenenfalls einfach oder unabhängig voneinander mehrfach mit Alkoxy, substituiert ist; Alkenyl, welches gegebenenfalls einfach oder unabhängig voneinander mehrfach mit Halogen substituiert ist; Cycloalkyl, welches gegebenenfalls einfach oder unabhängig voneinander mehrfach mit Alkyl, Haloalkyl und/oder Halogen substituiert ist; Haloalkyl, welches gegebenenfalls einfach oder unabhängig voneinander mehrfach mit Alkoxy substituiert ist; CH=NOH, CH=NOCH₃ oder CN steht;
(c) besonders bevorzugt, in welchen der Rest R¹ für CH₃, CH₂CH₃, CH(CH₃)₂, CH₂CH₃CH₃, C(CH₃)₃, C(OCH₃)HCH₂CH₃, CH(OCH₃)₂, CH=CH₂, Prop-1-en-2-yl, Cyclopropyl, CF₃, CHFCH₃, CHF₂, CF₂Cl, CF₂Br, CF₂CF₃, CF_{Z}CH₃, CF₂CF₂CF₃, CF₂CF₂H, 2-Fluorpropan-2-yl, 1,1,2,2 Tetrafluor-2-methoxyethyl, CH=NOH, CH=NOCH₃, oder CN steht.

In einer **dritten** Ausführungsform der vorliegenden Erfindung sind Verbindungen der allgemeinen Formel (I) bevorzugt, in welchen der Rest R² für Amino oder substituiertes Amino, wobei das substituierte Amino einfach oder unabhängig voneinander zweifach substituiert sein kann mit Alkyl, Haloalkyl, Cycloalkylalkyl, gegebenenfalls durch Halogen oder Phenyl substitituiertes Alkenyl, Alkinyl, Heterocyclylalkyl und/oder Heteroarylalkyl, wobei der heteroaromatische Ring gegebenenfalls einfach oder mehrfach substituiert sein kann mit einem oder mehreren Substituenten unabhängig voneinander ausgewählt aus Halogen, Alkyl, Haloalkyl und Alkoxy; Benzyl, wobei der Phenylring im Benzyl gegebenenfalls einfach oder mehrfach substituiert sein kann mit einem oder mehreren Substituenten unabhängig voneinander ausgewählt aus Halogen, Alkyl, Haloalkyl und Alkoxy steht;
(a) weiter bevorzugt, in welchen der Rest R² für Amino oder substituiertes Amino, wobei das substituierte Amino einfach oder unabhängig voneinander zweifach substituiert sein kann mit Alkyl, gegebenenfalls durch Halogen oder Phenyl substitituiertes Alkenyl, Alkinyl, Heteroarylalkyl, wobei der heteroaromatische Ring gegebenenfalls einfach oder mehrfach substituiert sein kann mit einem oder mehreren Substituenten unabhängig voneinander ausgewählt aus Halogen und/oder Alkyl; Benzyl, wobei der Phenylring im Benzyl gegebenenfalls einfach oder mehrfach substituiert sein kann mit einem oder mehreren Substituenten unabhängig voneinander ausgewählt aus Halogen und Alkoxy steht;
(b) besonders bevorzugt, in welchen der Rest R² für Amino, Methylamino, Dimethylamino, Benzylamino, Dibenzylamino, (4-Chlorbenzyl)amino, Bis(4-chlorbenzyl)amino, (4-Methoxybenzyl)amino, Bis(4-methoxybenzyl)amino, (2-Methylprop-2-en-1-yl)amino, Prop-2-en-1-ylamino, Diprop-2-en-1-ylamino, Bis(2-methylprop-2-en-1-yl)amino, Prop-2-in-1-ylamino, Bis(prop-2-in-1-yl)amino, (Pyrazin-2-ylmethyl)amino, (6-Methyl-pyridin-2-ylmethyl)amino, Bis(6-methyl-pyridin-2-ylmethyl)amino oder (Pyridin-2-ylmethyl)amino steht.

In einer **vierten** Ausführungsform der vorliegenden Erfindung sind Verbindungen der allgemeinen Formel (I)
(a) bevorzugt, in welchen die Reste R³ und R⁴ unabhängig voneinander für Wasserstoff, Halogen, Alkyl, Cycloalkyl, Haloalkyl, Cyano und/oder Hydroxy;
(b) weiter bevorzugt, in welchen die Reste R³ und R⁴ unabhängig voneinander für Wasserstoff, Halogen und/oder Alkyl;
(c) besonders bevorzugt, in welchen die Reste R³ und R⁴ unabhängig voneinander für Wasserstoff, Chlor und/oder Methyl stehen.

In einer **fünften** Ausführungsform der vorliegenden Erfindung sind Verbindungen der allgemeinen Formel (I)
(a) bevorzugt, in welchen der Rest R⁵ für Halogen, Alkyl, Haloalkyl, Hydroxy, Alkoxy, Haloalkoxy, Alkylsulfanyl, Haloalkylsulfanyl, Alkylsulfinyl, Haloalkylsulfinyl, Alkylsulfonyl, Haloalkylsulfonyl, Cyano, Amino, Alkylamino, Dialkylamino, Alkylsulfonylamino, Dialkylsulfonylamino oder Heteroaryl steht, wobei der heteroaromatische Ring gegebenenfalls einfach oder mehrfach substituiert sein kann mit einem oder mehreren Substituenten unabhängig voneinander ausgewählt aus Halogen, Alkyl, Haloalkyl und Alkoxy;
(b) weiter bevorzugt, in welchen der Rest R⁵ für Halogen, Alkyl, Haloalkyl, Alkoxy, Haloalkoxy, Alkylsulfanyl, Alkylsulfinyl, Haloalkylsulfinyl, Alkylsulfonyl, Cyano, Dialkylamino oder Heteroaryl steht, wobei der heteroaromatische Ring gegebenenfalls einfach oder mehrfach substituiert sein kann mit einem oder mehreren Substituenten unabhängig voneinander ausgewählt aus Halogen und Alkyl; und
(c) besonders bevorzugt, in welchen der Rest R⁵ für Chlor, Brom, Methyl, CF₃, Methoxy, Ethoxy, Propoxy, Propan-2-yloxy, Dimethylamino, Cyano, Methylsulfanyl, Methylsulfinyl, Methylsulfonyl, 1H-Pyrazol-1-yl, 1H-Imidazol-1-yl, oder 4-Fluor-1H-pyrazol-1-yl; steht.

In einer weiteren Ausführungsform der vorliegenden Erfindung sind Verbindungen der allgemeinen Formel (I) bevorzugt, in welchen
der Rest X für Phenyl, 2-Pyridyl oder 3-Pyridyl steht, welche jeweils substituiert sind durch einen oder mehrere Substituenten, ausgewählt aus der Gruppe, bestehend aus Halogen, Alkyl, Haloalkyl, Alkoxy, Haloalkoxy, Alkylsulfanyl, Haloalkylsulfanyl, Alkylsulfinyl, Haloalkylsulfinyl, Alkylsulfonyl, Haloalkylsulfonyl, Cyano, Nitro und Dialkylamino; oder für gegebenenfalls mit einem oder mehreren Halogenatomen, Cyano, Nitro, Alkyl, Alkoxy oder Haloalkyl substituiertes Phenyl, 2-Pyridyl und 3-Pyridyl steht, wobei vicinale Alkyl-, Haloalkyl- und/oder Alkoxygruppen an dem Phenylsubstituenten, 2-Pyridylsubstituenten oder 3-Pyridylsubstituenten zusammen mit den Kohlenstoffatomen, an die sie gebunden sind, ein fünf- bis siebengliedriges cyclisches System bilden können, das 0 bis 2 Sauerstoff- oder Stickstoffatome enthält, wobei zwei Sauerstoffatome nicht direkt miteinander verbunden sind, und dessen Alkylanteil gegebenenfalls mit einem oder mehreren Halogenatomen und/oder weiteren Alkylresten substituiert sein kann;
der Rest R¹ für Wasserstoff, Alkyl, welches gegebenenfalls einfach oder unabhängig voneinander mehrfach mit Alkoxy, Haloalkoxy, Alkylsulfanyl, Haloalkylsulfanyl, Alkylsulfinyl, Haloalkylsulfinyl, Alkylsulfonyl, Haloalkylsulfonyl, Alkylcarbonyl, Alkoxycarbonyl, Hydroxy und/oder Cycloalkyl substituiert ist;
Alkenyl, welches gegebenenfalls einfach oder unabhängig voneinander mehrfach mit Halogen, Alkoxy, Haloalkoxy, Alkylsulfanyl, Haloalkylsulfanyl, Alkylsulfinyl, Haloalkylsulfinyl, Alkylsulfonyl, Haloalkylsulfonyl, Alkylcarbonyl, Alkoxycarbonyl, und/oder Cycloalkyl substituiert ist;
Cycloalkyl, welches gegebenenfalls einfach oder unabhängig voneinander mehrfach mit Alkyl, Haloalkyl und/oder Halogen substituiert ist;
Haloalkyl, welches gegebenenfalls einfach oder unabhängig voneinander mehrfach mit Alkoxy, Alkylsulfanyl, Haloalkylsulfanyl, Alkylsulfinyl, Haloalkylsulfinyl, Alkylsulfonyl, Haloalkylsulfonyl und/oder Phenyl substituiert ist;
CH=NOH, CH=NOCH₃ oder CN steht;
der Rest R² für Amino und substituiertes Amino, wobei das substituierte Amino einfach oder unabhängig voneinander zweifach substituiert sein kann mit Alkyl, Haloalkyl, Cycloalkylalkyl, gegebenenfalls durch Halogen oder Phenyl substitituiertes Alkenyl, Alkinyl, Heterocyclylalkyl und/oder Heteroarylalkyl, wobei der heteroaromatische Ring gegebenenfalls einfach oder mehrfach substituiert sein kann mit einem oder mehreren Substituenten unabhängig voneinander ausgewählt aus Halogen, Alkyl, Haloalkyl und Alkoxy; Benzyl, wobei der Phenylring im Benzyl gegebenenfalls einfach oder mehrfach substituiert sein kann mit einem oder mehreren Substituenten unabhängig voneinander ausgewählt aus Halogen, Alkyl, Haloalkyl und Alkoxy, steht;
die Reste R³ und R⁴ unabhängig voneinander für Wasserstoff, Halogen, Alkyl, Cycloalkyl, Haloalkyl, Cyano und/oder Hydroxy stehen und
der Rest R⁵ für Halogen, Alkyl, Haloalkyl, Hydroxy, Alkoxy, Haloalkoxy, Alkylsulfanyl, Haloalkylsulfanyl, Alkylsulfinyl, Haloalkylsulfinyl, Alkylsulfonyl, Haloalkylsulfonyl, Cyano, Amino, Alkylamino, Dialkylamino, Alkylsulfonylamino, Dialkylsulfonylamino oder Heteroaryl steht, wobei der heteroaromatische Ring gegebenenfalls einfach oder mehrfach substituiert sein kann mit einem oder mehreren Substituenten unabhängig voneinander ausgewählt aus Halogen, Alkyl, Haloalkyl und Alkoxy.

In einer weiteren Ausführungsform der vorliegenden Erfindung sind Verbindungen der allgemeinen Formel (I) besonders bevorzugt, in welchen
der Rest X für Phenyl, 2-Pyridyl oder 3-Pyridyl steht, welche jeweils substituiert sind durch einen oder mehrere Substituenten, ausgewählt aus der Gruppe bestehend aus Fluor, Chlor, Brom, Iod, CF₃, Methoxy, Ethoxy, Propanyloxy, Trifluorethoxy, Methylsulfanyl, 2,2,2-Trifluorethylsulfanyl, Methylsulfinyl, 2,2,2-Trifluorethylsulfinyl, Methylsulfonyl, 2,2,2-Trifluorethylsulfonyl, Cyano und Dimethylamino; oder gegebenenfalls für mit einem oder mehreren Halogenatomen, Cyano, Nitro, Methyl, Methoxy oder CF₃ substituiertes Phenyl steht, wobei vicinale Alkyl- oder Alkoxygruppen an dem Phenylsubstituenten zusammen mit den Kohlenstoffatomen, an die sie gebunden sind, ein fünf- bis siebengliedriges cyclisches System bilden können, das 1 oder 2 Sauerstoffatome enthält, wobei zwei Sauerstoffatome nicht direkt miteinander verbunden sind, und dessen Alkylanteil mit einem oder mehreren weiteren Alkylresten substituiert sein kann;
der Rest R¹ für CH₃, CH₂CH₃, CH(CH₃)₂, CH₂CH₃CH₃, C(CH₃)₃, C(OCH₃)HCH₂CH₃, CH(OCH₃)₂, CH=CH₂, Prop-1-en-2-yl, Cyclopropyl, CF₃, CHFCH₃, CHF₂, CF₂Cl, CF₂Br, CF₂CF₃, CF₂CH₃, CF₂CF₂CF₃, CF₂CF₂H, 2-Fluorpropan-2-yl, 1,1,2,2-Tetrafluor-2-methoxyethyl, CH=NOH, CH=NOCH₃, oder CN steht,
der Rest R² für Amino, Methylamino, Dimethylamino, Benzylamino, Dibenzylamino, (4-Chlorbenzyl)amino, Bis(4-chlorbenzyl)amino, (4-Methoxybenzyl)amino, Bis(4-methoxybenzyl)amino, (2-Methylprop-2-en-1-yl)amino, Prop-2-en-1-ylamino, Diprop-2-en-1-ylamino, Bis(2-methylprop-2-en-1-yl)amino, Prop-2-in-1-ylamino, Bis(prop-2-in-1-yl)amino, (Pyrazin-2-ylmethyl)amino, (6-Methyl-pyridin-2-ylmethyl)amino, Bis(6-methyl-pyridin-2-ylmethyl)amino oder (Pyridin-2-ylmethyl)amino steht,
die Reste R³ und R⁴ unabhängig voneinander für Wasserstoff, Chlor und/oder Methyl stehen und
der Rest R⁵ Chlor, Brom, Methyl, CF₃, Methoxy, Ethoxy, Propoxy, Propan-2-yloxy, Dimethylamino, Cyano, Methylsulfanyl, Methylsulfinyl, Methylsulfonyl, 1H-Pyrazol-1-yl, 1H-Imidazol-1-yl oder 4-Fluor-1H-pyrazol-1-yl steht.

In einer weiteren Ausführungsform der vorliegenden Erfindung sind Verbindungen der allgemeinen Formel (I) besonders bevorzugt, in welchen
der Rest X für Phenyl, 2-Pyridyl oder 3-Pyridyl steht, welche jeweils substituiert sind durch einen oder mehrere Substituenten, ausgewählt aus der Gruppe bestehend aus Fluor, Chlor, Brom, Iod, CF₃, Methoxy, Ethoxy, Propanyloxy, Trifluorethoxy, Methylsulfanyl, 2,2,2-Trifluorethylsulfanyl, 2,2,2-Trifluorethylsulfinyl, Cyano und Dimethylamino; oder für gegebenenfalls mit einem oder mehreren Halogenatomen, Cyano oder CF₃ substituiertes Phenyl steht, wobei vicinale Alkyl- oder Alkoxygruppen an dem Phenylsubstituenten zusammen mit den Kohlenstoffatomen, an die sie gebunden sind, ein fünf - bis siebengliedriges cyclisches System bilden können, das 1 oder 2 Sauerstoffatome enthält, wobei zwei Sauerstoffatome nicht direkt miteinander verbunden sind, und dessen Alkylanteil mit einem oder mehreren weiteren Alkylresten substituiert sein kann;
der Rest R¹ für CH₃, CH₂CH₃, CH(CH₃n, CH₂CH₃CH₃, C(CH₃)₃, C(OCH₃)HCH₂CH₃, CH(OCH₃)₂, CH=CH₂, Prop-1-en-2-yl, Cyclopropyl, CF₃, CHFCH₃, CHF₂, CF₂Cl, CF₂CF₃, CF₂CH₃, CF₂CF₂CF₃, CF₂CF₂H, 2-Fluorpropan-2-yl, 1,1,2,2 Tetrafluor-2-methoxyethyl, CH=NOH, CH=NOCH₃, oder CN steht,
der Rest R² für Amino, Methylamino, Dimethylamino, Benzylamino, Dibenzylamino, (4-Chlorbenzyl)amino, Bis(4-chlorbenzyl)amino, (4-Methoxybenzyl)amino, Bis(4-methoxybenzyl)amino, (2-Methylprop-2-en-1-yl)amino, Prop-2-en-1-ylamino, Diprop-2-en-1-ylamino, Bis(2-methylprop-2-en-1-yl)amino, Bis(prop-2-in-1-yl)amino, (Pyrazin-2-ylmethyl)amino, Bis(6-methyl-pyridin-2-ylmethyl)amino oder (Pyridin-2-ylmethyl)amino steht,
die Reste R³ und R⁴ unabhängig voneinander für Wasserstoff, Chlor und/oder Methyl stehen und
der Rest R⁵ für Chlor, Brom, Methyl, CF₃, Methoxy, Ethoxy, Propoxy, Propan-2-yloxy, Dimethylamino, Cyano, Methylsulfanyl, Methylsulfinyl, Methylsulfonyl, 1H-Pyrazol-1-yl, 1H-Imidazol-1-yl oder 4-Fluor-1H-pyrazol-1-yl steht.

In einer weiteren Ausführungsform der vorliegenden Erfindung sind Verbindungen der allgemeinen Formel (I) besonders bevorzugt, in welchen
der Rest X für Phenyl oder 2-Pyridyl steht, welche jeweils substituiert sind durch einen oder mehrere Substituenten, ausgewählt aus der Gruppe bestehend aus Fluor, Chlor, Brom, Iod, CF₃, Methoxy, Ethoxy, Propanyloxy, Trifluorethoxy, Cyano und Dimethylamino; oder gegebenenfalls für mit einem oder mehreren Halogenatomen, Cyano oder CF₃ substituiertes Phenyl steht, wobei vicinale Alkyl- oder Alkoxygruppen an dem Phenylsubstituenten zusammen mit den Kohlenstoffatomen, an die sie gebunden sind, ein fünf- bis siebengliedriges cyclisches System bilden können, das 1 oder 2 Sauerstoffatome enthält, wobei zwei Sauerstoffatome nicht direkt miteinander verbunden sind, und dessen Alkylanteil mit einem oder mehreren weiteren Alkylresten substituiert sein kann;
der Rest R¹ für CH₂CH₃, CH(CH₃)₂, CH₂CH₃CH₃, C(OCH₃)HCH₂CH₃, CH=CH₂, Prop-1-en-2-yl, Cyclopropyl, CF₃, CHF₂, CF₂Cl, CF₂CF₃, CF₂CH₃, CF₂CF₂CF₃, CF₂CF₂H, 2-Fluorpropan-2-yl oder 1,1,2,2 Tetrafluor-2-methoxyethyl steht,
der Rest R² für Amino, Dimethylamino, Benzylamino, (4-Methoxybenzyl)amino, Bis(4-methoxybenzyl)amino, (2-Methylprop-2-en-1-yl)amino oder Diprop-2-en-1-ylamino steht,
die Reste R³ und R⁴ für Wasserstoff stehen und
der Rest R⁵ für Methyl, CF₃, Methoxy, Ethoxy, Propoxy, Propan-2-yloxy, Cyano, Methylsulfanyl oder 1H-Imidazol-1-yl steht.

Im Rahmen der vorliegenden Erfindung sind von der Verbindung der allgemeinen Formel (I) auch Verbindungen umfasst, die durch a) Protonierung, b) Alkylierung oder c) Oxidation an einem Stickstoffatom quatemiert sind.

Die Verbindungen der allgemeinen Formel (I) können gegebenenfalls durch Anlagerung einer geeigneten anorganischen oder organischen Säure, wie beispielsweise HCl, HBr, H₂SO₄ oder HNO₃, aber auch Oxalsäure oder Sulfonsäuren an eine basische Gruppe, wie z.B. Amino oder Alkylamino, Salze bilden. Geeignete Substituenten, die in deprotonierter Form, wie z. B. Sulfonsäuren oder Carbonsäuren, vorliegen, können innere Salze mit ihrerseits protonierbaren Gruppen, wie Aminogruppen bilden. Salze können ebenfalls dadurch gebildet werden, dass bei geeigneten Substituenten, wie z. B. Sulfonsäuren oder Carbonsäuren, der Wasserstoff durch ein im agrochemischen Bereich geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze mit Kationen der Formel [NRR'R"R"']+, worin R bis R'" jeweils unabhängig einen organischen Rest, insbesondere Alkyl, Aryl, Aralkyl oder Alkylaryl darstellen.

In der allgemeinen Formel (I) und allen übrigen Formeln in der vorliegenden Erfindung können die Reste Alkyl, Alkoxy, Haloalkyl, Haloalkoxy, Alkylamino, Alkylsulfinyl und Alkylsulfonyl, sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z. B. mit 1 bis 6 Kohlenstoflatomen, insbesondere 1 bis 4 Kohlenstoffatomen, bzw. bei ungesättigten Gruppen mit 2 bis 6 Kohlenstoffatomen, insbesondere 2 bis 4 Kohlenstoffatomen, bevorzugt. Alkylreste, auch in den zusammengesetzten Bedeutungen wie Alkoxy, Haloalkyl usw., bedeuten z.B. Methyl; Ethyl; Propyl wie n- oder i-Propyl; Butyl wie n-, i-, t- oder 2-Butyl; Pentyl wie n-Pentyl, iso-Pentyl und neo-Pentyl; Hexyl wie n-Hexyl, i-Hexyl, 3-Methylpentyl, 2,2-Dimethylbutyl und 2,3-Dimethylbutyl; und Heptyl wie n-Heptyl, 1-Methylhexyl und 1,4-Dimethylpentyl; Alkenyl- und Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw. Dreifachbindung enthalten ist. Alkenyl bedeutet z. B. Vinyl, 1-Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Ethinyl, Propargyl/Propinyl, But-2-in-1-yl, But-3-in-1-yl und 1-Methyl-but-3-in-1-yl.

Cycloalkyl-Gruppen sind z. B. Cyclopropyl, Cyclobutyl, Cyclopentyl, Cyclohexyl, Cycloheptyl und Cyclooctyl. Die Cycloalkyl Gruppen können in bi- oder tri-cyclischer Form vorkommen.

Wenn Haloalkylgruppen und Haloalkylreste von Haloalkoxy, Haloalkenyl, Haloalkinyl u.a. angegeben sind, sind bei diesen Resten die niederen Kohlenstoffgerüste, z. B. mit 1 bis 6 Kohlenstoffatomen oder 2 bis 6, insbesondere 1 bis 4 Kohlenstoffatomen oder bevorzugt 2 bis 4 Kohlenstoffatomen, sowie die entsprechenden ungesättigten und/oder substituierten Reste im Kohlenstoffgerüst jeweils geradkettig oder verzweigt, bevorzugt. Beispiele sind Trifluormethyl, Difluormethyl, 2,2,2-Trifluorethyl, Trifluorallyl und 1-Chlorprop-1-yl-3-yl.

Alkylen-Gruppen sind bei diesen Resten die niederen Kohlenstoffgerüste, z. B. mit 1 bis 10 Kohlenstoffatomen, insbesondere 1 bis 6 Kohlenstoffatomen oder bevorzugt 2 bis 4 Kohlenstoffatomen, sowie die entsprechenden ungesättigten und/oder substituierten Reste im Kohlenstoffgerüst, die jeweils geradkettig oder verzweigt sein können. Beispiele sind Methylen, Ethylen, n- und i-Propylen und n-, s-, i-, t-Butylen.

Hydroxyalkylgruppen sind bei diesen Resten die niederen Kohlenstoffgerüste, z. B. mit 1 bis 6 Kohlenstoffatomen, insbesondere 1 bis 4 Kohlenstoffatomen, sowie die entsprechenden ungesättigten und/oder substituierten Resten im Kohlenstoffgerüst, die jeweils geradkettig oder verzweigt sein können. Beispiele hierzu sind 1,2-Dihydroxyethyl und 3-Hydroxypropyl.

Halogen bedeutet Fluor, Chlor, Brom oder lod, Haloalkyl, -alkenyl und -alkinyl bedeuten durch Halogen, vorzugsweise durch Fluor, Chlor oder Brom, insbesondere durch Fluor und/oder Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Aryl bedeutet ein mono-, bi- oder polycyclisches aromatisches System, beispielsweise Phenyl oder Naphthyl, vorzugsweise Phenyl.

Ein heterocyclischer Rest (Heterocyclyl) enthält mindestens einen heterocyclischen Ring (= carbocyclischer Ring, in dem mindestens ein C-Atom durch ein Heteroatom ersetzt ist, vorzugsweise durch ein Heteroatom aus der Gruppe, bestehend aus N, O, S, P, B, Si, Se) der gesättigt, ungesättigt oder heteroaromatisch ist und dabei unsubstituiert oder substituiert sein kann, wobei die Bindungsstelle an einem Ringatom lokalisiert ist.

Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden auch mehrcyclische Systeme umfaßt, wie beispielsweise 8-Aza-bicyclo[3.2.1]octanyl oder 1-Aza-bicyclo[2.2.1]heptyl. Im Falle von gegebenenfalls substituiertem Heterocyclyl werden auch spirocyclische Systeme umfaßt, wie beispielsweise 1-Oxa-5-aza-spiro[2.3]hexyl.

Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe, bestehend aus N, O, und S, wobei jedoch nicht zwei Sauerstoffatome direkt benachbart sein sollen.

Unter dem Begriff Heteroaryl versteht man im Rahmen der vorliegenden Verbindung wie vorstehend unter "Heterocyclyl" definierte Systeme, die jedoch heteroaromatisch sind, d. h. eine vollständig ungesättigte aromatische heterocyclische Verbindung darstellen.

Mit der Definition "mit einem oder mehreren Resten substituiert ist" sind, wenn nicht anders definiert, unabhängig voneinander ein oder mehrere gleiche oder verschiedene Reste gemeint, wobei zwei oder mehrere Reste an einem Cyclus als Grundkörper einen oder mehrere Ringe bilden können.

Substituierte Reste, wie ein substituierter Alkyl-, Alkenyl-, Alkinyl-, Cycloalkyl-, Cycloalkenyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy oder eine der Carboxygruppe äquivalente Gruppe, Cyano, Isocyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, Trialkylsilyl und gegebenenfalls substituiertes Cycloalkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfonylgruppe umfasst sind, Alkylsulfonyl, Alkylphosphinyl, Alkylphosphonyl und, im Falle cyclischer Reste (= "cyclischer Grundkörper"), auch Alkyl, Haloalkyl, Alkylthio-alkyl, Alkoxy-alkyl, gegebenfalls substituiertes Mono- und Dialkylaminoalkyl und Hydroxyalkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Alkenylthio, Alkinylthio, Alkenyloxycarbonyl, Alkinyloxycarbonyl, Alkenylcarbonyl, Alkinylcarbonyl, Mono- und Dialkenylaminocarbonyl, Mono- und Dialkinylaminocarbonyl, Mono- und Dialkenylamino, Mono- und Dialkinylamino, Trialkenylsilyl, Trialkinylsilyl, gegebenenfalls substituiertes Cycloalkenyl, gegebenenfalls substituiertes Cycloalkinyl, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden oder einer Oxogruppe, Iminogruppe oder substituierten Iminogruppe substituiert sind.

Wenn zwei oder mehrere Reste einen oder mehrere Ringe bilden, so können diese carbocyclisch, heterocyclisch, gesättigt, teilgesättigt, ungesättigt, beispielsweise auch aromatisch und gegebenenfalls weiter substituiert sein. Die annellierten Ringe sind vorzugsweise 5- oder 6-Ringe, besonders bevorzugt sind benzokondensierte Cyclen.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugte Substituenten für die Substituentenebenen sind beispielsweise Amino, Hydroxy, Halogen, Nitro, Cyano, Isocyano, Mercapto, Isothiocyanato, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxycarbonyl, Alkenyloxy-carbonyl, Alkinyloxy-carbonyl, Aryloxycarbonyl, Alkanoyl, Alkenylcarbonyl, Alkinyl-carbonyl, Aryl-carbonyl, Alkylthio, Cycloalkylthio, Alkenylthio, Cycloalkenylthio, Alkinylthio, Alkylsulfenyl, Alkylsulfinyl, wobei beide Enantiomere der Alkylsulfinylgruppe umfasst sind, Alkylsulfonyl, Monoalkyl-aminosulfonyl, Dialkyl-aminosulfonyl, Alkylphosphinyl, Alkylphosphonyl, wobei für Alkylphosphinyl bzw. Alkylphosphonyl beide Enantiomere umfasst sind, N-Alkyl-aminocarbonyl, N,N-Dialkyl-aminocarbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl und Trialkylsilyl.

Wie bereits erwähnt sind bei Resten mit Kohlenstoffatomen solche mit 1 bis 6 Kohlenstoffatomen, vorzugsweise 1 bis 4 Kohlenstoffatomen, insbesondere 1 oder 2 Kohlenstoffatomen, bevorzugt. Bevorzugt sind in der Regel Substituenten aus der Gruppe Halogen, z.B. Fluor, Chlor und Brom, (C1-C4)-Alkyl, vorzugsweise Methyl oder Ethyl, (C1-C4)-Haloalkyl, vorzugsweise Trifluormethyl, (C1-C4)-Alkoxy, vorzugsweise Methoxy oder Ethoxy, (C1-C4)-Haloalkoxy, Nitro und Cyano.

Gegebenenfalls substituiertes Aryl oder Heteroaryl ist vorzugsweise Phenyl oder Heteroaryl, das unsubstituiert oder ein oder mehrfach, vorzugsweise bis zu dreifach durch gleiche oder verschiedene Reste aus der Gruppe Halogen, (C1-C4)-Alkyl, (C3-C6)-Cycloalkyl, (C1-C4)-Alkoxy, (C1-C4)-Halogenalkyl, (C1-C4)-Halogenalkoxy, (C1-C4)-Alkylthio, Cyano und Nitro substituiert ist, z. B. o-, m- und p-Tolyl, Dimethylphenyle, 2-, 3- und 4-Chlorphenyl, 2-, 3- und 4-Trifluormethyl und 2-, 3- und 4-Trichlormethyl-phenyl, 2,4-, 3,5-, 2,5- und 2,3-Dichlorphenyl, o-, m- und p-Methoxyphenyl.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen Verbindungen der allgemeinen Formel (I) sind durch verschiedene Herstellungsverfahren zugänglich, die ebenfalls Gegenstand der vorliegenden Erfindung sind. Zur Differenzierung der einzelnen Herstellungsverfahren werden die Verbindungen der allgemeinen Formel (I) in die Verbindungen der allgemeinen Formeln (IA), (IB) und (IC) unterteilt: 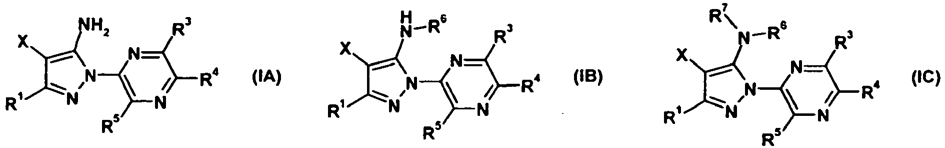 wobei
- X, R', R³, R⁴ und R⁵: die oben angegebenen Bedeutungen haben und
- R⁶, R⁷: für Alkyl, Haloalkyl, Alkoxyalkyl, Alkylsulfanylalkyl, Alkylsulfinylalkyl, Alkylsulfonylalkyl, Alkylcarbonyl, Cycloalkyl, Cycloalkylalkyl, Alkenyl (gegebenenfalls substituiert durch Halogen, Cyano, Alkoxy, Alkoxycarbonyl und Phenyl, wobei der Phenylring gegebenenfalls einfach oder mehrfach substituiert sein kann mit einem oder mehreren Substituenten unabhängig voneinander ausgewählt aus Halogen, Alkyl, Haloalkyl und Alkoxy), Alkinyl, Alkoxycarbonyl, Alkenyloxycarbonyl, Alkinyloxycarbonyl, Alkoxycarbonylalkyl, Alkoxycarbonylcarbonyl, Heterocyclylalkyl, Heteroarylalkyl (wobei der heteroaromatische Ring gegebenenfalls einfach oder mehrfach substituiert sein kann mit einem oder mehreren Substituenten unabhängig voneinander ausgewählt aus Halogen, Alkyl, Haloalkyl und Alkoxy), Benzyl oder Phenylcarbonyl (wobei der Phenylring im Benzyl und Phenylcarbonyl gegebenenfalls einfach oder mehrfach substituiert sein kann mit einem oder mehreren Substituenten unabhängig voneinander ausgewählt aus Halogen, Alkyl, Haloalkyl und Alkoxy) stehen.

### Verfahren (A)

Verbindungen der Formel (IA) werden z.B. nach folgendem Verfahren (A) synthetisiert: 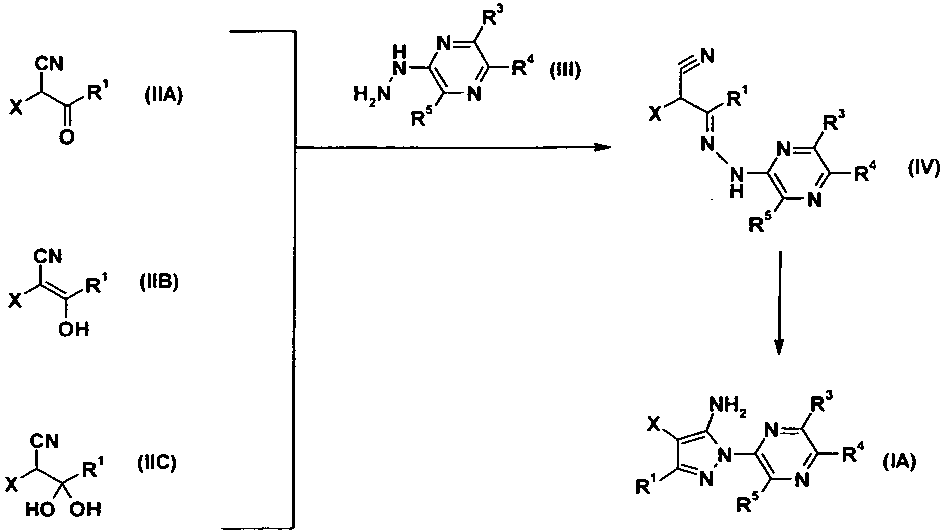 wobei X, R¹, R³, R⁴ und R⁵ die oben angegebenen Bedeutungen haben.

Beim erfindungsgemäßen Verfahren (A) zur Herstellung der Verbindungen der Formel (IA) werden Ketonitrile, deren Tautomere oder Hydrate der Formeln (IIA), (IIB) bzw. (IIC) mit Pyrazinyl-Hydrazinen der allgemeinen Formel (III) kondensiert, wobei zunächst Hydrazone der Formel (IV) als Intermediat entstehen und bei längerer Reaktionszeit und höherer Temperatur der Ringschluss zum Aminopyrazol der Formel (IA) erfolgt. Dabei können Säuren als Katalysator zugesetzt werden, wobei anorganische Säuren wie Salzsäure und organische Säuren wie Sulfonsäuren oder Essigsäure geeignet sein können.

Die Synthese von strukturverwandten Aminopyrazolen wird beschrieben in R. Aggarwal et al, Bioorg. Med. Chem. 14 (2006), 6, 1785-1791; S. P. Singh et al, Eur. J. Med. Chem. 40 (2005), 922-927; DE 26 43 640 A; US 3,041,342; WO 2008/077483 A.

Die Ketonitrile können in den tautomeren Formen (IIA) und (IIB) und als Hydrat (IIC) vorliegen. Die Ausgangsverbindungen können auch in Form ihrer Salze eingesetzt werden, z.B. können die Ketonitrile in Form ihrer Alkalisalze und die Pyrazinyl-Hydrazine in Form ihrer Hydrochloride verwendet werden.

Die Ketonitrile der Formel (II) lassen sich nach bekannten Methoden herstellen, z.B. W. R. Nes, Alfred Burger, J. Amer. Chem. Soc. 72 (1950), 5409-5413.

Die Pyrazinyl-Hydrazine der Formel (III) sind zum Teil kommerziell verfügbar. Die Herstellung von Pyrazinyl-Hydrazinen der Formel (III) erfolgt beispielsweise nach den Methoden beschrieben in: Methoden der Organischen Chemie (Houben-Weyl), Organische Stickstoff-Verbindungen, Band E 16a, Teil 1, S. 678-775, Georg Thieme Verlag Stuttgart- New York, 1990 und WO 98/32739 A.

### Verfahren (B)

Alternativ können Verbindungen der Formel (IA) nach dem erfindungsgemäßen Verfahren (B) synthetisiert werden: 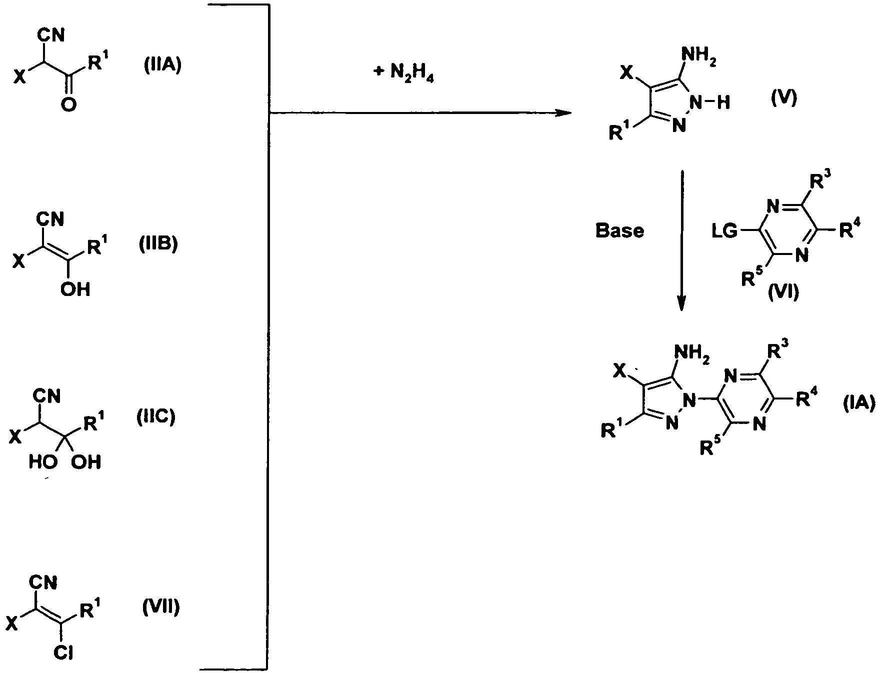 LG = Halogen oder Alkylsulfonyl
wobei X, R¹, R³, R⁴ und R⁵ die oben angegebenen Bedeutungen haben.

Beim erfindungsgemäßen Verfahren (B) zur Herstellung der Verbindungen der Formel (IA) werden 1-H-Aminopyrazole der Formel (V) mit Pyrazinyl-Halogeniden bzw. -Alkylsulfonen der Formel (VI) in Gegenwart einer Base in organischen Lösungsmitteln umgesetzt, wobei bevorzugt ein bestimmtes Isomer, das Aminopyrazol der Formel (IA), gebildet wird.

Die 1-H-Aminopyrazole der Formel (V) lassen sich nach bekannten Methoden herstellen (siehe auch DE-A 2643640, C. Chen et al, Bioorg. Med. Chem. Lett., 14 (2004), 14, 3669; Gilligan, Paul J. et al, Bioorg. Med. Chem., 8, (2000), 1, 181 - 190).

Die Pyrazinyl-Halogenide oder -Alkylsulfone der Formel (VI) sind zum Teil kommerziell verfügbar oder lassen sich nach dem Fachmann bekannten Methoden synthetisieren. Umsetzungen von Pyrazinyl-Halogeniden mit 1-H-Pyrazolen sind u.a. beschrieben in: Journal of Chemical Research, Synopses, 1989 (7), 189 und Dalton Transactions, 2003 (10), 2053-2060.

### Verfahren (C)

Verbindungen der Formel (IA) können alternativ nach folgendem Verfahren (C) synthetisiert werden: 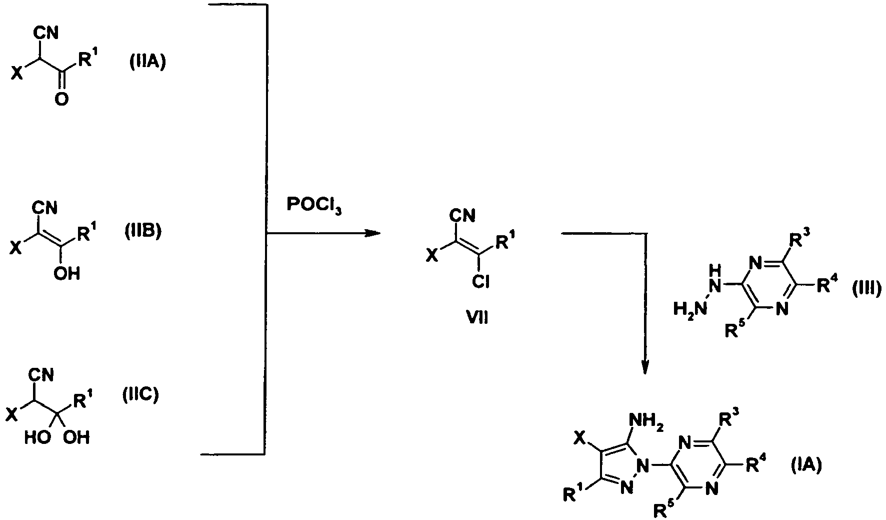 wobei X, R¹, R³, R⁴ und R⁵ die oben angegebenen Bedeutungen haben.

Beim erfindungsgemäßen Verfahren (C) zur Herstellung der Verbindungen der Formel (IA) werden Ketonitrile, deren Tautomere oder Hydrate der Formeln (IIA), (IIB) bzw. (IIC) mit Chlorierungsmitteln, z.B. Phosphorylchlorid, Thionylchlorid, Phosgen, Chlor oder Oxalylchlorid, gegebenenfalls in einem inerten organischen Lösungsmittel verdünnt bzw. mit Hilfsbasen wie Stickstoffbasen unterstützt, zu Chlor-Acrylnitrilen (VII) umgesetzt, wobei die Reaktion im Temperaturbereich von -20 °C bis 120°C durchgeführt werden kann.

In einem anschließenden Schritt erfolgt die Kondensation mit Pyrazinyl-Hydrazinen (III) in einem geeigneten organischen Lösungsmittel in Gegenwart von basischen Hilfsreagentien, z.B. Alkoholaten oder Stickstoffbasen, wobei die Reaktion im Temperaturbereich von -20 °C bis 120 °C durchgeführt werden kann.

Die Ketonitrile können in den tautomeren Formen (IIA) und (IIB) und als Hydrat (IIC) vorliegen. Die Ausgangsverbindungen können auch in Form ihrer Salze eingesetzt werden, z.B. können die Ketonitrile in Form ihrer Alkalisalze und die Pyrazinyl-Hydrazine in Form ihrer Hydrochloride verwendet werden.

Die Ketonitrile der Formel (II) lassen sich - wie bereits hinsichtlich des Verfahrens (A) erwähnt - nach bekannten Methoden herstellen: W. R. Nes, Alfred Burger, J. Amer. Chem. Soc. 72 (1950), 5409-5413.

Die Pyrazinyl-Hydrazine der Formel (III) sind zum Teil kommerziell verfügbar. Die Herstellung von Pyrazinyl-Hydrazinen der Formel (III) erfolgt beispielsweise nach den Methoden beschrieben in: Methoden der Organischen Chemie (Houben-Weyl), Organische Stickstoff-Verbindungen, Band E 16a, Teil 1, S. 678-775, Georg Thieme Verlag Stuttgart- New York, 1990 und WO 98/32739 A.

Die Chlor-Acrylnitrile der Formel (VII) lassen sich ebenfalls nach bekannten Methoden herstellen (siehe auch JP08-208620 (X = 4-CF₃C₆H₆ und R¹ = CF₃))

### Verfahren (D)

Erfindungsgemäße Verbindungen der Formel (IB) und (IC), in denen mindestens ein Rest von R⁶ und R⁷ nicht für Wasserstoff (substituiertes Amin) steht, lassen sich ausgehend von der erfindungsgemäßen Verbindung (IA) nach dem erfindungsgemäßen Verfahren (D) synthetisieren: 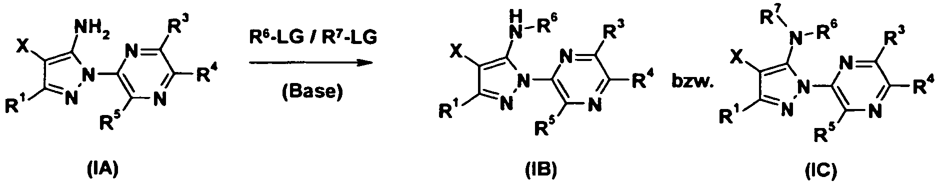 LG = Halogen oder Alkylsulfonyl
wobei X, R¹, R³, R⁴ und R⁵ die oben angegebenen Bedeutungen haben können;
- LG: für Halogen oder Alkylsulfonyl steht; und
- R⁶ und R⁷,: jeweils unabhängig voneinander, für Wasserstoff, Alkyl, Haloalkyl, Alkoxyalkyl, Alkylsulfanylalkyl, Alkylsulfinylalkyl, Alkylsulfonylalkyl, Alkylcarbonyl, Cycloalkyl, Cycloalkylalkyl, Alkenyl, wobei die vorstehenden Reste gegebenenfalls substituiert sind durch Halogen, Cyano, Alkoxy, Alkoxycarbonyl und Phenyl, wobei der Phenylring gegebenenfalls einfach oder mehrfach substituiert ist mit einem oder mehreren Substituenten unabhängig voneinander ausgewählt aus Halogen, Alkyl, Haloalkyl und Alkoxy; Alkinyl, Alkoxycarbonyl, Alkenyloxycarbonyl, Alkinyloxycarbonyl, Alkoxycarbonylalkyl, Alkoxycarbonylcarbonyl, Heterocyclyl, Heteroaryl, Heterocyclylalkyl oder Heteroarylalkyl, wobei der heterocyclische oder heteroaromatische Ring gegebenenfalls einfach oder mehrfach substituiert sein kann mit einem oder mehreren Substituenten, unabhängig voneinander ausgewählt aus Halogen, Alkyl, Haloalkyl und Alkoxy; Benzyl oder Phenylcarbonyl, wobei der Phenylring im Benzyl und Phenylcarbonyl gegebenenfalls einfach oder mehrfach substituiert ist mit einem oder mehreren Substituenten unabhängig voneinander ausgewählt aus Halogen, Alkyl, Haloalkyl und Alkoxy; mit der Massgabe, dass mindestens ein Rest von R⁶ und R⁷ ungleich Wasserstoff ist.

Beim erfindungsgemäßen Verfahren (D) zur Herstellung der Verbindungen der Formel (IB) oder (IC) werden Verbindungen der Formel (IA) mit einem oder zwei Alkylierungsmitteln oder Acylierungsmitteln R⁶-LG bzw. R⁷-LG umgesetzt, wobei durch Monosubstitution und Disubstitution Aminopyrazole der Formeln (IIB) und (IC) entstehen. Geeignete Alkylierungsmittel sind Alkylbromide, Alkyldibromide, Alkyliodide, Alkyldiiodide, Dialkylsulfate und Alkylsulfonate. Als Acylierungsmittel werden Carbonsäureanhydride und Carbonsäurechloride verwendet.

### Verfahren (E)

Verbindungen der Formel (IA) können alternativ auch nach folgendem Verfahren (E) synthetisiert werden: 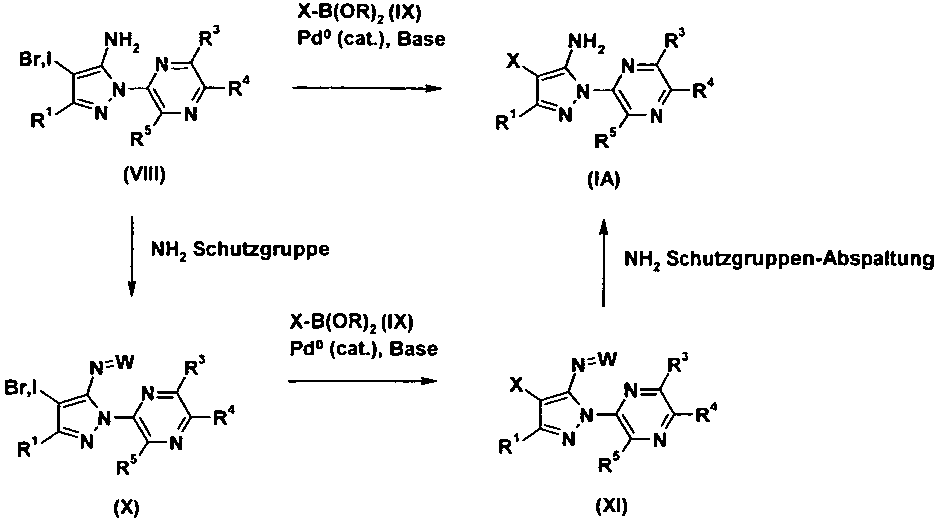 W = Schutzgruppe, z.B. DMF-DMA-Addukt
wobei X, R¹, R³, R⁴ und R⁵ die oben angegebenen Bedeutungen haben.

Beim erfindungsgemäßen Verfahren (E) zur Herstellung der Verbindungen der Formel (IA) werden Bromide oder Iodide der Formel (VIII) mit Boronsäuren oder Boronsäureestern der Formel (IX) in Gegenwart von geeigneten Palladium-Katalysatoren und Basen (Suzuki-Reaktion) im Temperaturbereich von -20 °C bis 120°C in geeigneten Lösungsmitteln zur Reaktion gebracht.

Die Bromide oder Iodide der Formel (VIII) lassen sich nach bekannten Methoden herstellen, beschrieben z.B. in: Chemistry of Heterocyclic Compounds (New York, NY, United States), 41(1), 105-110; 2005; Bioorganic & Medicinal Chemistry, 12, 2004 (12), 3345-3355; Journal of Medicinal Chemistry, 20, 1977 (12), 1562-1569.

Die Boronsäuren oder Boronsäureester der Formel (IX) sind zum Teil kommerziell verfügbar oder sie sind nach bekannten Methoden leicht herstellbar. Dies ist beispielsweise beschrieben in WO 99/64428 A.

Die NH₂-Gruppe der Bromide oder Iodide der Formel (VIII) kann zur Verbesserung der Ausbeuten der Suzuki-Reaktion mit einer Schutzgruppe versehen werden, z.B. mittels Umsetzung von (VIII) mit Dimethylformamid-Dimethylacetal (DMF-DMA). Die resultierenden Imine (XI) können nach erfolgreicher Suzuki-Kupplung in Gegenwart starker Säuren, z.B. Salzsäure, in geeigneten Lösungsmitteln, z.B. Methanol, zu den Endverbindungen (IA) umgesetzt werden.

Beispiele für Umsetzungen von Aminopyrazolen mit DMF-DMA sind beispielsweise in US 2006/0014802 A, Bioorganic & Medicinal Chemistry Letters, 18, 2008 (3), 959-962 beschrieben.

Die erfindungsgemäßen Verbindungen der allgemeinen Formel (I) eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., A-leurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-FettalkoholEther, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, AlkylAryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignet sind Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im Allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryI-IIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) behandelt werden. Die bei den Wirkstoffen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Darüber hinaus können die erfindungsgemäßen Verbindungen zur Bekämpfung einer Vielzahl verschiedener Schädlinge einschließlich beispielsweise schädlicher saugender Insekten, beißender Insekten und anderen an Pflanzen parasitierenden Schädlingen, Vorratsschädlingen, Schädlingen, die industrielle Materialien zerstören und Hygieneschädlingen einschließlich Parasiten im Bereich Tiergesundheit verwendet und zu ihrer Bekämpfung wie zum Beispiel ihrer Auslöschung und Ausmerzung eingesetzt werden. Die vorliegende Erfindung schließt somit auch ein Verfahren zur Bekämpfung von Schädlingen ein.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder Endoparasiten. Der Begriff Endoparasiten schließt insbesondere Helminthen wie Cestoden, Nematoden oder Trematoden, und Protozoen wie Kozzidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen.

Zu diesen Parasiten gehören:
Aus der Ordnung der Anoplurida z.B. Haematopinus spp., Linognathus spp., Pediculus spp., Phtirus spp., Solenopotes spp.; spezielle Beispiele sind: Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;

Aus der Ordnung der Mallophagida und den Unterordnungen Amblycerina und Ischnocerina z.B. Trimenopon spp., Menopon spp., Trinoton spp., Bovicola spp., Werneckiella spp., Lepikentron spp., Damalina spp., Trichodectes spp., Felicola spp.; spezielle Beispiele sind: Bovicola bovis, Bovicola ovis, Bovicola limbata, Damalina bovis, Trichodectes canis, Felicola subrostratus, Bovicola caprae, Lepikentron ovis, Werneckiella equi;

Aus der Ordnung der Diptera und den Unterordnungen Nematocerina und Brachycerina z.B. Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; spezielle Beispiele sind: Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca;

Aus der Ordnung der Siphonapterida z.B. Pulex spp., Ctenocephalides spp., Tunga spp., Xenopsylla spp., Ceratophyllus spp.; spezielle Beispiele sind: Ctenocephalides canis, Ctenocephalides felis, Pulex irritans, Tunga penetrans, Xenopsylla cheopis;

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp. (z.B. Suppella longipalpa);

Aus der Unterklasse der Acari (Acarina) und den Ordnungen der Meta- und Mesostigmata z.B. Argas spp., Ornithodorus spp., Otobius spp., Ixodes spp., Amblyomma spp., Rhipicephalus (Boophilus) spp., Dermacentor spp., Haemophysalis spp., Hyalomma spp., Dermanyssus spp., Rhipicephalus spp. (der ursprünglichen Gattung der Mehrwirtszecken), Ornithonyssus spp., Pneumonyssus spp., Raillietia spp., Pneumonyssus spp., Sternostoma spp., Varroa spp., Acarapis spp.; spezielle Beispiele sind: Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Omithonyssus bursa, Omithonyssus sylviarum, Varroa jacobsoni;

Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.; spezielle Beispiele sind: Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic mange, Pneumonyssoides caninum, Acarapis woodi.

Die erfindungsgemäßen Wirkstoffe eignen sich auch zur Bekämpfung von Arthropoden, Helminthen und Protozoen, die Tiere befallen. Zu den Tieren zählen landwirtschaftliche Nutztiere wie z.B. Rinder, Schafe, Ziegen, Pferde, Schweine, Esel, Kamele, Büffel, Kaninchen, Hühner, Puten, Enten, Gänse, Zuchtfische, Honigbienen. Zu den Tieren zählen außerdem Haustiere - die auch als Heimtiere bezeichnet werden - wie z.B. Hunde, Katzen, Stubenvögel, Aquarienfische sowie sogenannte Versuchstiere wie z.B. Hamster, Meerschweinchen, Ratten und Mäuse.

Durch die Bekämpfung dieser Arthropoden, Helminthen und/oder Protozoen sollen Todesfälle vermindert und die Leistung (bei Fleisch, Milch, Wolle, Häuten, Eiern, Honig usw.) und die Gesundheit des Wirtstieres verbessert werden, so dass durch den Einsatz der erfindungsgemäßen Wirkstoffe eine wirtschaftlichere und einfachere Tierhaltung möglich ist.

So ist es beispielsweise wünschenswert, die Aufnahme von Blut des Wirts durch die Parasiten (falls zutreffend) zu verhindern oder zu unterbrechen. Eine Bekämpfung der Parasiten kann außerdem dazu beitragen, die Übertragung infektiöser Substanzen zu verhindern.

Der Begriff "Bekämpfung", so wie er hier bezogen auf den Bereich Tiergesundheit verwendet wird, bedeutet, dass die Wirkstoffe wirken, indem sie das Vorkommen des betreffenden Parasiten in einem mit solchen Parasiten befallenen Tier auf unschädliche Niveaus reduzieren. Genauer gesagt bedeutet "Bekämpfung", wie hier verwendet, dass der Wirkstoff den betreffenden Parasiten tötet, sein Wachstum hemmt oder seine Proliferation inhibiert.

Im allgemeinen können die erfindungsgemäßen Wirkstoffe, wenn sie für die Behandlung von Tieren eingesetzt werden, direkt angewendet werden. Vorzugsweise werden sie als pharmazeutische Zusammensetzungen angewendet, die im Stand der Technik bekannte pharmazeutisch unbedenkliche Exzipienten und/oder Hilfsstoffe enthalten können.

Die Anwendung (= Verabreichung) der Wirkstoffe im Bereich Tiergesundheit und in der Tierhaltung erfolgt in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subkutan, intravenös, intraperitoneal u.a.), Implantate, durch nasale Applikation, durch dermale Applikation in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw. Die Wirkstoffe können als Shampoo oder als geeignete, in Aerosolen oder drucklosen Sprays, z.B. Pumpsprays und Zerstäubersprays, anwendbare, Formulierungen formuliert werden.

Bei der Anwendung für Nutztiere, Geflügel, Haustiere etc. kann man die erfindungsgemäßen Wirkstoffe als Formulierungen (beispielsweise Pulver, Spritzpulver [wettable powders, "WP"], Emulsionen, Emulsionskonzentrate [emulsifiable concentrates ,"EC"], fließfähige Mittel, homogene Lösungen und Suspensionskonzentrate [suspension concentrates, "SC"], die die Wirkstoffe in einer Menge von 1 bis 80 Gew.-% enthalten, direkt oder nach Verdünnung (z.B. 100- bis 10 000facher Verdünnung) anwenden oder sie als chemisches Bad verwenden.

Beim Einsatz im Bereich Tiergesundheit können die erfindungsgemäßen Wirkstoffe in Kombination mit geeigneten Synergisten oder anderen Wirkstoffen wie beispielsweise Akariziden, Insektiziden, Anthelmintika, Mittel gegen Protozoen, verwendet werden.

Die erfindungsgemäßen Verbindungen eignen sich insbesondere zur Verwendung im Materialschutz, vorzusweise zum Schutz technischer Materialien, wobei hierunter nicht-lebende Materialien zu verstehen sind, wie vorzugsweise Kunststoffe, Klebstoffe, Leime, Papiere und Kartone, Leder, Holz, Holzverarbeitungsprodukte und Anstrichmittel. Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Die anwendungsfertigen Mittel können gegebenenfalls noch weitere Wirkstoffe zur Bekämpfung von Bewuchs, Insekten, Bakterien oder Pilzen, wie bspw. Insektizide, Herbizide oder Mikrobizide (Fungizide, Antimykotika, Bakterizide, Virizide (einschließlich Mittel gegen Viroide) oder Mittel gegen MLO (Mycoplasma-like-organism) und RLO (Rickettsia-like-organism)) enthalten.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus. Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae. Aus der Ordnung der Araneae z.B. Aviculariidae, Araneidae. Aus der Ordnung der Opiliones z.B. Pseudoscorpiones chelifer, Pseudoscorpiones cheiridium, Opiliones phalangium. Aus der Ordnung der Isopoda z.B. Oniscus asellus, Porcellio scaber. Aus der Ordnung der Diplopoda z.B. Blaniulus guttulatus, Polydesmus spp.. Aus der Ordnung der Chilopoda z.B. Geophilus spp.. Aus der Ordnung der Zygentoma z.B. Ctenolepisma spp., Lepisma saccharina, Lepismodes inquilinus. Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa. Aus der Ordnung der Saltatoria z.B. Acheta domesticus. Aus der Ordnung der Dermaptera z.B. Forficula auricularia. Aus der Ordnung der Isoptera z.B. Kalotermes spp., Reticulitermes spp. Aus der Ordnung der Psocoptera z.B. Lepinatus spp., Liposcelis spp. Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum. Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa. Aus der Ordnung der Lepidoptera z.B. Achroia grisella, Galleria mellonella, Plodia interpunctella, Tinea cloacella, Tinea pellionella, Tineola bisselliella. Aus der Ordnung der Siphonaptera z.B. Ctenocephalides canis, Ctenocephalides felis, Pulex irritans, Tunga penetrans, Xenopsylla cheopis. Aus der Ordnung der Hymenoptera z.B. Camponotus herculeanus, Lasius fuliginosus, Lasius niger, Lasius umbratus, Monomorium pharaonis, Paravespula spp., Tetramorium caespitum. Aus der Ordnung der Anoplura z.B. Pediculus humanus capitis, Pediculus humanus corporis, Pemphigus spp., Phylloera vastatrix, Phthirus pubis. Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die vorliegende Erfindung wird ahand der nachfolgenden Beispiele näher erläutert, welche die Erfindung jedoch keinesfalls beschränken.

### A. Synthesebeispiele

### Herstellung der Verbindung (26)

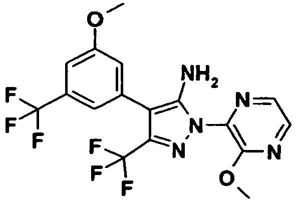

### Schritt 1:

500 mg (1,52 mmol) 2-[3-Methoxy-5-(trifluoromethyl)-phenyl]-4,4,4-trifluoro-3-oxo-butyronitril-Hydrat wurden in 1,16 g (7,60 mmol) Phosphorylchlorid vorgelegt und mit 0,21 ml (1,52 mmol) Triethylamin versetzt. Das Reaktionsgemisch wurde 4 h bei 80-100 °C gerührt und vorsichtig in warmes Wasser eingerührt. Nach dem Extrahieren mit Essigsäurethylester wurde die organische Phase mit Magnesiumsulfat getrocknet, filtriert und im Vakuum eingeengt. Dabei verblieben 366 mg (73 %) 3-Chloro-2-(3-methoxy-5-(trifluoromethyl)-phenyl)-4,4,4-trifluoro-but-2-enenitril.

### Schritt 2:

366 mg (1,11 mmol) 3-Chloro-2-[3-methoxy-5-(trifluoromethylpphenyl]-4,4,4-trifluoro-but-2-enenitril wurden in 10 ml Ethanol vorgelegt und mit 156 mg (1,11 mmol) 3-Methoxy-pyrazin-2-yl-hydrazin sowie 0,155 ml (1,11 mmol) Triethylamin versetzt. Das Gemisch wurde für 10 h unter Rückfluß erhitzt. Nach dem Abkühlen wurden 75 ml Wasser zugegeben und mit Essigsäureethylester extrahiert. Die organische Phase wird vom Lösungsmittel befreit und flashchromatografisch im Cyclohexan/Esigsäureethylester-Gradienten gereinigt. Es verblieben 180 mg (36 %) 1-(3-Methoxypyrazin-2-yl)-4-[3-methoxy-5-(trifluoromethyl)phenyl]-3-(trifluoromethyl)-1H-pyrazol-5-amin.
¹H-NMR (400 MHz, DMSO-d6), δ 8.44 (d, 1H), 8.25 (d,1H), 7.21 - 7.18 (m, 3H), 5.75 (s, 2H, NH₂), 4,00 (s, 3H), 3.88 (s, 3H).

### Herstellung der Verbindungen (107), (108)

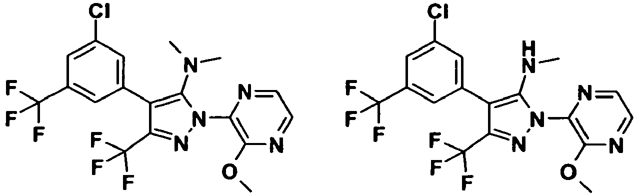

150 mg (0,34 mmol) 1-(3-Methoxypyrazin-2-yl)-4-[3-chlor-5-(trifluoromethyl)phenyl]-3-(trifluoromethyl)-1H-pyrazol-5-amin wurden in 5 mL Acetonitril gelöst und mit 335 mg (1,03 mmol) Cäsiumcarbonat, 4,4 mg (0,02 mmol) Cäsiumiodid und 97,3 mg (0,67 mmol) Iodmethan versetzt. Das Reaktionsgemisch wurde 12 Stunden bei 80 °C gerührt, dann filtriert. Das Filtrat wurde direkt über eine Kieselgelkartusche am Cyclohexan/Essigester-Gradienten chromatographiert. Die gereinigten Fraktionen ergaben das mono- und das bisalkylierte Produkt.
53 % (107) 1-(3-Methoxypyrazin-2-yl)-4-[3-chlor-5-(trifluoromethyl)phenyl]-3-(trifluoromethyl)-1H-pyrazol-5-dimethylamin
¹H-NMR (400 MHz, DMSO-d6), δ 8.47 (d, 1H), 8.27 (d, 1H), 7.82 (m, 1H), 7.75 (m, 1H), 7.66 (m, 1H), 5.82 (m, 1H), 4.01 (s, 3H), 2.33 (d, 3H).
33 % (108) 1-(3-Methoxypyrazin-2-yl)-4-[3-chlor-5-(trifluoromethyl)phenyl]-3-(trifluoromethyl)-1H-pyrazol-5-methylamin
¹H-NMR (400 MHz, DMSO-d6), δ 8.52 (d, 1H), 8.33 (d, 1H), 7.88 (m, 1H), 7.81 (m, 1H), 7.72 (m, 1H), 4.01 (s, 3H), 2.44 (s, 6H).

Mit analogen Verfahrensweisen wurde erhalten:

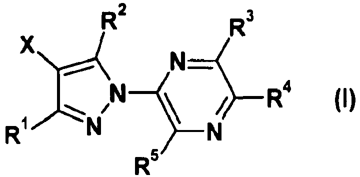

**Tabelle 1**

| Bsp | R¹ | R² | R³ | R⁴ | R⁵ | X | Log p |
|---|---|---|---|---|---|---|---|
| 1 | CF₃ | Amino | H | H | Chlor | 3,4-Dichlorphenyl | 3,99**; 4,02_{*} |
| 2 | CF₃ | Amino | H | H | Methoxy | 4-Bromphenyl | 3,4*** |
| 3 | 1-Methoxypropyl | Amino | H | H | Methoxy | 3,4-Dichlorphenyl | |
| 4 | CF₃ | Amino | H | H | Propoxy | 4-Bromphenyl | 4,11** |
| 5 | CF₃ | Amino | H | H | Ethoxy | 4-Bromphenyl | 3,6** |
| 6 | CF₃ | Amino | H | H | Methoxy | 3,5-Dichlorphenyl | 3,95* |
| 7 | CF₃ | Amino | H | H | Chlor | 4-Chlorphenyl | 3,59* |
| 8 | Ethenyl | Amino | H | H | Methoxy | 3,5-Dichlorphenyl | 3,51* |
| 9 | Ethenyl | Amino | H | H | Methoxy | 3-Chlor-5-(trifluormethyl)phenyl | 3,63* |
| 10 | CF₃ | Amino | H | H | Methoxy | 3-Chlor-5-(trifluormethyl)phenyl | 4,07* |
| 11 | Chlor(difluor)methyl | Amino | H | H | Methoxy | 3,5-Bis(trifluormethyl)phenyl | |
| 12 | CF₃ | Amino | H | H | Ethoxy | 3-Chlor-5-(trifluormethyl)phenyl | 4,47* |
| 13 | CF₃ | Amino | H | H | Propan-2-yloxy | 3-Chlor-5-(trifluormethyl)phenyl | 4,84* |
| 14 | CF₃ | Amino | H | H | Methoxy | 3,5-Bis(trifluormethyl)phenyl | 4,2* |
| 15 | Pentafluorethyl | Amino | H | H | Methoxy | 3-Chlor-5-(trifluormethyl)phenyl | 4,58* |
| 16 | CF₃ | Amino | H | H | Chlor | 3-Chlor-5-(trifluormethyl)phenyl | 4,28* |
| 17 | Chlor(difluor)methyl | Amino | H | H | Methoxy | 3-Chlor-5-(trifluormethyl)phenyl | 4,24* |
| 18 | CF₃ | Amino | H | H | Methoxy | 4-Chlor-3-(trifluormethyl)phenyl | 3,94* |
| 19 | CF₃ | Amino | Chlor | H | H | 3-Chlor-5-(trifluormethyl)phenyl | 5,26* |
| 20 | 1,1-Difluorethyl | Amino | H | H | Methoxy | 4-Chlor-3-(trifluormethyl)phenyl | 3,74* |
| 21 | Pentafluorethyl | Amino | H | H | Methoxy | 3-Fluor-5-(trifluormethyl)phenyl | 4,23* |
| 22 | CF₃ | Amino | H | H | Methoxy | 3,5-Dibromphenyl | 4,1* |
| 23 | CF₃ | Amino | H | H | CH₃ | 3-Chlor-5-(trifluormethyl)phenyl | 4,32* |
| 24 | CF₃ | Amino | CH₃ | H | CH₃ | 3-Chlor-5-(trifluormethyl)phenyl | 4,56* |
| 25 | CF₃ | Amino | H | H | Dimethylamino | 3-Chlor-5-(trifluormethyl)phenyl | 4,28* |
| 26 | CF₃ | Amino | H | H | Methoxy | 3-Methoxy-5-(trifluormethyl)phenyl | 3,63* |
| 27 | CF₃ | Amino | H | H | Cyan | 3-Chlor-5-(trifluormethyl)phenyl | 3,93* |
| 28 | CF₃ | Amino | H | H | Methylsulfanyl | 3-Chlor-5-(trifluormethyl)phenyl | 4,9* |
| 29 | CF₃ | Amino | H | H | Methylsulfinyl | 3-Chlor-5-(trifluormethyl)phenyl | 3,29* |
| 30 | CF₃ | Amino | H | H | Methylsulfonyl | 3-Chlor-5-(trifluormethyl)phenyl | 3,54* |
| 31 | CF₃ | Amino | H | H | 1H-Pyrazol-1-yl | 3-Chlor-5-(trifluormethyl)phenyl | 3,94* |
| 32 | CF₃ | Dibenzylamino | H | H | Methoxy | 3-Chlor-5-(trifluormethyl)phenyl | 6,67* |
| 33 | CF₃ | Benzylamino | H | H | Methoxy | 3-Chlor-5-(trifluormethyl)phenyl | 5,28* |
| 34 | CF₃ | Dibenzylamino | H | H | Ethoxy | 3-Chlor-5-(trifluormethyl)phenyl | 7* |
| 35 | CF₃ | Benzylamino | H | H | Ethoxy | 3-Chlor-5-(trifluormethyl)phenyl | 5,62* |
| 36 | CF₃ | Bis(4-chlorbenzyl)amino | H | H | Ethoxy | 3-Chlor-5-(trifluormethyl)phenyl | 7,36** |
| 37 | CF₃ | (4-Chlorbenzyl)amino | H | H | Ethoxy | 3-Chlor-5-(trifluormethyl)phenyl | 5,84* |
| 38 | CF₃ | Bis(4-chlorbenryl)amino | H | H | Methoxy | 3-Chlor-5-(trifluormethyl)phenyl | 7,11* |
| 39 | CF₃ | (4-Chlorbenzyl)amino | H | H | Methoxy | 3-Chlor-5-(trifluormethyl)phenyl | 5,51* |
| 40 | CF₃ | (4-Methoxybenzyl)amino | H | H | Ethoxy | 3-Chlor-5-(trifluormethyl)phenyl | 5,47* |
| 41 | CF₃ | (4-Methoxybenryl)amino | H | H | Methoxy | 3-Chlor-5-(trifluormethyl)phenyl | 5,13* |
| 42 | CF₃ | Bis(4-methoxybenzyl)amino | H | H | Methoxy | 3-Chlor-5-(trifluormethyl)phenyl | 6,25* |
| 43 | CF₃ | Bis(4-methoxybenzyl)amino | H | H | Ethoxy | 3-Chlor-5-(trifluormethyl)phenyl | 6,57* |
| 44 | CF₃ | Amino | H | H | 1H-Imidazol-1-yl | 3-Chlor-5-(trifluormethyl)phenyl | 2,76* |
| 45 | CF₃ | Amino | H | H | 4-Fluor-1H-pyrazol-1-yl | 3-Chlor-5-(trifluormethyl)phenyl | 4,24* |
| 46 | Heptafluorpropyl | Amino | H | H | Methoxy | 3-Chlor-5-(trifluormethyl)phenyl | 4,91* |
| 47 | 1, 1,2,2-Tetrafluor-2-methoxyethyl | Amino | H | H | Methoxy | 3-Chlor-5-(trifluormethyl)phenyl | 3,98* |
| 48 | CF₃ | Amino | H | H | Methoxy | 7-Chlor-1,3-benzodioxol-5-yl | 3,14* |
| 49 | CF₃ | Amino | H | H | Methoxy | Pyridin-2-yl | 2,59* |
| 50 | CF₃ | Amino | H | H | Methoxy | 5-Brompyridin-3-yl | 2,62* |
| 51 | Pentafluorethyl | Amino | H | H | Methoxy | 3-Chlor-4,5-dimethoxyphenyl | 3,8* |
| 52 | CF₃ | Amino | H | H | Methoxy | 3,5-Dichlorpyridin-2-yl | 2,94* |
| 53 | CF₃ | Amino | H | H | Methoxy | 5-Chlorpyridin-2-yl | 3,55* |
| 54 | Pentafluorethyl | Amino | 1H | H | Methoxy | 5-Chlorpyridin-2-yl | 4,17* |
| 55 | CF₃ | Amino | H | H | Methoxy | 6-Chlor-4-(trifluormethyl)pyridin-2-yl | 4,54* |
| 56 | CF₃ | Amino | H | H | Chlor | 3,5-Dichlorphenyl | 4,16* |
| 57 | CF₃ | Amino | H | H | Methoxy | 2-Chlorpyridin-3-yl | 2,19* |
| 58 | Pentafluorethyl | Amino | H | H | Methoxy | 6-Chlorpyridin-3-yl | 3,14* |
| 59 | 1, 1,2,2-Tetrafluorethyl | Amino | H | H | Methoxy | 3-Chlor-5-(trifluormethyl)phenyl | 4,05* |
| 60 | 1,1,2,2-Tetrafluorethyl | Amino | H | H | Ethoxy | 3-Chlor-5-(trifluormethyl)phenyl | |
| 61 | 1,1,2,2-Tetrafluorethyl | (Pyrazin-2-ylmethyl)amino | H | H | Methoxy | 3-Chlor-5-(trifluormethyl)phenyl | |
| 62 | 1,1,2,2-Tetrafluorethyl | (2-Methylprop-2-en-1-yl)amino | H | H | Methoxy | 3-Chlor-5-(trifluormethyl)phenyl | |
| 63 | 1,1,2,2-Tetrafluorethyl | Prop-2-en-1-ylamino | H | H | Methoxy | 3-Chlor-5-(trifluormethyl)phenyl | |
| 64 | 1,1,2,2-Tetrafluorethyl | (Pyridin-2-ylmethyl)amino | H | H | Methoxy | 3-Chlor-5-(trifluormethyl)phenyl | |
| 65 | 1,1,2,2-Tetrafluorethyl | Amino | H | H | Methoxy | 7-Chlor-1,3-benzodioxol-5-yl | 3,18* |
| 66 | 1,1,2,2-Tetrafluorethyl | Amino | H | H | Methoxy | 7-Chlor-2,3-dihydro-1-benzofur-5-yl | 3,22* |
| 67 | 1,1,2,2-Tetrafluorethyl | Amino | H | H | Methoxy | 7-(Trifluormethyl)-1,3-benzodioxol-5-yl | |
| 68 | 1,1,2,2-Tetrafluorethyl | Amino | H | H | Methoxy | 7-Brom-1,3-benzodioxol-5-yl | |
| 69 | Pentafluorethyl | Amino | H | H | Methoxy | 7-Chlor-2,3-dihydro-1-benzofur-5-yl | |
| 70 | Pentafluorethyl | Amino | H | H | Methoxy | 7-(Trifluormethyl)-1,3-benzodioxol-5-yl | |
| 71 | Pentafluorethyl | Amino | H | H | Methoxy | 7-Brom-1,3-benzodioxol-5-yl | |
| 72 | CF₃ | Amino | H | H | Methoxy | 7-Chlor-2,3-dihydro-1-benzofur-5-yl | |
| 73 | CF₃ | Amino | H | H | Methoxy | 7-(Trifluormethyl)-1,3-benzodioxol-5-yl | |
| 74 | CF₃ | Amino | H | H | Methoxy | 7-Brom-1,3-benzodioxol-5-yl | 3,18* |
| 75 | CH₃ | Amino | H | H | Methoxy | 7-Chlor-1,3-benzodioxol-5-yl | 2,26* |
| 76 | Ethyl | Amino | H | H | Methoxy | 7-Chlor-1,3-benzodioxol-5-yl | 2,57* |
| 77 | Propan-2-yl | Amino | H | H | Methoxy | 7-Chlor-1,3-benzodioxol-5-yl | 2,96* |
| 78 | tert-Butyl | Amino | H | H | Methoxy | 7-Chlor-1,3-benzodioxol-5-yl | |
| 79 | Cyclopropyl | Amino | H | H | Methoxy | 7-Chlor-1,3-benzodioxol-5-yl | 2,79* |
| 80 | CH₃ | Amino | H | H | Methoxy | 7-Chlor-2,3-dihydro-1-benzofur-5-yl | |
| 81 | Ethyl | Amino | H | H | Methoxy | 7-Chlor-2,3-dihydro-1-benzofur-5-yl | |
| 82 | Propan-2-yl | Amino | H | H | Methoxy | 7-Chlor-2,3-dihydro-1-benzofur-5-yl | |
| 83 | tert-Butyl | Amino | H | H | Methoxy | 7-Chlor-2,3-dihydro-1-benzofur-5-yl | |
| 84 | Cyclopropyl | Amino | H | H | Methoxy | 7-Chlor-2,3-dihydro-1-benzofur-5-yl | |
| 85 | tert-Butyl | Amino | H | H | Methoxy | 3-Chlor-5-(trifluormethyl)phenyl | |
| 86 | CF₃ | Amino | H | H | Methoxy | 3,4-Dichlor-5-(trifluormethyl)phenyl | 4,47** |
| 87 | CF₃ | Amino | H | H | Methoxy | 3,4,5-Trichlorphenyl | 4,35* |
| 88 | CF₃ | Amino | H | H | Methoxy | 3,5-Dichlor-4-methoxyphenyl | |
| 89 | CF₃ | Amino | H | H | Methoxy | 3,4-Dimethoxy-5-(trifluormethyl)phenyl | 3,56* |
| 90 | CF₃ | Amino | H | H | Methoxy | 3,5-Dichlor-4-methylphenyl | |
| 91 | CF₃ | (Pyridin-2-ylmethyl)amino | H | H | Methoxy | 7-Chlor-1,3-benzodioxol-5-yl | |
| 92 | CF₃ | (Pyrazin-2-ylmethyl)amino | H | H | Methoxy | 7-Chlor-1,3-benzodioxol-5-yl | |
| 93 | CF₃ | Prop-2-en-1-ylamino | H | H | Methoxy | 7-Chlor-1,3-benzodioxol-5-yl | |
| 94 | CF₃ | (2-Methylprop-2-en-1-yl)amino | H | H | Methoxy | 7-Chlor-1,3-benzodioxol-5-yl | |
| 95 | CF₃ | (Pyrazin-2-ylmethyl)amino | H | H | Methoxy | 3-Chlor-5-(trifluormethyl)phenyl | |
| 96 | CF₃ | (2-Methylprop-2-en-1-yl)amino | H | H | Methoxy | 3-Chlor-5-(trifluormethyl)phenyl | |
| 97 | CF₃ | Prop-2-en-1-ylamino | H | H | Methoxy | 3-Chlor-5-(trifluormethyl)phenyl | |
| 98 | CF₃ | (Pyridin-2-ylmethyl)amino | H | H | Methoxy | 3-Chlor-5-(trifluormethyl)phenyl | |
| 99 | Pentafluorethyl | (Pyrazin-2-ylmethyl)amino | H | H | Methoxy | 3.Chlor-5-(trifluormethyl)phenyl | |
| 100 | Pentafluorethyl | (2-Methylprop-2-en-1-yl)amino | H | H | Methoxy | 3-Chlor-5-(trifluormethyl)phenyl | |
| 101 | Pentafluorethyl | Prop-2-en-1-ylamino | H | H | Methoxy | 3-Chlor-5-(trifluormethyl)phenyl | |
| 102 | Pentafluorethyl | (Pyridin-2-ylmethyl)amino | H | H | Methoxy | 3-Chlor-5-(trifluormethyl)phenyl | |
| 103 | CF₃ | Diprop-2-en-1-ylamino | H | H | Methoxy | 3-Chlor-5-(trifluormethyl)phenyl | 4,08* |
| 104 | CF₃ | Bis(2-methylprop-2-en-1-yl)amino | H | H | Methoxy | 3-Chlor-5-(trifluormethyl)phenyl | 5,22* |
| 105 | CF₃ | Diprop-2-in-1-ylamino | H | H | Methoxy | 3-Chlor-5-(trifluormethyl)phenyl | 4,91* |
| 106 | CF₃ | Methylamino | H | H | Methoxy | 3-Chlor-5-(trifluormethyl)phenyl | 4,51** |
| 107 | CF₃ | Dimethylamino | H | H | Methoxy | 3-Chlor-5-(trifluormethyl)phenyl | 5,11* |
| 108 | CF₃ | Bis[(6-methylpyridin-2-yl)methyl]amino | H | H | Methoxy | 3-Chlor-5-(trifluormethyl)phenyl | 3,79* |
| 109 | Difluormethyl | Amino Amino | H | H | Methoxy | 7-Chlor-1,3-benzodioxol-5-yl | |
| 110 | 1-Fluorethyl | Amino | H | H | Methoxy | 7-Chlor-1,3-benzodioxol-5-yl | |
| 111 | Propyl | Amino | H | H | Methoxy | 7-Chlor-1,3-benzodioxol-5-yl | |
| 112 | Cyan | Amino | H | H | Methoxy | 7-Chlor-1,3-benzodioxol-5-yl | |
| 113 | Dimethoxymethyl | Amino | H | H | Methoxy | 7-Chlor-1,3-benzodioxol-5-yl | |
| 114 | (Z)-(Methoxyimino)methyl | Amino | H | H | Methoxy | 7-Chlor-1,3-benzodioxol-5-yl | |
| 115 | (Z)-(Hydroxyimino)methyl | Amino | H | H | Methoxy | 7-Chlor-1,3-benzodioxol-5-yl | |
| 116 | 1,1,2,2-Tetrafluorethyl | Amino | H | H | Methoxy | 3,4,5-Trichlorphenyl | 4,37** |
| 117 | CF₃ | Amino | H | H | Methoxy | 7-Brom-2,3-dihydro-1-benzofur-5-yl | |
| 118 | CF₃ | Amino | H | H | Methoxy | 7-Chlor-2-methyl-2,3-dihydro-1-benzofur-5-yl | |
| 119 | CF₃ | Amino | H | H | Methoxy | 1-Chlor-2,2-dimethyl-2,3-dihydro-1-benzofur-5-yl | |
| 120 | CF₃ | Amino | H | H | Methoxy | 7-Chlor-2,2-dimethyl-1,3-benzodioxol-5-Y1 | |
| 121 | CF₃ | Amino | H | H | Methoxy | 3,5-Dichlor-4-(dimethylamino)phenyl | 4,46* |
| 122 | CF₃ | Amino | H | H | Methoxy | 3-Chlor-4-(dimethylamino)phenyl | 3,19* |
| 123 | CF₃ | Amino | H | H | Methoxy | 3-Chlor-4-(dimethylamino)-5-(trifluormethyl)phenyl | 4,72* |
| 124 | CF₃ | Amino | H | H | Methoxy | 3-Chlor-5-fluor-4-methoxyphenyl | 3,5***; 3,47* |
| 125 | CF₃ | Amino | H | H | Methoxy | 3-Methoxy-4-(2,2,2-trifluorethoxy)phenyl | 3,07* |
| 126 | CF₃ | Amino | H | H | Methoxy | 3-Methoxy-4-(propan-2-yloxy)-5-(trifluormethyl)phenyl | 4,28* |
| 127 | CF₃ | Amino | H | H | Methoxy | 3-Ethoxy-4-(2,2,2-trifluorethoxy)phenyl | 3,68* |
| 128 | CF₃ | Amino | H | H | Methoxy | 2-Brom-3,4-dimethoxyphenyl | 2,87* |
| 129 | CF₃ | Amino | H | H | Brom | 3-Chlor-5-(trifluormethyl)phenyl | 4,27* |
| 130 | CF₃ | Amino | H | H | CF₃ | 3-Chlor-5-(trifluormethyl)phenyl | 4,57* |
| 131 | CF₃ | Amino | H | H | Methoxy | 9-Brom-3,4-dihydro-2H-1,5-benzodioxepin-7-yl | 3,32* |
| 132 | CF₃ | Amino | H | H | Methoxy | 8-Brom-2,3-dihydro-1,4-benzodioxin-6-yl | 3,09* |
| 133 | CF₃ | Amino | H | H | Methoxy | 8-Chlor-2,3-dihydro-1,4-benzodioxin-6-yl | 3,02* |
| 134 | CF₃ | Amino | H | H | Methoxy | 3-Chlor-5-methoxy-4-(2,2,2-trifluorethoxy)phenyl | 3,88* |
| 135 | CF₃ | Amino | H | H | Methoxy | 8-Chlor-4H-1,3-benzodioxin-6-yl | 3,02* |
| 136 | CF₃ | Amino | H | H | Methoxy | 9-Chlor-3,4-dihydro-2H-1,5-benzodioxepin-7-yl | 3,25* |
| 137 | CF₃ | Amino | H | H | Methoxy | 2-Bromphenyl | 3* |
| 138 | CF₃ | Amino | H | H | Methoxy | 2-Iodphenyl | 3,09* |
| 139 | CF₃ | Amino | H | H | Methoxy | 7-Iod-1,3-benzodioxol-5-yl | 3,27* |
| 140 | CF₃ | Amino | H | H | Methoxy | 3-Brom-5-ethoxy-4-(2,2,2-trifluorethoxy)phenyl | 4,3* |
| 141 | CF₃ | Amino | H | H | Methoxy | 3-Chlor-4,5-dimethoxyphenyl | 3,22* |
| 142 | CF₃ | Amino | H | H | Methoxy | 2,3-Dichlor-6-fluorphenyl | 3,31* |
| 143 | CF₃ | Amino | H | H | Methoxy | 6-Chlor-1,3-benzodioxol-5-yl | 2,86* |
| 144 | CF₃ | Amino | H | H | Methoxy | 3-Chlor-5-ethoxy-4-(2,2,2-trifluorethoxy)phenyl | 4,21* |
| 145 | CF₃ | Amino | H | H | Methoxy | 2,2-Dimethyl-3,4-dihydro-2H-chromen-6-yl | 3,68* |
| 146 | CF₃ | Amino | H | H | Methoxy | 3-Brom-4,5-dimethoxyphenyl | |
| 147 | CF₃ | Amino | H | H | Methoxy | 3-Iod-4,5-dimethoxyphenyl | 3,47* |
| 148 | CF₃ | Amino | H | H | Methoxy | 3-Fluor-4,5-dimethoxyphenyl | |
| 149 | CF₃ | Amino | H | H | Methoxy | 3-Cyan-4,5-dimethoxyphenyl | |
| 150 | CF₃ | Amino | H | H | Methoxy | 7-Cyan-1,3-benzodioxol-5-yl | 2,66* |
| 151 | CF₃ | Amino | H | H | Methoxy | 7-Cyan-2,3-dihydro-1-benzofur-5-yl | |
| 152 | 2-Fluorpropan-2-yl | Amino | H | H | Methoxy | 3,4-Dimethoxy-5-(trifluormethyl)phenyl | |
| 153 | CH₃ | Amino | H | H | Methoxy | 3,4-Dimethoxy-5-(trifluormethyl)phenyl | |
| 154 | Ethyl | Amino | H | H | Methoxy | 3,4-Dimethoxy-5-(trifluormethyl)phenyl | |
| 155 | Propan-2-yl | Amino | H | H | Methoxy | 3,4-Dimethoxy-5-(trifluormethyl)phenyl | |
| 156 | Cyclopropyl | Amino | H | H | Methoxy | 3,4-Dimethoxy-5-(trifluormethyl)phenyl | |
| 157 | 1,1,2,2-Tetrafluorethyl | Amino | H | H | Methoxy | 3,4-Dimethoxy-5-(trifluormethyl)phenyl | 3,58* |
| 158 | Pentafluorethyl | Amino | H | H | Methoxy | 3,4-Dimethoxy-5-(trifluormethyl)phenyl | |
| 159 | 2-Fluorpropan-2-yl | Amino | H | H | Methoxy | 3-Chlor-4,5-dimethoxyphenyl | |
| 160 | CH₃ | Amino | H | H | Methoxy | 3-Chlor-4,5-dimethoxyphenyl | |
| 161 | Ethyl | Amino | H | H | Methoxy | 3-Chlor-4,5-dimethoxyphenyl | 2,66* |
| 162 | Propan-2-yl | Amino | H | H | Methoxy | 3-Chlor-4,5-dimethoxyphenyl | |
| 163 | Cyclopropyl | Amino | H | H | Methoxy | 3-Chlor-4,5-dimethoxyphenyl | |
| 164 | 1,1,2,2-Tetrafluorethyl | Amino | H | H | Methoxy | 3-Chlor-4,5-dimethoxyphenyl | |
| 165 | CF₃ | Amino | H | H | Methoxy | 2,4-Dichlor-3-methylsulfanylphenyl | 4,14* |
| 166 | CF₃ | Amino | H | H | Methoxy | 2,4-Dichlor-5-fluorphenyl | 3,66* |
| 167 | Prop-1-en-2-yl | Amino | H | H | Methoxy | 3,5-Dichlorphenyl | 3,79* |
| 168 | 2-Fluorpropan-2-yl | Amino | H | H | Methoxy | 3,5-Dichlorphenyl | 3,77* |
| 169 | Difluorchlormethyl | Amino | H | H | Methoxy | 7-Chlor-1,3-benzodioxol-5-yl | 3,26* |
| 170 | Ethyl | Amino | H | H | Ethoxy | 7-Chlor-1,3-benzodioxol-5-yl | 2,96* |
| 171 | CF₃ | Amino | H | H | Ethoxy | 7-Chlor-1,3-benzodioxol-5-yl | 3,48* |
| 172 | CF₃ | Amino | H | H | Ethoxy | 7-Cyano-1,3-benzodioxol-5-yl | 3,01* |
| 173 | CF₃ | Amino | H | H | Methoxy | 8-(Trifluormethyl)-2,3-dihydro-1,4-benzdioxin-6-yl | 3,27* |
| 174 | CF₃ | Amino | H | H | Methoxy | 7-Fluor-1,3-benzodioxol-5-yl | 2,91* |
| 175 | CF₃ | Amino | H | H | Ethoxy | 7-Fluor-1,3-benzodioxol-5-yl | 3,27* |
| 176 | CF₃ | Amino | H | H | Methoxy | 3-Iod-4,5-dimethoxyphenyl | 3,87* |
| 177 | Ethyl | Amino | H | H | Ethoxy | 7-Brom-1,3-benzodioxol-5-yl | 3,03* |
| 178 | Ethyl | Amino | H | H | Methoxy | 7-Brom-1,3-benzodioxol-5-yl | 2,65* |
| 179 | CF₃ | Amino | H | H | Methoxy | 2,4-Dichlor-5-[(2,2,2-trifluorethyl)sulfanyl]phenyl | 4,14* |
| 180 | CF₃ | Amino | H | H | Methoxy | 3,4-Dichlor-5-[(2,2,2-trifluorethyl)sulfanyl]phenyl | 4,43* |
| 181 | CF₃ | Amino | H | H | Methoxy | 2-Chlor-4-fluor-5-[(2,2,2-trifluorethyl) sulfanyl]phenyl | |
| 182 | CF₃ | Amino | H | H | Methoxy | 3,4-Dichlor-5-[(2,2,2-triluorethyl)sulfinyl]phenyl | 3,69* |
| | | | | | | | |

| | | | | | | | |
|---|---|---|---|---|---|---|---|
| Die Bestimmung der logP Werte erfolgte gemäß EEC Directive 79/831 Annex V.A8 durch HPLC (High Performance Liquid Chromatography) an reversed-phase Säulen (C 18), mit nachfolgenden Methoden: * Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril ** Die Bestimmung erfolgt im sauren Bereich bei pH 2.3 mit 0,1% wässriger Phosphorsäure und Acetonitril als Eluenten; linearer Gradient von 10% Acetonitril bis 95% Acetonitril. *** Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. Die Eichung erfolgt mit unverzweigten Alkan-2-onen (mit 3 bis 16 Kohlenstoffatomen), deren logP-Werte bekannt sind (Bestimmung der IogP-Werte anhand der Retentionszeiten durch lineare Interpolation zwischen zwei aufeinander folgenden Alkanonen). Die lambda-maX Werte wurden an Hand der UV-Spektren von 200 nm bis 400 nm in den Maxima der chromatographischen Signale ermittelt. | | | | | | | |

### NMR-Daten:

Bsp. 2: ¹H-NMR (300 MHz, CDCl₃), δ 8.21 (d, 1H), 8.08 (d, 1H), 7.58 (d, 2H), 7.28 (d, 2H), 4.71 (bs, 2H, NH₂), 4.10 (s, 3H).
Bsp. 3: ¹H-NMR (300 MHz, CDCl₃), δ 8.14 (d, 1H), 8.05 (d, 1H), 7.58 (d, 1H, 7.47 (d, 1H), 7.34 (dd, 1H), 4.60 (bs, 2H, NH₂), 4.21 (tr, 1H), 4.08 (s, 3H), 3.36 (s, 3H), 1.71 (m, 2H), 0.87 (tr, 3H).
Bsp. 4: ¹H-NMR (300 MHz, CDCl₃), δ 8.17 (d, 1H), 8.05 (d, 1H), 7.59 (d, 2H), 7.29 (d, 2H), 4.72 (bs, 2H, NH₂), 4.42 (tr, 2H), 1.85 (m, 2H), 1.07 (tr, 3H).
Bsp. 5: ¹H-NMR (400 MHz, DMSO-d6), δ 8.44 (d, 1H), 8.25 (d, 1H), 7.63 (d, 2H), 7.31 (d, 2H), 5.84 (bs, 2H, NH₂), 4.46 (qu, 2H), 1.30 (tr, 3H).
Bsp. 6: ¹H-NMR (400 MHz, DMSO-d6), δ 8.45 (d, 1H), 8.26 (d, 1H), 7.53 (s, 1H), 7.35 (s, 2H), 5.86 (bs, 2H, NH₂), 4.00 (s, 3H).
Bsp. 7: ¹H-NMR (400 MHz, DMSO-d6), δ 8.73 (s, 2H), 7.50 (d, 2H), 7.39 (d, 2H), 5.87 (bs, 2H, NH₂).
Bsp. 8: ¹H-NMR (400 MHz, DMSO-d6), δ 8.23 (d, 1H), 8.12 (d, 1H), 7.37 (s, 1H), 7.34 (s, 2H), 6.61- 6.53 (dd, 1H), 5.73 - 5.68 (dd, 1H), 5.28 - 5.25 (dd, 1H), 4.71 (bs, 2H, NH₂), 4.03 (s, 3H).
Bsp. 9: ¹H-NMR (400 MHz, DMSO-d6), δ 8.26 (d, 1H), 8.15 (d, 1H), 7.66 (s, 2H), 7.62 (s, 1H), 6.61 - 6.54 (dd, 1H), 5.73 - 5.68 (dd, 1H), 5.29 - 5.26 (dd, 1H), 4.83 (bs, 2H, NH₂), 4.02 (s, 3H).
Bsp. 10: ¹H-NMR (400 MHz, DMSO-d6), δ 4.04 (s, 3H), 5.91 (bs, 2H), 7.59 (s, 1H), 7.68 (s, 1H), 7.76 (s, 1H), 8.26 (d, 1H), 8.45 (d, 1H).
Bsp. 11: ¹H-NMR (400 MHz, CDCl3), δ 7.88 (m, 2H), 7.86 (m, 1H), 7.83 (d, 1H), 7.59 (d, 1H), 6.06 (bs, 2H, NH₂), 4.27 (s, 3H).
Bsp. 12: ¹H-NMR (400 MHz, DMSO-d6), δ 1.33 (t, 3H), 4.49 (q, 2H), 5.91 (bs, 2H), 7.59 (s, 1H), 7.68 (s, 1H); 7.76 (s, 1H); 8.24 (d, 1H); 8.42 (d, 1H).
Bsp. 13: ¹H-NMR (400 MHz, DMSO-d6), δ 1.33 (d, 6H), 5.34 (m, 1H), 5.90 (bs, 2H), 7.58 (s, 1H), 7.67 (s, 1H), 7.76 (s, 1H), 8.22 (d, 1H), 8.41 (d, 1H).
Bsp. 14: ¹H-NMR (400 MHz, DMSO-d6), δ 4.01 (s, 3H), 5.97 (bs, 2H), 7.95 (s, 2H), 8.00 (s, 1H), 8.27 (d, 1H), 8.46 (d, 1H).
Bsp. 15: ¹H-NMR (400 MHz, DMSO-d6), δ 4.00 (s, 3H), 5.85 (bs, 2H), 7.57 (s, 1H), 7.66 (s, 1H), 7.77 (s, 1H), 8.27 (d, 1H), 8.45 (d, 1H).
Bsp. 16: ¹H-NMR (400 MHz, DMSO-d6), δ 6.14 (bs, 2H), 7.60 (s, 1H), 7.69 (s, 1H), 7.78 (s, 1H), 8.75 (s, 2H).
Bsp. 17: ¹H-NMR (400 MHz, DMSO-d6), δ 4.01 (s, 3H), 5.,85 (bs, 2H), 7.61 (s, 1H), 7.69 (s, 1H), 7.76 (s, 1H), 8,26 (d, 1H), 8.45 (d, 1H).
Bsp. 18: ¹H-NMR (400 MHz, DMSO-d6), δ 4.,00 (s, 3H), 5.85 (bs, 2H), 7.65 (dd, 1H), 7.73-7.77 (m, 2H), 8.26 (d, 1H), 8.45 (d, 1H).
Bsp. 19: ¹H-NMR (400 MHz, DMSO-d6), δ 6.78 (bs, 2H), 7.60 (s, 1H), 7.70 (s, 1H), 7.82 (s, 1H), 8.78 (d, 1H), 9.13 (d, 1H).
Bsp. 20: ¹H-NMR (400 MHz, DMSO-d6), δ 1.92 (t, 3H), 3.99 (s, 3H), 5.60 (bs, 2H), 7.66-7.72 (m, 2H), 7.79 (s, 1H), 8.24 (d, 1H), 8.40 (d, 1H).
Bsp. 21: ¹H-NMR (400 MHz, DMSO-d6), δ 4.00 (s, 3H), 5.83 (bs, 2H), 7.46-7.48 (m, 2H), 7.58 (d, 1H), 8.27 (d, 1H), 8.45 (d, 1H).
Bsp. 22: ¹H-NMR (400 MHz, DMSO-d6), δ 4.00 (s, 3H), 5.84 (bs, 2H), 7.51 (d, 2H), 7.76 (t, 1H), 8.26 (d, 1H), 8.46 (d, 1H).
Bsp. 23: ¹H-NMR (400 MHz, DMSO-d6), δ 2.53 (s, 3H), 6.03 (bs, 2H), 7.61 (s, 1H), 7.70 (s, 1H), 7.78 (s, 1H), 8.53 (d, 1H), 8.71 (d, 1H).
Bsp. 24: ¹H-NMR (400 MHz, DMSO-d6), δ 2.46 (s, 3H), 2.54 (s, 3H), 5.97 (bs, 2H), 7.61 (s, 1H), 7.71 (s, 1H), 7.77 (s, 1H), 8.60 (s, 1H).
Bsp. 25: ¹H-NMR (400 MHz, DMSO-d6), δ 2.83 (s, 6H), 5.89 (bs, 2H), 7.57 (s, 1H), 7.66 (s, 1H), 7.76 (s, 1H), 7.87 (d, 1H), 8.30 (d, 1H).
Bsp. 27: ¹H-NMR (400 MHz, DMSO-d6), δ 7.81 (s, 2H), 7.94 (s, 1H), 8.06 (bs, 2H), 8.98 (d, 1H), 9.06 (d, 1H).
Bsp. 28: ¹H-NMR (400 MHz, DMSO-d6), δ 2.52 (s, 3H), 6.18 (bs, 2H), 7.59 (s, 1H), 7.69 (s, 1H), 7.78 (s, 1H), 8.35 (d, 1H), 8.68 (d, 1H).
Bsp. 29: ¹H-NMR (400 MHz, DMSO-d6), δ 2.93 (s, 3H), 6.74 (bs, 2H), 7.61 (s, 1H), 7.71 (s, 1H), 7.81 (s, 1H), 8.74 (d, 1H), 8.93 (d, 1H).
Bsp. 30: ¹H-NMR (400 MHz, DMSO-d6), δ 3.40 (s, 3H), 6.14 (bs, 2H), 7.57 (s, 1H), 7.66 (s, 1H), 7.78 (s, 1H), 9.03 (d, 1H), 9.05 (d, 1H).
Bsp. 31: ¹H-NMR (400 MHz, DMSO-d6), δ 5.90 (bs, 2H), 6.54 (dd, 1H), 7.54 (s, 1H), 7.62 (s, 1H), 7.68 (dd, 1H), 7.75 (s, 1H), 8.35 (dd, 1H), 8.73 (d, 1H), 8.81 (d, 1H).
Bsp. 32: ¹H-NMR (400 MHz, DMSO-d6), δ 3.87 (s, 4H), 4.03 (s, 3H), 6.93-6.95 (m, 5H), 7.05 (s, 1H), 7.21-7.24 (m, 6H), 7.81 (s, 1H), 8.41 (d, 1H), 8.59 (d, 1H).
Bsp. 33: ¹H-NMR (400 MHz, DMSO-d6), δ 3.91 (d, 2H), 4.00 (s, 3H), 6.42 (t, 1H), 6.82-6.84 (m, 2H), 7.13-7.17 (m, 3H), 7.40 (s, 1H), 7.48 (s, 1H), 7.74 (s, 1H), 8.30 (d, 1H), 8.47 (d, 1H).
Bsp. 34: ¹H-NMR (400 MHz, DMSO-d6), δ 1.30 (t, 3H), 3.88 (s, 4H), 4.52 (q, 2H), 6.90 (s, 1H), 6.94-6.97 (m, 4H), 6.99 (s, 1H), 7.21-7.24 (m, 6H), 7.80 (s, 1H), 8.40 (d, 1H), 8.57 (d, 1H).
Bsp. 35: ¹H-NMR (400 MNz, DMSO-d6), δ 1.33 (t, 3H), 3.92 (d, 2H), 4.48 (q, 2H), 6.40 (t, 1H), 6.82-6.85 (m, 2H), 7.12-7.16 (m, 3H), 7.37 (s, 1H), 7.46 (s, 1H), 7.73 (s, 1H), 8.28 (d, 1H), 8.45 (d, 1H).
Bsp. 36: ¹H-NMR (400 MHz, DMSO-d6), δ 1.29 (t, 3H), 3.89 (s, 4H), 4.51 (q, 2H), 6.96 (d, 4H), 6.99 (s, 1H), 7.08 (s, 1H), 7.27 (d, 4H), 7.83 (s, 1H), 8.39 (d, 1H), 8.57 (d, 1H).
Bsp. 37: ¹H-NMR (400 MHz, DMSO-d6), δ 1.33 (t, 3H), 3.92 (d, 2H), 4.48 (q, 2H), 6.46 (t, 1H), 6.84 (d, 2H), 7.18 (d, 2H), 7.37 (s, 1H), 7.47 (s, 1H), 7.73 (s, 1H), 8.28 (d, 1H), 8.45 (d, 1H).
Bsp. 38: ¹H-NMR (400 MHz, DMSO-d6), δ 3.88 (s, 4H), 4.03 (s, 3H), 6.95 (d, 4H), 7.03 (s, 1H), 7.13 (s, 1H), 7.27 (d, 4H), 7.83 (s, 1H), 8.40 (d, 1H), 8.59 (d, 1H).
Bsp. 39: ¹H-NMR (400 MHz, DMSO-d6), δ 3.91 (d, 2H), 4.01 (s, 3H), 6.47 (t, 1H), 6.84 (d, 2H), 7.20 (d, 2H), 7.39 (s, 1H), 7.49 (s, 1H), 7.74 (s, 1H), 8.30 (d, 1H), 8.48 (d, 1H).
Bsp. 40: ¹H-NMR (400 MHz, DMSO-d6), δ 1.33 (t, 3H), 3.69 (s, 3H), 3.83 (d, 2H), 4.48 (q, 2H), 6.46 (t, 1H), 6.84 (d, 2H), 7.18 (d, 2H), 7.37 (s, 1H), 7.47 (s, 1H), 7.73 (s, 1H), 8.28 (d, 1H), 8.45 (d, 1H).
Bsp. 41: ¹H-NMR (400 MHz, DMSO-d6), δ 3.72 (s, 3H), 3.85 (d, 2H), 4.02 (s, 3H), 6.35 (t, 1H), 6.72-6.77 (m, 4H), 7.43 (s, 1H), 7.51 (s, 1H), 7.78 (s, 1H), 8.32 (d, 1H), 8.50 (d, 1H).
Bsp. 42: ¹H-NMR (400 MHz, DMSO-d6), δ 3.73 (s, 6H), 3.76 (s, 4H), 4.04 (s, 3H), 6.79 (d, 4H), 6.86 (d, 4H), 6.92 (s, 1H), 7.05 (s, 1H), 7.80 (s, 1H), 8.42 (d, 1H), 8.60 (d, 1H).
Bsp. 43: ¹H-NMR (400 MHz, DMSO-d6), δ 1.30 (t, 3H), 3.73 (s, 6H), 3.77 (s, 4H), 4.52 (q, 2H), 6.79 (d, 2H), 6.86-6.88 (m, 3H), 7.00 (s, 1H), 7.79 (s, 1H), 8.40 (d, 1H), 8.58 (d, 1H).
Bsp. 44: ¹H-NMR (400 MHz, DMSO-d6), δ 6.13 (bs, 2H), 7.09 (dd, 1H), 7.17 (dd, 1H), 7.56 (s, 1H), 7.64 (s, 1H), 7.75 (dd, 1H), 7.79 (s, 1H), 8.78 (d, 1H), 8.87 (d, 1H).
Bsp. 45: ¹H-NMR (400 MHz, DMSO-d6), δ 5.95 (bs, 2H), 7.54 (s, 1H), 7.62 (s, 1H), 7.75 (s, 1H), 7.80 (dd, 1H), 8.46 (dd, 1H), 8.75 (d, 1H), 8.81 (d, 1H).
Bsp. 46: ¹H-NMR (400 MHz, DMSO-d6), δ 8.45 (d, 1H), 8.27 (d, 1H), 7.78 (s, 1H), 7.64 (s, 1H), 7.54 (s, 1H), 5.81 (bs, 2H, NH₂), 4.00 (s, 3H).
Bsp. 47: ¹H-NMR (400 MHz, DMSO-d6), δ 8.43 (d, 1H), 8.25 (d, 1H), 7.73 (s, 1H), 7.62 (s, 1H), 7.56 (s, 1H), 5.64 (bs, 2H, NH₂), 3.99 (s, 1H), 3.58 (s, 3H).
Bsp. 48: ¹H-NMR (400 MHz, DMSO-d6), δ 4.00 (s, 3H), 5.60 (bs, 2H), 6.15 (s, 2H), 6.83-6.84 (m, 2H), 8.24 (d, 1H), 8.42 (d, 1H).
Bsp. 49: ¹H-NMR (400 MHz, DMSO-d6), 8 4.00 (s, 3H), 6.83 (bs, 2H), 7.21 (dd, 1H), 7.48 (d, 1H), 7.84 (dd, 1H), 8.28 (d, 1H), 8.46 (d, 1H), 8.59 (dd, 1H).
Bsp. 50: ¹H-NMR (400 MHz, DMSO-d6), δ 4.01 (s, 3H), 5.92 (bs, 2H), 7.95 (d, 1H), 8.26 (d, 1H), 8.44 (d, 1H), 8.52 (d, 1H), 8.65 (d, 1H).
Bsp. 51: ¹H-NMR (400 MHz, DMSO-d6), δ 8.43 (d, 1H), 8.25 (d, 1H), 6.94 (s, 2H), 5.60 (bs, 2H, NH₂), 3.99 (s, 3H), 3.86 (s, 3H), 3.8 (s, 3H).
Bsp. 52: ¹H-NMR (400 MHz, DMSO-d6), δ 4.00 (s, 3H), 5.80 (bs, 2H), 8.23 (d, 1H), 8.25 (d, 1H), 8.43 (d, 1H), 8.64 (d, 1H).
Bsp. 53: ¹H-NMR (400 MHz, DMSO-d6), δ 4.00 (s, 3H), 6.75 (bs, 2H), 7.49 (d, 1H), 7.95 (dd, 1H), 8.28 (d, 1H), 8.47 (d, 1H), 8.62 (d, 1H).
Bsp: 54: ¹H-NMR (400 MHz, DMSO-d6), δ 3.99 (s, 3H), 6.65 (bs, 2H), 7.53 (d, 1H), 7.95 (dd, 1H), 8.28 (d, 1H), 8.47 (d, 1H), 8.62 (d, 1H).
Bsp. 55: ¹H-NMR (400 MHz, DMSO-d6), δ 4.01 (s, 3H), 6.78 (bs, 2H), 7.64 (s, 1H), 7.72 (s, 1H), 8.30 (d, 1H), 8.49 (d, 1H).
Bsp. 56: ¹H NMR (400 MHz , DMSO-d6), δ 8.74 (app. s, 2H), 7.54 (t, 1 H, J = 1.9 Hz), 7.34 (d, 2H, J = 1.9 Hz), 6.08 (bs, 2H, NH₂).
Bsp. 57: ¹H NMR (400 MHz , DMSO-d6), δ 4.00 (s, 3H), 5.74 (bs, 2H), 7.46 (dd, 1H), 7.81 (dd, 1H), 8.24 (d, 1H), 8.41-8.43 (m, 2H).
Bsp. 58: ¹H NMR (400 MHz , DMSO-d6), δ 4.00 (s, 3H), 5.80 (bs, 2H), 7.55 (d, 1H), 7.78 (dd, 1H), 8.26 (d, 1H), 8.34 (d, 1H), 8.44 (d, 1H).
Bsp. 59: ¹H NMR (400 MHz , DMSO-d6), δ 8.44 (d, 1H), 8.26 (d, 1H), 7.74 (s, 1H), 7.67 (s, 1H), 7.60 (s, 1H), 6.80 - 6.55 (tt, 1H, CHF₂), 5.83 (bs, 2H, NH₂), 4,00 (s, 3H).
Bsp. 65: ¹H NMR (400 MHz , DMSO-d6), δ 8.41 (d, 1H), 8.24 (d, 1H), 6.84 (s, 1H), 6.83 (s, 1H), 6.75 - 6.49 (tt, 1H, CHF₂), 6.15 (s, 2H), 5.53 (bs, 2H, NH₂), 3.99 (s, 3H).
Bsp. 66: ¹H NMR (400 MHz , DMSO-d6), δ 8.4 (d, 1H), 8.24 (d, 1H), 7.15 (s, 1H), 7.08 (s, 1H), 6.74 - 6.48 (tt, 1H, CHF₂), NH₂ breites Signal, 4.68 - 4.64 (dd, 2H), 3.99 (s, 3H), 3.34 - 3.29 (dd, 2H).
Bsp. 74: ¹H NMR (400 MHz , DMSO-d6), δ 8.45 (d, 1H), 8.26 (d, 2H), 6.95 (s, 1H), 6.87 (s, 1H), 6.17 (s, 2H), 5.80 (s, 2H), 3.98 (s, 3H).
Bsp. 75: ¹H NMR (400 MHz , DMSO-d6), δ 8.27 (d, 1H), 8.15 (d, 1H), 6.87 (s, 1H), 6.84 (s, 1H), 6.11 (s, 2H), 5.21 (bs, 2H, NH₂), 3.96 (s, 3H), 2.11 (s, 3H).
Bsp. 76: ¹H NMR (400 MHz , DMSO-d6), δ 8.28 (d, 1H), 8.16 (d, 1H), 6.85 (s, 1H), 6.82 (s, 1H), 6.12 (s, 2H), 5.14 (bs, 2H, NH₂), 3.97 (s, 3H), 2.56 - 2.49 (q, 2H), 1.08 (t, 3H).
Bsp. 77: ¹H NMR (400 MHz , DMSO-d6), δ 8.31 (d, 1H), 8.18 (d, 1H), 6.85 (s, 1H), 6.82 (s, 1H), 6.15 (s, 2H), 5.21 (bs, 2H, NH₂), 3.96 (s, 3H), 2.98 - 2.93 (m, 1H), 1.11 (d, 3H).
Bsp. 79: ¹H NMR (400 MHz , DMSO-d6), δ 8.31 (d, 1H), 8.17 (d, 1H), 6.98 (s, 1H), 6.95 (s, 1H), 6.14 (s, 2H), 5.34 8bs, 2H, NH₂), 3.97 (s, 3H), 1.77 - 1.73 (m, 1H), 0.81 - 0.73 (m, 4 H).
Bsp. 86: ¹H NMR (400 MHz, DMSO-d6), δ 8.49 (d, 1H), 8.29 (d, 1H), 7.89 (d, 1H), 7.72 (d, 1H), 6.18 (bs, 2H), 3.99 (s, 3H).
Bsp. 87: ¹H NMR (400 MHz , DMSO-d6), δ 8.44 (d, 1H), 8.26 (d, 1H), 7.54 (s, 2H), 5.91 (bs, 2H, NH₂), 4,00 (s, 3H).
Bsp. 89: ¹H NMR (400 MHz , DMSO-d6), δ 8.48 (d, 1H), 8.28 (d, 1H), 7.30 (s, 1H), 7.10 (s, 1H), 5.93 (bs, 2H, NH₂), 3.99 (s, 3H), 3.93 (s, 3H), 3.86 (s, 3H).
Bsp. 96: ¹H NMR (400 MHz , DMSO-d6), δ 8.47 (d, 1H), 8.28 (d, 1H), 7.83 (s, 1H), 7.70 (s, 1H), 7.59 (s, 1H), 6.15 (br t, 1H), 4.65 (br s, 1H), 4.60 (br. s, 1H), 4.00 (s, 3H), 3.18 (d, 2H), 1.25 (s, 3H).
Bsp. 103: ¹H-NMR (400 MHz, CDCl₃), δ 7.82 (d, 1H), 7.67 (m, 1H), 7.63 (m, 2 H), 7.60 (d, 1H), 6.09 (bs, 2H, NH₂), 4.25 (s, 3H).
Bsp. 105: ¹H NMR (400 MHz , DMSO-d6), δ 8.53 (d, 1H), 8.32 (d, 1H), 7.89 (s, 1H), 7.87 (s, 1H), 7.79 (s, 1H), 4.00 (s, 3H), 3.67 (d, 4H), 2.96 (t, 2H).
Bsp. 106: ¹H NMR (400 MHz , DMSO-d6), δ 8.53 (d, 1H), 8.32 (d, 1H), 7.89 (m, 1H), 7.87 (m, 1H), 7.78 (m, 1H), 4.00 (s, 3H), 3.67 (d, 4H), 2.96 (t, 2H).
Bsp. 109: ¹H NMR (400 MHz, DMSO-d6), δ 8.50 (d, 1H), 8.28 (d, 1H), 7.80 (m, 1H), 7.49 (t, 2H), 7.42 (m, 2H), 7.04 (d, 2H), 6.85 (d, 2H), 4.06 (s, 4H), 3.91 (s, 3H), 2.33 (s, 6H).
Bsp. 116: ¹H NMR (400 MHz , DMSO-d6), δ 8.47 (d, 1H), 8.28 (d, 1H), 7.55 (s, 2H), 7.91-6.62 (1H, m) 6.03 (bs, 2H), 3.98 (s, 3H).
Bsp. 121: ¹H NMR (400 MHz , DMSO-d6), δ 8.47 (d, 1H), 8.28 (d, 1H), 7.34 (s, 2H), 5.97 (bs, 2H, NH₂), 3.98 (s, 3H), 2.86 (s, 6H).
Bsp. 122: ¹H NMR (400 MHz , DMSO-d6), δ 8.46 (d, 1H), 8.27 (d, 1H), 7.33 - 7.21 (m, 3H), 5.77 (bs, 2H, NH₂), 3.99 (s, 3H), 2.77 (s, 6H).
Bsp. 123: ¹H NMR (400 MHz, DMSO-d6), δ 8.48 (d, 1H), 8.29 (d, 1H), 7.67 (s, 1H), 7.56 (d, 1H), 6.08 (bs, 2H, NH₂), 3.99 (s, 3H), 2.83 (s, 6H).
Bsp. 124: ¹H NMR (400 MHz , DMSO-d6), δ 8.47 (d, 1H), 8.28 (d, 1H), 7.24 (s, 1H), 7.22 (s, 1H), 5.96 (bs, 2H, NH₂), 3.99 (s, 3H), 3.93 (s, 3H).
Bsp. 125: ¹H NMR (400 MHz, DMSO-d6), δ 8.46 (d, 1H), 8.27 (d, 1H), 7.15 (d, 1H), 6.99 (s, 1H), 6.90 (d, 1H), 5.72 (bs, 2H, NH₂), 4.75 - 4.69 (quart, 2H), 3.99 (s, 3H), 3.83 (s, 3H).
Bsp. 126: ¹H NMR (400 MHz , DMSO-d6), δ 8.48 (d, 1H), 8.28 (d, 1H), 7.25 (s, 1H), 7.09 (s, 1H), 5.90 (bs, 2H, NH₂), 4.93 - 4.87 (m, 1H), 3.99 (s, 3H), 3.90 (s, 3 H), 1.24 (d, 6H).
Bsp. 127: ¹H NMR (400 MHz, DMSO-d6), δ 8.46 (d, 1H), 8.27 (d, 1H), 7.16 (d, 1H), 6.97 (d, 1H), 6.90 (d, 1H), 5.67 (bs, 2H, NH₂), 4.75 - 4.68 (quart, 2H), 4.12 - 4.09 (quart, 2H), 3.99 (s, 3H), 1.36 (t, 3H).
Bsp. 128: ¹H NMR (400 MHz , DMSO-d6), δ 8.44 (d, 1H), 8.26 (d, 1H), 7.14 (d, 1H), 7.09 (d, 1H), 5.59 (bs, 2H, NH₂), 3.99 (s, 3H), 3.87 (s, 3H), 3.77 (s, 3H).
Bsp. 129: ¹H NMR (400 MHz , DMSO-d6), δ 8.80 (d, 1H), 8.76 (d, 1H), 7.84 (s, 1H), 7.69 (s, 1H), 6.31 (bs, 2H, NH₂).
Bsp. 130: ¹H NMR (400 MHz , DMSO-d6), δ 9.10 (s, 2H), 7.86 (s, 1H), 7.69 (s, 1H), 7.59 (s, 1H), 6.34 (bs, 2H, NH₂).
Bsp. 131: ¹H NMR (400 MHz , DMSO-d6), δ 8.46 (d, 1H), 8.27 (d, 1H), 7.20 (d, 1H), 6.94 (d, 1H), 5.74 (bs, 2H), 4.21 (m, 2H), 3.98 (s, 3H), 2.18 (m, 2H).
Bsp. 132: ¹H NMR (400 MHz, DMSO-d6), δ 8.45 (d, 1H), 8.26 (d, 1H), 7.06 (d, 1H), 6.84 (d, 1H), 5.76 (bs, 2H), 4.37 (m, 2H), 4.31 (m, 2H), 3.98 (s, 3H).
Bsp. 133: ¹H NMR (400 MHz , DMSO-d6), δ 8.45 (d, 1H), 8.26 (d, 1H), 6.92 (d, 1H), 6.80 (d, 1H), 5.74 (bs, 2H), 4.37 (m, 2H), 4.32 (m, 2H), 3.98 (s, 3H).
Bsp. 134: ¹H NMR (400 MHz , DMSO-d6), δ 8.47 (d, 1H), 8.28 (d, 1H), 7.02 (s, 1H), 6.99 (s, 1H), 5.90 (bs, 2H, NH₂), 4.69 - 4.82 (quart, 2H), 3.99 (s, 3H), 3.90 (s, 3H).
Bsp. 135: ¹H NMR (400 MHz , DMSO-d6), δ 8.47 (d, 1H), 8.27 (d, 1H), 7.23 (s, 1H), 7.03 (s, 1H), 5.81 (bs, 2H, NH₂), 5.40 (s, 2H), 4.94 (s, 2H), 3.98 (s, 3H).
Bsp. 136: ¹H NMR (400 MHz , DMSO-d6), δ 8.46 (d, 1H), 8.27 (d, 1H), 7.06 (d, 1H), 6.90 (d, 1H), 5.82 (bs, 2H), 4.24 (m, 4H), 3.98 (s, 3H), 2.17 (m, 2H).
Bsp. 137: ¹H NMR (400 MHz, CD₃CN), δ 8.32 (d, 1H), 8.18 (d, 1 H), 7.75 (dd, 1H), 7.44 (m, 2H), 7.35 (m, 1H), 4.77 (bs, 2H), 4.04 (s, 3H).
Bsp. 138: ¹H NMR (400 MHz , DMSO-d6), δ 8.44 (d, 1H), 8.26 (d, 1H), 7.95 (m, 1H), 7.47 (m, 1H), 7.15 (m, 1H), 5.10 (bs, 2H), 3.99 (s, 3H).
Bsp. 139: ¹H NMR (600 MHz , CD₃CN), δ 8.33 (d, 1H), 8.17 (d, 1H), 7.18 (d, 1H), 6.86 (d, 1H), 6.08 (s, 2H), 4.83 (s, 2H), 4.03 (s, 3 H).
Bsp. 140: ¹H NMR (400 MHz, DMSO-d6), δ 8.47 (d, 1H), 8.28 (d, 1H), 7.12 (s, 1H), 7.02 (s, 1H), 5.88 (bs, 2H, NH₂), 4.68 - 4.61 (quart, 2H), 4.18 - 4.13 (quart, 2H), 3.99 (s, 3H), 1.38 (t, 3H).
Bsp. 141: ¹H NMR (400 MHz , DMSO-d6), δ 8.47 (d, 1H), 8.27 (d, 1H), 6.96 (s, 2H), 5.87 (bs, 2H, NH₂), 3.99 (s, 3H), 3.87 (s, 3H), 3.80 (s, 3H).
Bsp. 142: ¹H NMR (400 MHz , DMSO-d6), δ 8.46 (d, 1H), 8.27 (d, 1H), 7.77 (m, 1H), 7.41 (t, 1H), 6.00 (bs, 2H), 3.99 (s, 3H), 2.18 (m, 2H).
Bsp. 143: ¹H NMR (400 MHz , DMSO-d6), δ 8.44 (d, 1H), 8.26 (d, 1H), 7.18 (s, 1H), 6.89 (s, 1H), 6.12 (s, 2H), 5.66 (s, 2H), 3.98 (bs, 2H, NH₂).
Bsp. 144: ¹H NMR (400 MHz , DMSO-d6), δ 8.47 (d, 1H), 8.28 (d, 1H), 6.98 (s, 2H), 5.88 (bs, 2H, NH₂), 4.69 - 4.62 (quart, 2H), 4.18 - 4.13 (quart, 2H), 3.99 (s, 3H), 1.38 (t, 3H).
Bsp. 145: ¹H NMR (400 MHz , DMSO-d6), δ 8.45 (d, 1H), 8.27 (d, 1H), 7.07 (s, 1H), 7.04 (d, 1H), 6.76 (d, 1H), 5.58 (bs, 2H, NH₂), 3.98 (s, 3H), 2.79 - 2.75 (dt, 2H), 1.80 - 1.77 (dt, 2H), 1.30 (s, 6H).
Bsp. 147: ¹H NMR (400 MHz , DMSO-d6), δ 8.46 (d, 1H), 8.27 (d, 1H), 7.28(s, 1H), 7.00 (s, 1H), 5.84 (bs, 2H, NH₂), 3.99 (s, 3H), 3.85 (s, 3H), 3.75 (s, 3H).
Bsp. 150: ¹H NMR (400 MHz, DMSO-d6), δ 8.47 (d, 1H), 8.27 (d, 1H), 7.15 (s, 1H), 7.11 (s, 1H), 6.30 (s, 2H), 5.87 (bs, 2H, NH₂), 3.99 (s, 3H).
Bsp. 157: ¹H NMR (400 MHz , DMSO-d6), δ 8.45 (d, 1H), 8.28 (d, 1H), 7.30 (s, 1H), 7.12 (s, 1H), 6.75 (tt, CHF₂), 5.84 (bs, 2H, NH₂), 3.98 (s, 3H), 3.92 (s, 3H), 3.86 (s, 3H).
Bsp. 161: ¹H NMR (400 MHz , DMSO-d6), δ 8.32 (d, 1H), 8.19 (d, 1H), 6.95 (s, 2H), 5.37 (bs, 2H, NH₂), 3.96 (s, 3H), 3.87 (s, 3H), 3.77 (s, 3H), 2.59 (q, 2H), 1.08 (t, 3H).
Bsp. 165: ¹H NMR (400 MHz , DMSO-d6), δ 8.45 (d, 1H), 8.27 (d, 1H), 7.81 (s, 1H), 7.66 (s, 1H), 5.85 (bs, 2H, NH₂), 3.99 (s, 3H), under solvents (s, 3H).
Bsp. 166: ¹H NMR (400 MHz , DMSO-d6), δ 8.46 (d, 1H), 8.27 (d, 1H), 7.92 (d, 1H), 7.52 (d, 1H), 5.90 (bs, 2H, NH₂), 3.99 (s, 3H).
Bsp. 167: ¹H NMR (400 MHz , DMSO-d6), δ 8.37 (d, 1H), 8.23 (d, 1H), 7.44 (s, 1H), 7.31 (s, 2H), 5.50 (bs, 2H, NH₂), 5.08 (s, 1H), 4.85 (s, 1H), 3.97 (s, 3H), 1.96 (s, 3H).
Bsp. 168: ¹H NMR (400 MHz , DMSO-d6), δ 8.38 (d, 1H), 8.23 (d, 1H), 7.48 (s, 1H), 7.38 (s, 2H), 5.50 (bs, 2H, NH₂), 3.97 (s, 3H), 1.57 (s, 3H), 1.52 (s, 3H).
Bsp. 169: ¹H NMR (400 MHz , DMSO-d6), δ 8.45 (d, 2H), 8.27 (d, 2H), 6.85 (s, 2H), 6.18 (s, 2H), 5.74 (bs, 2H, NH₂), 3.99 (s, 3H).
Bsp. 170: ¹H NMR (400 MHz, DMSO-d6), δ 8.27 (d, 1H), 8.16 (d, 1H), 6.88 (s, 1H), 6.84 (s, 1H), 6.14 (s, 2H), 5.30 (bs, 2H, NH₂), 4.44 (q, 2H), 2.53 (q, 2H), 1.33 (t, 3H), 1.08 (t, 3H).
Bsp. 171: ¹H NMR (400 MHz , DMSO-d6), δ 8.43 (d, 1H), 8.24 (d, 1H), 6.84 (s, 2H), 6.18 (s, 2H), 5.78 (s, 2H), 4.47 (q, 2H), 1.32 (t, 3H).
Bsp. 172: ¹H NMR (400 MHz , DMSO-d6), δ 8.43 (d, 1H), 8.25 (d, 1H), 7.14 (s, 1H), 7.10 (s, 1H), 6.30 (s, 2H), 5.86 (bs, 2H, NH₂), 4.48 (q, 2H), 1.32 (t, 3H).
Bsp. 173: ¹H NMR (400 MHz, DMSO-d6), δ 8.47 (d, 1H), 8.28 (d, 2H), 7.11 (s, 1H), 7.09 (s, 1H), 5.89 (bs, 2H, NH₂), 4.42 - 4.36 (m, 4H), 3.98 (s, 3H).
Bsp. 174: ¹H NMR (400 MHz , DMSO-d6), δ 8.46 (d, 1H), 8.27 (d, 1H), 6.76 (s, 2H), 6.17 (s, 2H), 5.80 (bs, 2H, NH₂), 3.98 (s, 3H).
Bsp. 175: ¹H NMR (400 MHz, DMSO-d6), δ 8.43 (d, 1H), 8.24 (d, 1H), 6.75 (s, 2H), 6.17 (s, 2H), 5.80 (bs, 2H, NH₂), 4.47 (q, 2H), 1.32 (t, 3H).
Bsp. 176: ¹H NMR (400 MHz , DMSO-d6), δ 8.43 (d, 1H), 8.25 (d, 1H), 7.28 (s, 1H), 7.00 (s, 1H), 5.84 (bs, 2H, NH₂), 4.48 (q, 2H), 3.85 (s, 3H), 3.75 (s, 3H), 1.33 (t, 3H).
Bsp. 177: ¹H NMR (400 MHz , DMSO-d6), δ 8.28 (d, 1H), 8.16 (d, 1H), 6.94 (s, 1H), 6.90 (s, 1H), 6.13 (s, 2H), 5.30 (bs, 2H, NH₂), 4.44 (q, 2H), 2.53 (q, 2H), 1.33 (t, 3H), 1.08 (t, 3H).
Bsp. 178: ¹H NMR (400 MHz , DMSO-d6), δ 8.31 (d, 1H), 8.18 (d, 1H), 6.94 (s, 1H), 6.90 (s, 1H), 6.13 (s, 2H), 5.30 (bs, 2H, NH₂), 3.96(s, 3H), 2.52 (q, 2H), 1.06 (t, 3H).
Bsp. 179: ¹H NMR (400 MHz , DMSO-d6), δ 8.45 (d, 1H), 8.27 (d, 1H), 7.81 (s, 1H), 7.66 (s, 1H), 5.85 (bs, 2H, NH₂), 4.29 - 4.04 (m, 2H), 3.99 (s, 3H).
Bsp. 180: ¹H NMR (400 MHz , DMSO-d6), δ 8.48 (d, 1H), 8.28 (d, 1H), 7.51 (s, 1H), 7.46 (s, 1H), 6.00 (bs, 2H, NH₂), 4.22 (q, 2H), 3.99 (s, 3H).
Bsp. 181: ¹H NMR (400 MHz , DMSO-d6), δ 8.48 (d, 1H), 8.28 (d, 1H), 7.61 - 7.64 (d+d, 2H), 5.95 (bs, 2H, NH₂), 4.12 - 3.96 (m, 2H), 4.00 (s, 3H).
Bsp. 182: ¹H NMR (400 MHz , DMSO-d6), δ 8.50 (d, 1H), 8.30 (d, 1H), 7.80 (s, 2H), 6.16 (bs, 2H, NH₂), 4.36 - 4.14 (m, 2H), 4.00 (s, 3H).

### Biologische Beispiele

### Boophilus microplus (dip)

### Testtiere: adulte gesogene Weibchen von Boophilus microplus Stamm Parkhurst - SP-resistent)

### Lösungsmittel: Dimethylsulfoxid

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration. Diese Wirkstoffzubereitung wird in Röhrchen pipettiert. 8-10 Zecken werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt. Die Wirkungskontrolle erfolgt nach 7 Tagen auf Ablage fertiler Eier. Eier, deren Fertilität nicht äußerlich sichtbar ist, werden in Glasröhrchen bis zum Larvenschlupf im Klimaschrank aufbewahrt. Eine Wirkung von 100 % bedeutet, dass keine Zecke fertile Eier gelegt hat.

Bei diesem Test zeigen z.B. die folgenden Verbindungen der Herstellungsbeispiele eine Wirkung von 90 % bei einer Aufwandmenge von 100 ppm: Bsp. Nr. 8, 14, 26

Bei diesem Test zeigen z.B. die folgenden Verbindungen der Herstellungsbeispiele eine Wirkung von 95 % bei einer Aufwandmenge von 100 ppm: Bsp. Nr. 22, 133

Bei diesem Test zeigen z.B. die folgenden Verbindungen der Herstellungsbeispiele eine Wirkung von 100 % bei einer Aufwandmenge von 100 ppm: Bsp. Nr. 2, 5, 15, 17, 18, 20, 47, 48, 51, 75, 76, 132

### Boophilus microplus - test (BOOPMI Injection)

### Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration. Die Wirkstofflösung wird in das Abdomen (Boophilus microplus) injiziert, die Tiere werden in Schalen überführt und in einem klimatisierten Raum aufbewahrt. Die Wirkungskontrolle erfolgt auf Ablage fertiler Eier. Nach 7 Tagen wird die Wirkung in % bestimmt. Dabei bedeutet 100%, dass keine Zecke fertile Eier gelegt hat.

Bei diesem Test zeigen z.B. die folgenden Verbindungen der Herstellungsbeispiele eine Wirkung von 100 % bei einer Aufwandmenge von 20µg / Tier: Bsp. Nr. 2, 3, 5, 6, 8, 9, 10, 12, 14, 15, 17, 18, 20, 21, 22, 26, 28, 46, 47, 48, 51, 75, 76, 89, 131, 132, 133, 135, 136, 139, 141

### Ctenocephalides felis - test (CTECFE)

### Lösungsmittel: 1 Gewichtsteil Dimethylsulfoxid

Zwecks Herstellung einer zweckmäßigen Wirkstoffzubereitung vermischt man 10 mg Wirkstoff mit 0,5 ml Dimethylsulfoxid. Ein Teil des Konzentrats wird mit citriertem Rinderblut verdünnt und die gewünschte Konzentration hergestellt. Ca. 20 nüchterne adulte Flöhe (Ctenocephalides felis) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann. Nach 2 Tagen wird die Abtötung in % bestimmt. Dabei bedeutet 100 %, dass alle Flöhe abgetötet wurden; 0 % bedeutet, dass kein Floh abgetötet wurde.

Bei diesem Test zeigen z.B. die folgenden Verbindungen der Herstellungsbeispiele eine Wirkung von 80 % bei einer Aufwandmenge von 100 ppm: Bsp. Nr. 41, 46

Bei diesem Test zeigen z.B. die folgenden Verbindungen der Herstellungsbeispiele eine Wirkung von 90 % bei einer Aufwandmenge von 100 ppm: Bsp. Nr. 2, 6, 10, 12, 76

Bei diesem Test zeigen z.B. die folgenden Verbindungen der Herstellungsbeispiele eine Wirkung von 95 % bei einer Aufwandmenge von 100 ppm: Bsp. Nr. 15, 22, 26

Bei diesem Test zeigen z.B. die folgenden Verbindungen der Herstellungsbeispiele eine Wirkung von 100 % bei einer Aufwandmenge von 100 ppm: Bsp. Nr. 14, 17, 47, 48, 51, 75, 89, 131, 132, 133, 135, 136, 139, 141

### Lucillia cuprina - test (LUCICU)

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration. Gefäße, die Pferdefleisch enthalten, das mit der Wirkstoffzubereitung der gewünschten Konzentration behandelt wurde, werden mit ca 20 Lucilia cuprina Larven besetzt. Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100 % bei einer Aufwandmenge von 100 ppm: Bsp. Nr. 2, 3, 5, 6, 8, 9, 10, 12, 14, 15, 17, 18, 20, 21, 22, 26, 28, 41, 46, 47, 48, S 1, 75, 76, 89, 131, 132, 133, 135, 136, 139, 141

### Musca domestica - test

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration. Gefäße, die einen Schwamm enthalten, der mit der Wirkstoffzubereitung der gewünschten Konzentration behandelt wurde, werden mit Musca domestica Adulten besetzt. Nach 2 Tagen wird die Abtötung in % bestimmt. Dabei bedeutet 100 %, dass alle Fliegen abgetötet wurden; 0 % bedeutet, dass keine Fliegen abgetötet wurden.

Bei diesem Test zeigen z.B. die folgenden Verbindungen der Herstellungsbeispiele eine Wirkung von 80 % bei einer Aufwandmenge von 20 ppm: Bsp. Nr. 2, 133, 141

Bei diesem Test zeigen z.B. die folgenden Verbindungen der Herstellungsbeispiele eine Wirkung von 90 % bei einer Aufwandmenge von 20 ppm: Bsp. Nr. 14, 48

Bei diesem Test zeigen z.B. die folgenden Verbindungen der Herstellungsbeispiele eine Wirkung von 80 % bei einer Aufwandmenge von 100 ppm: Bsp. Nr. 5, 6, 47, 76, 89, 132

Bei diesem Test zeigen z.B. die folgenden Verbindungen der Herstellungsbeispiele eine Wirkung von 95 % bei einer Aufwandmenge von 100 ppm: Bsp. Nr. 15

Bei diesem Test zeigen z.B. die folgenden Verbindungen der Herstellungsbeispiele eine Wirkung von 100 % bei einer Aufwandmenge von 100 ppm: Bsp. Nr. 10, 12, 17, 21, 75, 131

### Amblyomma hebraeum - test (ANIBYHE)

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration. Zeckenymphen (Amblyomma hebraeum) werden in perforierte Plastikbecher gesetzt und in der gewünschten Konzentration eine Minute getaucht. Die Zecken werden auf Filterpapier in eine Petrischale überführt und in einem Klimaschrank gelagert. Nach 42 Tagen wird die Abtötung in % bestimmt. Dabei bedeutet 100 %, dass alle Zecken abgetötet wurden; 0 % bedeutet, dass keine Zecken abgetötet wurden.

Bei diesem Test zeigen z.B. die folgenden Verbindungen der Herstellungsbeispiele eine Wirkung von 100 % bei einer Aufwandmenge von 100 ppm: Bsp. Nr. 48, 76

### Myzus-Test (MYZUPE Spritzbehandlung)

| | | |
|---|---|---|
| Lösungsmittel: | 78 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (Brassica pekinensis), die von allen Stadien der Grünen Pfirsichblattlaus (Myzus persicae) befallen sind, werden mit einer Wirkstoffzubereitung der gewünschten Konzentration gespritzt. Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele eine Wirkung von 80 % bei einer Aufwandmenge von 500 g/ha: Bsp. Nr. 33, 46, 47, 76, 103

Bei diesem Test zeigen z.B. die folgenden Verbindungen der Herstellungsbeispiele eine Wirkung von 90 % bei einer Aufwandmenge von 500 g/ha: Bsp. Nr. 18, 41, 48, 74

Bei diesem Test zeigen z.B. die folgenden Verbindungen der Herstellungsbeispiele eine Wirkung von 100 % bei einer Aufwandmenge von 500 g/ha: Bsp. Nr. 8, 9, 14, 15, 17, 20, 21, 22, 59, 75, 77, 150, 167, 168, 169, 170, 171, 172, 174, 175

### Phaedon-Test (PHAECO Spritzbehandlung)

| | | |
|---|---|---|
| Lösungsmittel: | 78,0 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (Brassica pekinensis) werden mit einer Wirkstoffzubereitung der gewünschten Konzentration gespritzt und nach dem Abtrocknen mit Larven des Meerrettichblattkäfers (Phaedon cochleariae) besetzt. Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z.B. die folgende Verbindung der Herstellungsbeispiele eine Wirkung von 83 % bei einer Aufwandmenge von 500 g/ha: Bsp. Nr. 4, 13, 107

Bei diesem Test zeigen z.B. die folgenden Verbindungen der Herstellungsbeispiele eine Wirkung von 100 % bei einer Aufwandmenge von 500 g/ha: Bsp. Nr. 2, 3, 5, 6, 9, 12, 14, 15, 17, 18, 20, 21, 22, 23, 26, 27, 28, 33, 41, 43, 44, 46, 47, 48, 51, 52, 59, 65, 66, 74, 75, 76, 77, 79, 86, 87, 89, 116, 121, 122, 123, 124, 125, 127, 130, 131, 132, 133, 134, 135, 136, 139, 140, 141, 142, 144, 145, 150, 157, 161, 166, 167, 168, 169, 170, 171, 172, 173, 174, 175

### Spodoptera frugiperda-Test (SPODFR Spritzbehandlung)

| | | |
|---|---|---|
| Lösungsmittel: | 78,0 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Maisblattscheiben (Zea mays) werden mit einer Wirkstoffzubereitung der gewünschten Konzentration gespritzt und nach dem Abtrocknen mit Raupen des Heerwurms (Spodoptera frugiperda) besetzt. Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z.B. die folgenden Verbindungen der Herstellungsbeispiele eine Wirkung von 80 % bei einer Aufwandmenge von 500 g/ha: 135

Bei diesem Test zeigen z.B. die folgenden Verbindungen der Herstellungsbeispiele eine Wirkung von 83 % bei einer Aufwandmenge von 500 g/ha: Bsp. Nr. 6, 18, 26, 59, 76

Bei diesem Test zeigen z.B. die folgenden Verbindungen der Herstellungsbeispiele eine Wirkung von 100 % bei einer Aufwandmenge von 500 g/ha: Bsp. Nr. 9, 14, 1 S, 17, 20, 21, 22, 28, 43, 44, 46, 47, 48, 51, 65, 66, 74, 75, 77, 79, 86, 87, 89, 116, 121, 122, 123, 124, 130; 131, 132, 133, 134, 139, 140, 141, 144, 150, 157, 161, 168, 169, 170, 171, 172, 173, 174, 17S

### Tetranychus-Test; OP-resistent (TETRUR Spritzbehandlung)

| | | |
|---|---|---|
| Lösungsmittel: | 78,0 | GewichtsteileAceton |
| | 1,5 | Gewichtsteile Dimethylformamid |
| Emulgator : | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblattscheiben (Phaseolus vulgaris), die von allen Stadien der Gemeinen Spinnmilbe (Tetranychus urticae) befallen sind, werden mit einer Wirkstoffzubereitung der gewünschten Konzentration gespritzt. Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z.B. die folgenden Verbindungen der Herstellungsbeispiele eine Wirkung von 80 % bei einer Aufwandmenge von 500 g/ha: Bsp. Nr. 5, 46, 51, 123, 132, 139

Bei diesem Test zeigen z.B. die folgende Verbindunge der Herstellungsbeispiele eine Wirkung von 80 % bei einer Aufwandmenge von 100 g/ha: Bsp. Nr. 121

Bei diesem Test zeigen z.B. die folgende Verbindunge der Herstellungsbeispiele eine Wirkung von 90 % bei einer Aufwandmenge von 100 g/ha: Bsp. Nr. 133

Bei diesem Test zeigen z.B. die folgenden Verbindungen der Herstellungsbeispiele eine Wirkung von 90 % bei einer Aufwandmenge von 500 g/ha: Bsp. Nr. 4, 12, 33, 35, 65, 66, 74, 116, 124, 125, 127, 166, 173

Bei diesem Test zeigen z.B. die folgenden Verbindungen der Herstellungsbeispiele eine Wirkung von 100 % bei einer Aufwandmenge von 500 g/ha: Bsp. Nr. 2, 3, 9, 14, 15, 17, 18, 20, 21, 22, 23, 26, 47, 48, 59, 75, 77, 96, 131, 141, 144, 150, 157, 167, 168, 169, 170, 171, 172, 174, 175

## Patentansprüche

1. Pyrazinylpyrazole der allgemeinen Formel (I) 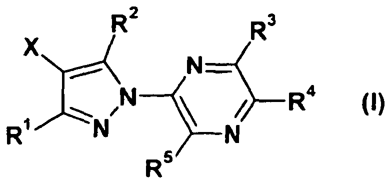 in welcher
X für Phenyl, 2-Pyridyl oder 3-Pyridyl steht, welche jeweils substituiert sind durch einen oder mehrere Substituenten, ausgewählt aus der Gruppe, bestehend aus Halogen, Alkyl, Haloalkyl, Alkoxy, Alkoxyalkyl, Alkoxyalkoxy, Cycloalkyl, Alkenyloxy, Alkinyloxy, Benzyloxy, Cycloalkylalkoxy, Haloalkoxy, Haloalkoxyalkyl, Alkylsulfanyl, Haloalkylsulfanyl, Alkylsulfinyl, Haloalkylsulfinyl, Alkylsulfonyl, Haloalkylsulfonyl, Cyano, Nitro, Alkylcarbonyl, Alkoxycarbonyl, Alkoxycarbonylalkyl, Carboxyl, Carboxamid, Dialkylcarboxamid, Trialkylsilyl, Amino, Alkylamino, Dialkylamino, Alkylsulfonylamino, Dialkylsulfonylamino, Formyl, -CH=NO-H, -CH=NO-Alkyl, -CH=NO-Halolkyl, - C(CH₃)=NO-H, -C(CH₃)=NO-Alkyl, -C(CH₃)=NO-Haloalkyl; oder für gegebenenfalls mit einem oder mehreren Halogenatomen, Cyano, Nitro, Alkyl, Alkoxy oder Haloalkyl substituiertes Phenyl, 2-Pyridyl und 3-Pyridyl steht, wobei vicinale Alkyl-, Haloalkyl-, Alkoxy- und/oder Haloalkoxygruppen an dem Phenylsubstituenten, 2-Pyridylsubstituenten oder 3-Pyridylsubstituenten zusammen mit den Kohlenstoffatomen, an die sie gebunden sind, ein fünf- bis siebengliedriges cyclisches System bilden können, das 0 bis 2 Sauerstoff- oder Stickstoffatome enthält, wobei zwei Sauerstoffatome nicht direkt miteinander verbunden sind, und dessen Alkylanteil gegebenenfalls mit einem oder mehreren Halogenatomen und/oder weiteren Alkylresten substituiert sein kann;
R¹ für Wasserstoff, Alkyl, welches gegebenenfalls einfach oder unabhängig voneinander mehrfach mit Alkoxy, Haloalkoxy, Alkylsulfanyl, Haloalkylsulfanyl, Alkylsulfinyl, Haloalkylsulfinyl, Alkylsulfonyl, Haloalkylsulfonyl, Alkylcarbonyl, Alkoxycarbonyl, Hydroxy und/oder Cycloalkyl substituiert ist;
Alkenyl, welches gegebenenfalls einfach oder unabhängig voneinander mehrfach mit Halogen, Alkoxy, Haloalkoxy, Alkylsulfanyl, Haloalkylsulfanyl, Alkylsulfinyl, Haloalkylsulfinyl, Alkylsulfonyl, Haloalkylsulfonyl, Alkylcarbonyl, Alkoxycarbonyl, und/oder Cycloalkyl substituiert ist;
Cycloalkyl, welches gegebenenfalls einfach oder unabhängig voneinander mehrfach mit Alkyl, Haloalkyl und/oder Halogen substituiert ist;
Haloalkyl, welches gegebenenfalls einfach oder unabhängig voneinander mehrfach mit Alkoxy, Alkylsulfanyl, Haloalkylsulfanyl, Alkylsulfinyl, Haloalkylsulfinyl, Alkylsulfonyl, Haloalkylsulfonyl und/oder Phenyl, gegebenenfalls einfach oder unabhängig voneinander mehrfach mit Halogen, Alkyl, Haloalkyl und/oder Alkoxy substituiert, substituiert ist;
Phenyl, welches gegebenenfalls einfach oder unabhängig voneinander mehrfach mit Halogen, Alkyl, Haloalkyl und/oder Alkoxy substituiert ist;
Benzyl, welches gegebenenfalls einfach oder unabhängig voneinander mehrfach mit Halogen, Alkyl, Haloalkyl und/oder Alkoxy substituiert ist;
Cyano, Formyl, Alkylcarbonyl, -CH=NO-H, -CH=NO-Alkyl, -CH=NO-Haloalkyl, -C(CH₃)=NO-H, -C(CH₃)=NO-Alkyl oder -C(CH₃)=NO-Haloalkyl steht;
R² für gegebenenfalls substituiertes Amino steht, wobei Amino einfach oder unabhängig voneinander zweifach substituiert sein kann mit Alkyl, Haloalkyl, Alkoxyalkyl, Alkylsulfanylalkyl, Alkylsulfinylalkyl, Alkylsulfonylalkyl, Alkylcarbonyl, Cycloalkyl, Cycloalkylalkyl, Alkenyl, wobei die vorstehenden Reste gegebenenfalls substituiert sind durch Halogen, Cyano, Alkoxy, Alkoxycarbonyl oder Phenyl, wobei der Phenylring gegebenenfalls einfach oder mehrfach substituiert ist mit einem oder mehreren Substituenten unabhängig voneinander ausgewählt aus Halogen, Alkyl, Haloalkyl und Alkoxy; Alkinyl, Alkoxycarbonyl, Alkenyloxycarbonyl, Alkinyloxycarbonyl, Alkoxycarbonylalkyl, Alkoxycarbonylcarbonyl, Heterocyclyl, Heteroaryl, Heterocyclylalkyl oder Heteroarylalkyl, wobei der heterocyclische oder heteroaromatische Ring gegebenenfalls einfach oder mehrfach substituiert sein kann mit einem oder mehreren Substituenten unabhängig voneinander ausgewählt aus Halogen, Alkyl, Haloalkyl und Alkoxy; Benzyl oder Phenylcarbonyl, wobei der Phenylring im Benzyl und Phenylcarbonyl gegebenenfalls einfach oder mehrfach substituiert ist mit einem oder mehreren Substituenten unabhängig voneinander ausgewählt aus Halogen, Alkyl, Haloalkyl und Alkoxy; und
R³, R⁴ unabhängig voneinander für Wasserstoff, Halogen, Alkyl, Cycloalkyl, Haloalkyl, Cyano, Hydroxy, Formyl, Alkylcarbonyl, -CH=NO-H, -CH=NO-Alkyl, -CH=NO-Haloalkyl, -C(CH₃)=NO-H, -C(CH₃)=NO-Alkyl, - C(CH₃)=NO-Haloalkyl, Nitro, Hydroxy, SH, Alkoxy, Alkylsulfanyl, Haloalkylsulfanyl, Alkylsulfinyl, Haloalkylsulfinyl, Alkylsulfonyl oder Haloalkylsulfonyl stehen;
R⁵ für Halogen, Alkyl, Haloalkyl, Hydroxy, Alkoxy, Alkoxyalkyl, Alkoxyalkoxy, Cycloalkyl, Alkenyloxy, Alkinyloxy, Benzyloxy, Cycloalkylalkoxy, Haloalkoxy, Haloalkoxyalkyl, -SH, Alkylsulfanyl, Haloalkylsulfanyl, Alkylsulfinyl, Haloalkylsulfinyl, Alkylsulfonyl, Haloalkylsulfonyl, Cyano, Nitro, Alkylcarbonyl, Alkoxycarbonyl, Alkoxycarbonylalkyl, Carboxyl, Carboxamid, Dialkylcarboxamid, Trialkylsilyl, Nitro, Amino, Alkylamino, Dialkylamino, Alkylsulfonylamino, Dialkylsulfonylamino, Formyl, -CH=NO-H, -CH=NO-Alkyl, -CH=NO-Haloalkyl, -C(CH₃)=NO-H, -C(CH₃)=NO-Alkyl, -C(CH₃)=NO-Haloalkyl oder Heteroaryl, wobei der heteroaromatische Ring gegebenenfalls einfach oder mehrfach substituiert sein kann mit einem oder mehreren Substituenten unabhängig voneinander ausgewählt aus Halogen, Alkyl, Haloalkyl und Alkoxy;
steht;
sowie die N-Oxide und Salze der Verbindungen der allgemeinen Formel (I).

2. Pyrazinylpyrazole der allgemeinen Formel (I) nach Anspruch 1, **dadurch gekennzeichnet, dass**
der Rest X für Phenyl, 2-Pyridyl oder 3-Pyridyl steht, welche jeweils substituiert sind durch einen oder mehrere Substituenten, ausgewählt aus der Gruppe, bestehend aus Halogen, Alkyl, Haloalkyl, Alkoxy, Haloalkoxy, Alkylsulfanyl, Haloalkylsulfanyl, Alkylsulfinyl, Haloalkylsulfinyl, Alkylsulfonyl, Haloalkylsulfonyl, Cyano, Nitro und Dialkylamino; oder für gegebenenfalls mit einem oder mehreren Halogenatomen, Cyano, Nitro, Alkyl, Alkoxy oder Haloalkyl substituiertes Phenyl, 2-Pyridyl und 3-Pyridyl steht, wobei vicinale Alkyl-, Haloalkyl- und/oder Alkoxygruppen an dem Phenylsubstituenten, 2-Pyridylsubstituenten oder 3-Pyridylsubstituenten zusammen mit den Kohlenstoffatomen, an die sie gebunden sind, ein fünf- bis siebengliedriges cyclisches System bilden können, das 0 bis 2 Sauerstoff- oder Stickstoffatome enthält, wobei zwei Sauerstoffatome nicht direkt miteinander verbunden sind, und dessen Alkylanteil gegebenenfalls mit einem oder mehreren Halogenatomen und/oder weiteren Alkylresten substituiert sein kann;
der Rest R¹ für Wasserstoff, Alkyl, welches gegebenenfalls einfach oder unabhängig voneinander mehrfach mit Alkoxy, Haloalkoxy, Alkylsulfanyl, Haloalkylsulfanyl, Alkylsulfinyl, Haloalkylsulfinyl, Alkylsulfonyl, Haloalkylsulfonyl, Alkylcarbonyl, Alkoxycarbonyl, Hydroxy und/oder Cycloalkyl substituiert ist;
Alkenyl, welches gegebenenfalls einfach oder unabhängig voneinander mehrfach mit Halogen, Alkoxy, Haloalkoxy, Alkylsulfanyl, Haloalkylsulfanyl, Alkylsulfinyl, Haloalkylsulfinyl, Alkylsulfonyl, Haloalkylsulfonyl, Alkylcarbonyl, Alkoxycarbonyl, und/oder Cycloalkyl substituiert ist;
Cycloalkyl, welches gegebenenfalls einfach oder unabhängig voneinander mehrfach mit Alkyl, Haloalkyl und/oder Halogen substituiert ist;
Haloalkyl, welches gegebenenfalls einfach oder unabhängig voneinander mehrfach mit Alkoxy, Alkylsulfanyl, Haloalkylsulfanyl, Alkylsulfinyl, Haloalkylsulfinyl, Alkylsulfonyl, Haloalkylsulfonyl und/oder Phenyl substituiert ist;
CH=NOH, CH=NOCH₃ oder CN steht,
der Rest R² für Amino und substituiertes Amino, wobei das substituierte Amino einfach oder unabhängig voneinander zweifach substituiert sein kann mit Alkyl, Haloalkyl, Cycloalkylalkyl, gegebenenfalls durch Halogen oder Phenyl substitituiertes Alkenyl, Alkinyl, Heterocyclylalkyl und/oder Heteroarylalkyl, wobei der heteroaromatische Ring gegebenenfalls einfach oder mehrfach substituiert sein kann mit einem oder mehreren Substituenten unabhängig voneinander ausgewählt aus Halogen, Alkyl, Haloalkyl und Alkoxy; Benzyl, wobei der Phenylring im Benzyl gegebenenfalls einfach oder mehrfach substituiert sein kann mit einem oder mehreren Substituenten unabhängig voneinander ausgewählt aus Halogen, Alkyl, Haloalkyl und Alkoxy, steht,
die Reste R³ und R⁴ unabhängig voneinander für Wasserstoff, Halogen, Alkyl, Cycloalkyl, Haloalkyl, Cyano und/oder Hydroxy stehen und
der Rest R⁵ für Halogen, Alkyl, Haloalkyl, Hydroxy, Alkoxy, Haloalkoxy, Alkylsulfanyl, Haloalkylsulfanyl, Alkylsulfinyl, Haloalkylsulfinyl, Alkylsulfonyl, Haloalkylsulfonyl, Cyano, Amino, Alkylamino, Dialkylamino, Alkylsulfonylamino, Dialkylsulfonylamino oder Heteroaryl steht, wobei der heteroaromatische Ring gegebenenfalls einfach oder mehrfach substituiert sein kann mit einem oder mehreren Substituenten unabhängig voneinander ausgewählt aus Halogen, Alkyl, Haloalkyl und Alkoxy.

3. Pyrazinylpyrazole der allgemeinen Formel (I) nach Anspruch 1 oder 2, **dadurch gekennzeichnet, dass**
der Rest X für Phenyl, 2-Pyridyl oder 3-Pyridyl steht, welche jeweils substituiert sind durch einen oder mehrere Substituenten, ausgewählt aus der Gruppe bestehend aus Fluor, Chlor, Brom, lod, CF₃, Methoxy, Ethoxy, Propanyloxy, Trifluorethoxy, Methylsulfanyl, 2,2,2-Trifluorethylsulfanyl, Methylsulfinyl, 2,2,2-Trifluorethylsulfinyl, Methylsulfonyl, 2,2,2-Trifluorethylsulfonyl, Cyano und Dimethylamino; oder für gegebenenfalls mit einem oder mehreren Halogenatomen, Cyano, Nitro, Methyl, Methoxy oder CF₃ substituiertes Phenyl steht, wobei vicinale Alkyl- oder Alkoxygruppen an dem Phenylsubstituenten zusammen mit den Kohlenstoffatomen, an die sie gebunden sind, ein fünf - bis siebengliedriges cyclisches System bilden können, das 1 oder 2 Sauerstoffatome enthält, wobei zwei Sauerstoffatome nicht direkt miteinander verbunden sind, und dessen Alkylanteil mit einem oder mehreren weiteren Alkylresten substituiert sein kann;
der Rest R¹ für CH₃, CH₂CH₃, CH(CH₃)₂, CH₂CH₃CH₃, C(CH₃)₃, C(OCH₃)HCH₂CH₃, CH(OCH₃)₂, CH=CH₂, Prop-1-en-2-yl, Cyclopropyl, CF₃, CHFCH₃, CHF₂, CF₂Cl, CF₂Br, CF₂CF₃, CF₂CH₃, CF₂CF₂CF₃, CF₂CF₂H, 2-Fluorpropan-2-yl, 1,1,2,2 Tetrafluor-2-methoxyethyl, CH=NOH, CH=NOCH₃ oder CN steht,
der Rest R² für Amino, Methylamino, Dimethylamino, Benzylamino, Dibenzylamino, (4-Chlorbenzyl)amino, Bis(4-chlorbenzyl)amino, (4-Methoxybenzyl)amino, Bis(4-methoxybenzyl)amino, (2-Methylprop-2-en-1-yl)amino, Prop-2-en-1-ylamino, Diprop-2-en-1-ylamino, Bis(2-methylprop-2-en-1-yl)amino, Prop-2-in-1-ylamino, Bis(prop-2-in-1-yl)amino, (Pyrazin-2-ylmethyl)amino, (6-Methyl-pyridin-2-ylmethyl)amino, Bis(6-methyl-pyridin-2-ylmethyl)amino oder (Pyridin-2-ylmethyl)amino; steht,
die Reste R³ und R⁴ unabhängig voneinander für Wasserstoff, Chlor und/oder Methyl stehen und
der Rest R⁵ für Chlor, Brom, Methyl, CF₃, Methoxy, Ethoxy, Propoxy, Propan-2-yloxy, Dimethylamino, Cyano, Methylsulfanyl, Methylsulfinyl, Methylsulfonyl, 1H-Pyrazol-1-yl, 1H-Imidazol-1-yl oder 4-Fluor-1H-pyrazol-1-yl steht.

4. Verfahren zur Herstellung von Pyrazinylpyrazolen der allgemeinen Formel (I) gemäß einem der Ansprüche 1 bis 3, **gekennzeichnet durch** die folgenden Verfahrensschritte:
(a) Kondensation von Ketonitrilen, deren Tautomere oder Hydraten der Formeln (IIA), (IIB) und/oder (IIC) mit Pyrazinyl-Hydrazinen der allgemeinen Formel (III) gemäß nachfolgendem Reaktionsschema: 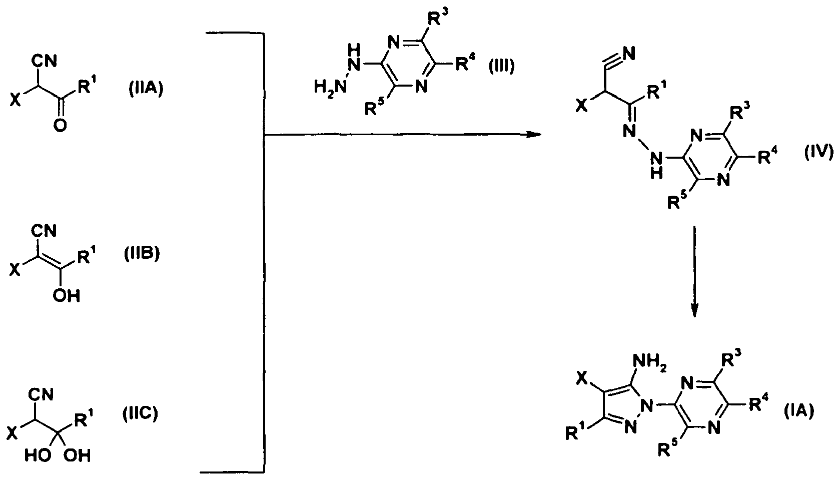 wobei X, R¹, R³, R⁴ und R⁵ die Bedeutungen gemäß einem der Ansprüche 1 bis 3 haben;
(b) Umsetzung von 1-H-Aminopyrazole der allgemeinen Formel (V) mit Pyrazinyl-Halogeniden und/oder -Alkylsulfonen der allgemeinen Formel (VI) in Gegenwart einer Base in organischen Lösungsmitteln gemäß nachfolgendem Reaktionsschema: 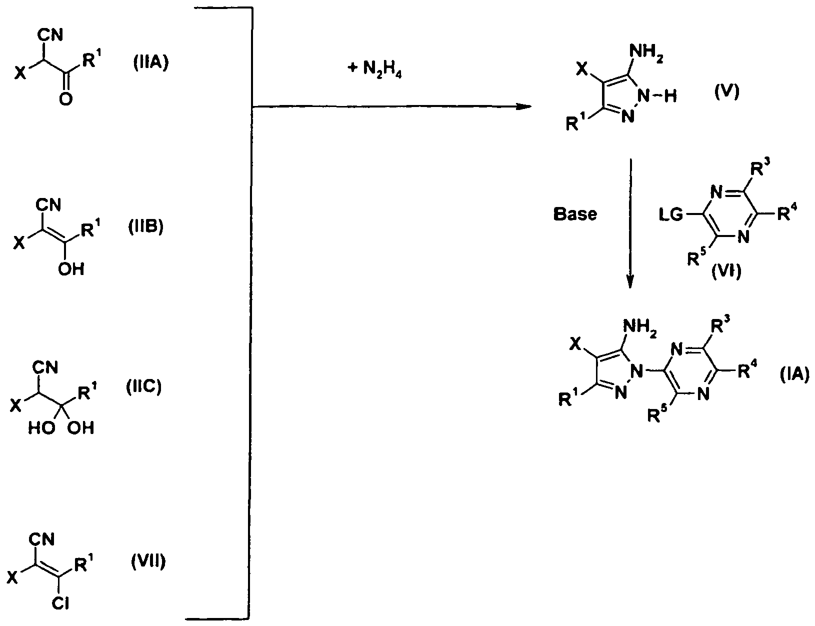 wobei X, R¹, R³, R⁴ und R⁵ die Bedeutungen gemäß einem der Ansprüche 1 bis 3 haben und LG für Halogen oder ein Alkylsulfonyl steht;
(c) Umsetzungen von Ketonitrilen, deren Tautomeren oder Hydraten der allgemeinen Formeln (IIA), (IIB) bzw. (IIC) mit Chlorierungsmitteln gegebenenfalls in einem inerten organischen Lösungsmittel verdünnt bzw. mit Hilfsbasen wie Stickstoffbasen unterstützt zu Chlor-Acrylnitrilen (VII) 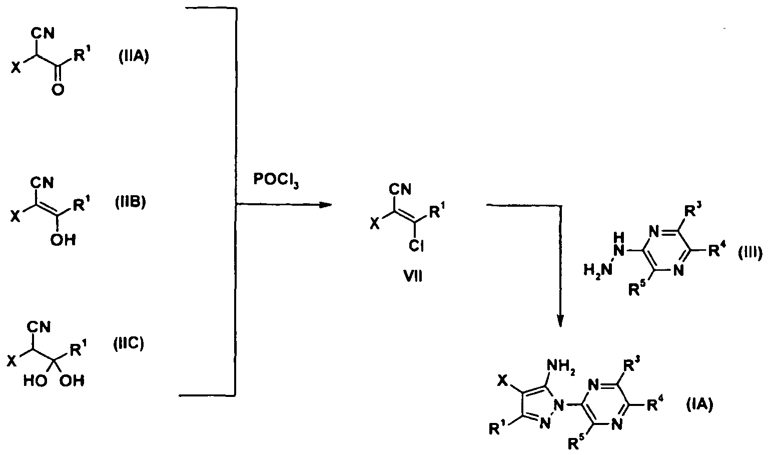 wobei X, R¹, R³, R⁴ und R⁵ die Bedeutungen gemäß einem der Ansprüche 1 bis 3 haben;
(d) Umsetzen von Verbindungen der allgemeinen Formel (IA) nach dem folgenden Reaktionsschema mit einem oder zwei Alkylierungsmitteln oder Acylierungsmitteln R⁶-LG und/oder R⁷-LG zum Erhalt von Verbindungen der allgemeinen Formel (IB) und (IC), in denen mindestens ein Rest von R⁶ und R⁷ nicht für Wasserstoff steht: 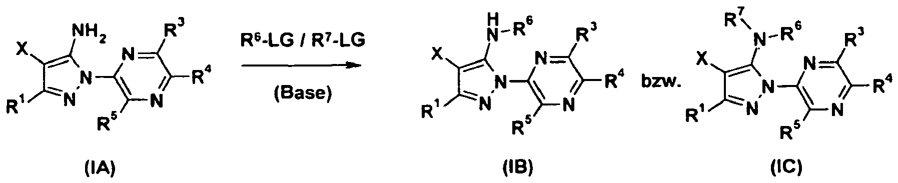 wobei
X, R¹, R³, R⁴ und R⁵ die Bedeutungen gemäß einem der Ansprüche 1 bis 3 haben;
LG für Halogen oder Alkylsulfonyl steht; und
R⁶ und R⁷, jeweils unabhängig voneinander, für Wasserstoff, Alkyl, Haloalkyl, Alkoxyalkyl, Alkylsulfanylalkyl, Alkylsulfinylalkyl, Alkylsulfonylalkyl, Alkylcarbonyl, Cycloalkyl, Cycloalkylalkyl, Alkenyl, wobei die vorstehenden Reste gegebenenfalls substituiert sind **durch** Halogen, Cyano, Alkoxy, Alkoxycarbonyl und Phenyl, wobei der Phenylring gegebenenfalls einfach oder mehrfach substituiert ist mit einem oder mehreren Substituenten unabhängig voneinander ausgewählt aus Halogen, Alkyl, Haloalkyl und Alkoxy; Alkinyl, Alkoxycarbonyl, Alkenyloxycarbonyl, Alkinyloxycarbonyl, Alkoxycarbonylalkyl, Alkoxycarbonylcarbonyl, Heterocyclyl, Heteroaryl, Heterocyclylalkyl oder Heteroarylalkyl, wobei der heterocyclische oder heteroaromatische Ring gegebenenfalls einfach oder mehrfach substituiert sein kann mit einem oder mehreren Substituenten, unabhängig voneinander ausgewählt aus Halogen, Alkyl, Haloalkyl und Alkoxy; Benzyl oder Phenylcarbonyl, wobei der Phenylring im Benzyl und Phenylcarbonyl gegebenenfalls einfach oder mehrfach substituiert ist mit einem oder mehreren Substituenten unabhängig voneinander ausgewählt aus Halogen, Alkyl, Haloalkyl und Alkoxy;
mit der Massgabe, dass mindestens ein Rest von R⁶ und R⁷ ungleich Wasserstoff ist; oder
(e) Umsetzen von Bromiden oder Iodiden der allgemeinen Formel (VII) mit Boronsäuren oder Boronsäureestern der Formel (IX) in Gegenwart von geeigneten Palladium-Katalysatoren und Basen in einem Temperaturbereich von -20 °C bis 120°C gemäß nachfolgendem Reaktionsschema: 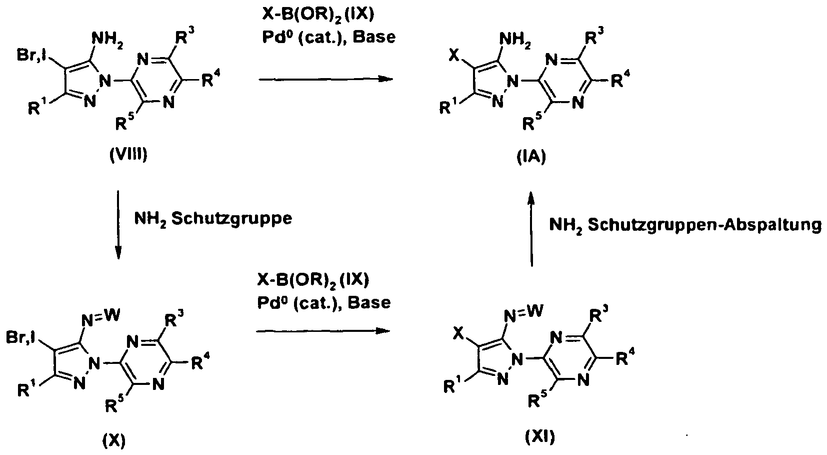 wobei X, R¹, R³, R⁴ und R⁵ die Bedeutungen gemäß einem der Ansprüche 1 bis 3 haben und W einer Schutzgruppe entspricht.

5. Mittel, enthaltend mindestens eine Verbindung der allgemeinen Formel (I) gemäß einem der Ansprüche 1 bis 3.

6. Verwendung von Verbindungen der Formel (I) gemäß einem der Ansprüche 1 bis 3 oder des Mittels gemäß Anspruch 5 zur Bekämpfung tierischer Schädlinge in der Landwirtschaft, im Gartenbau, in Forsten, in Gärten und Freizeiteinrichtungen, im Vorrats und Materialschutz.

7. Verfahren zur Bekämpfung tierischer Schädlinge, **dadurch gekennzeichnet, dass** man Verbindungen der Formel (I) gemäß einem der Ansprüche 1 bis 3 oder Mittel gemäß Anspruch 5 auf Saatgut einwirken lässt.

8. Verfahren zur Herstellung von Mitteln, **dadurch gekennzeichnet, dass** man Verbindungen der Formel (I) gemäß einem der Ansprüche 1 bis 5 mit Streckmitteln und/oder oberflächenaktiven Stoffen vermischt.

9. Verbindungen der Formel (I) gemäß einem der Ansprüche 1 bis 3 oder des Mittels gemäß Anspruch 5 zur Verwendung in in der Tierzucht oder dem Hygienesektor.

10. Verbindungen der Formel (I) gemäß einem der Ansprüche 1 bis 3 oder des Mittels gemäß Anspruch 5 zur Verwendung als Medikament.

## Claims

1. Pyrazinylpyrazoles of the general formula (I) 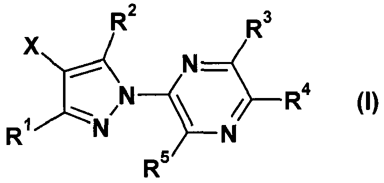 in which
X is phenyl, 2-pyridyl or 3-pyridyl, each of which is substituted by one or more substituents selected from the group consisting of halogen, alkyl, haloalkyl, alkoxy, alkoxyalkyl, alkoxyalkoxy, cycloalkyl, alkenyloxy, alkynyloxy, benzyloxy, cycloalkylalkoxy, haloalkoxy, haloalkoxyalkyl, alkylsulphanyl, haloalkylsulphanyl, alkylsulphinyl, haloalkylsulphinyl, alkylsulphonyl, haloalkylsulphonyl, cyano, nitro, alkylcarbonyl, alkoxycarbonyl, alkoxycarbonylalkyl, carboxyl, carboxamide, dialkylcarboxamide, trialkylsilyl, amino, alkylamino, dialkylamino, alkylsulphonylamino, dialkylsulphonylamino, formyl, -CH=NO-H, -CH=NO-alkyl, -CH=NO-halolkyl, -C(CH₃)=NO-H, -C(CH₃)=NO-alkyl, -C(CH₃)=NO-haloalkyl; or phenyl, 2-pyridyl and 3-pyridyl optionally substituted by one or more halogen atoms, cyano, nitro, alkyl, alkoxy or haloalkyl, where vicinal alkyl, haloalkyl, alkoxy and/or haloalkoxy groups on the phenyl substituent, 2-pyridyl substituent or 3-pyridyl substituent, together with the carbon atoms to which they are bonded, may form a five- to seven-membered cyclic system which contains 0 to 2 oxygen or nitrogen atoms, where no two oxygen atoms are directly bonded to one another, and the alkyl moiety of which may optionally be substituted by one or more halogen atoms and/or further alkyl radicals;
R¹ is hydrogen, alkyl which is optionally mono- or independently polysubstituted by alkoxy, haloalkoxy, alkylsulphanyl, haloalkylsulphanyl, alkylsulphinyl, haloalkylsulphinyl, alkylsulphonyl, haloalkylsulphonyl, alkylcarbonyl, alkoxycarbonyl, hydroxyl and/or cycloalkyl; alkenyl which is optionally mono- or independently polysubstituted by halogen, alkoxy, haloalkoxy, alkylsulphanyl, haloalkylsulphanyl, alkylsulphinyl, haloalkylsulphinyl, alkylsulphonyl, haloalkylsulphonyl, alkylcarbonyl, alkoxycarbonyl and/or cycloalkyl; cycloalkyl which is optionally mono- or independently polysubstituted by alkyl, haloalkyl and/or halogen; haloalkyl which is optionally mono- or independently polysubstituted by alkoxy, alkylsulphanyl, haloalkylsulphanyl, alkylsulphinyl, haloalkylsulphinyl, alkylsulphonyl, haloalkylsulphonyl and/or phenyl, optionally mono- or independently polysubstituted by halogen, alkyl, haloalkyl and/or alkoxy; phenyl which is optionally mono- or independently polysubstituted by halogen, alkyl, haloalkyl and/or alkoxy; benzyl which is optionally mono- or independently polysubstituted by halogen, alkyl, haloalkyl and/or alkoxy; cyano, formyl, alkylcarbonyl, -CH=NO-H, -CH=NO-alkyl, -CH=NO-haloalkyl, -C(CH₃)=NO-H, -C(CH₃)=NO-alkyl or -C(CH₃)=NO-haloalkyl;
R² is optionally substituted amino, where amino may be mono- or independently disubstituted by alkyl, haloalkyl, alkoxyalkyl, alkylsulphanylalkyl, alkylsulphinylalkyl, alkylsulphonylalkyl, alkylcarbonyl, cycloalkyl, cycloalkylalkyl, alkenyl, where the above radicals are optionally substituted by halogen, cyano, alkoxy, alkoxycarbonyl or phenyl, where the phenyl ring is optionally mono- or polysubstituted by one or more substituents selected independently from halogen, alkyl, haloalkyl and alkoxy; alkynyl, alkoxycarbonyl, alkenyloxycarbonyl, alkynyloxycarbonyl, alkoxycarbonylalkyl, alkoxycarbonylcarbonyl, heterocyclyl, heteroaryl, heterocyclylalkyl or heteroarylalkyl, where the heterocyclic or heteroaromatic ring may optionally be mono- or polysubstituted by one or more substituents selected independently from halogen, alkyl, haloalkyl and alkoxy; benzyl or phenylcarbonyl, where the phenyl ring in the benzyl and phenylcarbonyl is optionally mono- or polysubstituted by one or more substituents selected independently from halogen, alkyl, haloalkyl and alkoxy; and
R³, R⁴ are each independently hydrogen, halogen, alkyl, cycloalkyl, haloalkyl, cyano, hydroxyl, formyl, alkylcarbonyl, -CH=NO-H, -CH=NO-alkyl, -CH=NO-haloalkyl, -C(CH₃)=NO-H, - C(CH₃)=NO-alkyl, -C(CH₃)=NO-haloalkyl, nitro, hydroxyl, SH, alkoxy, alkylsulphanyl, haloalkylsulphanyl, alkylsulphinyl, haloalkylsulphinyl, alkylsulphonyl or haloalkylsulphonyl;
R⁵ is halogen, alkyl, haloalkyl, hydroxyl, alkoxy, alkoxyalkyl, alkoxyalkoxy, cycloalkyl, alkenyloxy, alkynyloxy, benzyloxy, cycloalkylalkoxy, haloalkoxy, haloalkoxyalkyl, -SH, alkylsulphanyl, haloalkylsulphanyl, alkylsulphinyl, haloalkylsulphinyl, alkylsulphonyl, haloalkylsulphonyl, cyano, nitro, alkylcarbonyl, alkoxycarbonyl, alkoxycarbonylalkyl, carboxyl, carboxamide, dialkylcarboxamide, trialkylsilyl, nitro, amino, alkylamino, dialkylamino, alkylsulphonylamino, dialkylsulphonylamino, formyl, -CH=NO-H, -CH=NO-alkyl, -CH=NO-haloalkyl, -C(CH₃)=NO-H, -C(CH₃)=NO-alkyl, -C(CH₃)=NO-haloalkyl or heteroaryl, where the heteroaromatic ring may optionally be mono- or polysubstituted by one or more substituents selected independently from halogen, alkyl, haloalkyl and alkoxy;
and the N-oxides and salts of the compounds of the general formula (I).

2. Pyrazinylpyrazoles of the general formula (I) according to Claim 1, **characterized in that** the X radical is phenyl, 2-pyridyl or 3-pyridyl, each of which is substituted by one or more substituents selected from the group consisting of halogen, alkyl, haloalkyl, alkoxy, haloalkoxy, alkylsulphanyl, haloalkylsulphanyl, alkylsulphinyl, haloalkylsulphinyl, alkylsulphonyl, haloalkylsulphonyl, cyano, nitro and dialkylamino; or phenyl, 2-pyridyl and 3-pyridyl optionally substituted by one or more halogen atoms, cyano, nitro, alkyl, alkoxy or haloalkyl, where vicinal alkyl, haloalkyl and/or alkoxy groups on the phenyl substituent, 2-pyridyl substituent or 3-pyridyl substituent, together with the carbon atoms to which they are bonded, may form a five- to seven-membered cyclic system which contains 0 to 2 oxygen or nitrogen atoms, where no two oxygen atoms are directly bonded to one another, and the alkyl moiety thereof may optionally be substituted by one or more halogen atoms and/or further alkyl radicals;
the R¹ radical is hydrogen, alkyl which is optionally mono- or independently polysubstituted by alkoxy, haloalkoxy, alkylsulphanyl, haloalkylsulphanyl, alkylsulphinyl, haloalkylsulphinyl, alkylsulphonyl, haloalkylsulphonyl, alkylcarbonyl, alkoxycarbonyl, hydroxyl and/or cycloalkyl; alkenyl which is optionally mono- or independently polysubstituted by halogen, alkoxy, haloalkoxy, alkylsulphanyl, haloalkylsulphanyl, alkylsulphinyl, haloalkylsulphinyl, alkylsulphonyl, haloalkylsulphonyl, alkylcarbonyl, alkoxycarbonyl and/or cycloalkyl; cycloalkyl which is optionally mono- or independently polysubstituted by alkyl, haloalkyl and/or halogen; haloalkyl which is optionally mono- or independently polysubstituted by alkoxy, alkylsulphanyl, haloalkylsulphanyl, alkylsulphinyl, haloalkylsulphinyl, alkylsulphonyl, haloalkylsulphonyl and/or phenyl; CH=NOH, CH=NOCH₃ or CN,
the R² radical is amino and substituted amino, where the substituted amino may be mono- or independently disubstituted by alkyl, haloalkyl, cycloalkylalkyl, optionally halogen- or phenyl-substitituted alkenyl, alkynyl, heterocyclylalkyl and/or heteroarylalkyl, where the heteroaromatic ring may optionally be mono- or polysubstituted by one or more substituents selected independently from halogen, alkyl, haloalkyl and alkoxy; benzyl, where the phenyl ring in the benzyl may optionally be mono- or polysubstituted by one or more substituents selected independently from halogen, alkyl, haloalkyl and alkoxy,
the R³ and R⁴ radicals are each independently hydrogen, halogen, alkyl, cycloalkyl, haloalkyl, cyano and/or hydroxyl and
the R⁵ radical is halogen, alkyl, haloalkyl, hydroxyl, alkoxy, haloalkoxy, alkylsulphanyl, haloalkylsulphanyl, alkylsulphinyl, haloalkylsulphinyl, alkylsulphonyl, haloalkylsulphonyl, cyano, amino, alkylamino, dialkylamino, alkylsulphonylamino, dialkylsulphonylamino or heteroaryl, where the heteroaromatic ring may optionally be mono- or polysubstituted by one or more substituents selected independently from halogen, alkyl, haloalkyl and alkoxy.

3. Pyrazinylpyrazoles of the general formula (I) according to Claim 1 or 2, **characterized in that** the X radical is phenyl, 2-pyridyl or 3-pyridyl, each of which is substituted by one or more substituents selected from the group consisting of fluorine, chlorine, bromine, iodine, CF₃, methoxy, ethoxy, propanyloxy, trifluoroethoxy, methylsulphanyl, 2,2,2-trifluoroethylsulphanyl, methylsulphinyl, 2,2,2-trifluoroethylsulphinyl, methylsulphonyl, 2,2,2-trifluoroethylsulphonyl, cyano and dimethylamino; or phenyl optionally substituted by one or more halogen atoms, cyano, nitro, methyl, methoxy or CF₃, where vicinal alkyl or alkoxy groups on the phenyl substituent, together with the carbon atom to which they are bonded, may form a five- to seven-membered cyclic system which contains 1 or 2 oxygen atoms, where no two oxygen atoms are directly bonded to one another, and the alkyl moiety thereof may be substituted by one or more alkyl radicals;
the R^{l} radical is CH₃, CH₂CH₃, CH(CH₃)₂, CH₂CH₃CH₃, C(CH₃)₃, C(OCH₃)HCH₂CH₃, CH(OCH₃)₂, CH=CH₂, prop-1-en-2-yl, cyclopropyl, CF₃, CHFCH₃, CHF₂, CF₂Cl, CF₂Br, CF₂CF₃, CF₂CH₃, CF₂CF₂CF₃, CF₂CF₂H, 2-fluoropropan-2-yl, 1,1,2,2-tetrafluoro-2-methoxyethyl, CH=NOH, CH=NOCH₃ or CN,
the R² radical is amino, methylamino, dimethylamino, benzylamino, dibenzylamino, (4-chlorobenzyl)amino, bis(4-chlorobenzyl)amino, (4-methoxybenzyl)amino, bis(4-methoxybenzyl)amino, (2-methylprop-2-en-1-yl)amino, prop-2-en-1-ylamino, diprop-2-en-1-ylamino, bis(2-methylprop-2-en-1-yl)amino, prop-2-yn-1-ylamino, bis(prop-2-yn-1-yl)amino, (pyrazin-2-ylmethyl)amino, (6-methylpyridin-2-ylmethyl)amino, bis(6-methyl-pyridin-2-ylmethyl)amino or (pyridin-2-ylmethyl)amino;
the R³ and R⁴ radicals are each independently hydrogen, chlorine and/or methyl and
the R⁵ radical is chlorine, bromine, methyl, CF₃, methoxy, ethoxy, propoxy, propan-2-yloxy, dimethylamino, cyano, methylsulphanyl, methylsulphinyl, methylsulphonyl, 1H-pyrazol-1-yl, 1H-imidazol-1-yl or 4-fluoro-1H-pyrazol-l-yl.

4. Process for preparing pyrazinylpyrazoles of the general formula (I) according to any of Claims 1 to 3, **characterized by** the following process steps:
(a) condensation of keto nitriles or of the tautomers or hydrates thereof, of the formulae (IIA), (IIB) and/or (IIC), with pyrazinylhydrazines of the general formula (III) according to the following reaction scheme: 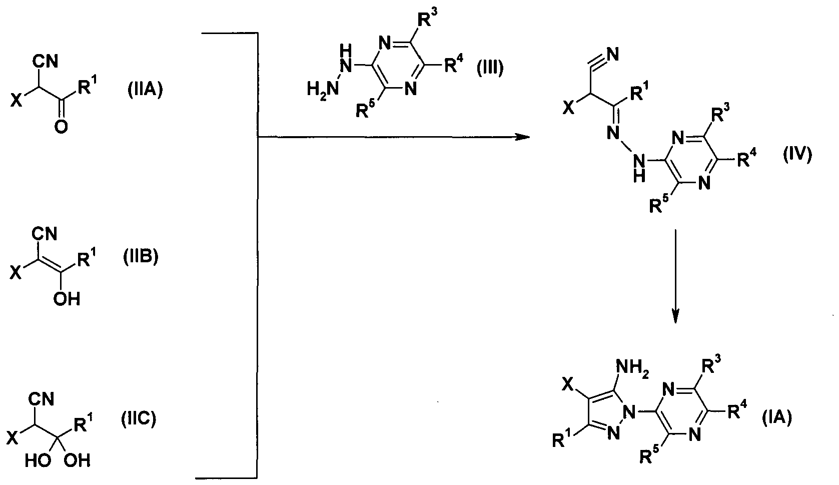 where X, R¹, R³, R⁴ and R⁵ are each as defined according to one of Claims 1 to 3;
(b) reaction of 1H-aminopyrazoles of the general formula (V) with pyrazinyl halides and/or pyrazinyl alkyl sulphones of the general formula (VI) in the presence of a base in organic solvents according to the following reaction scheme: 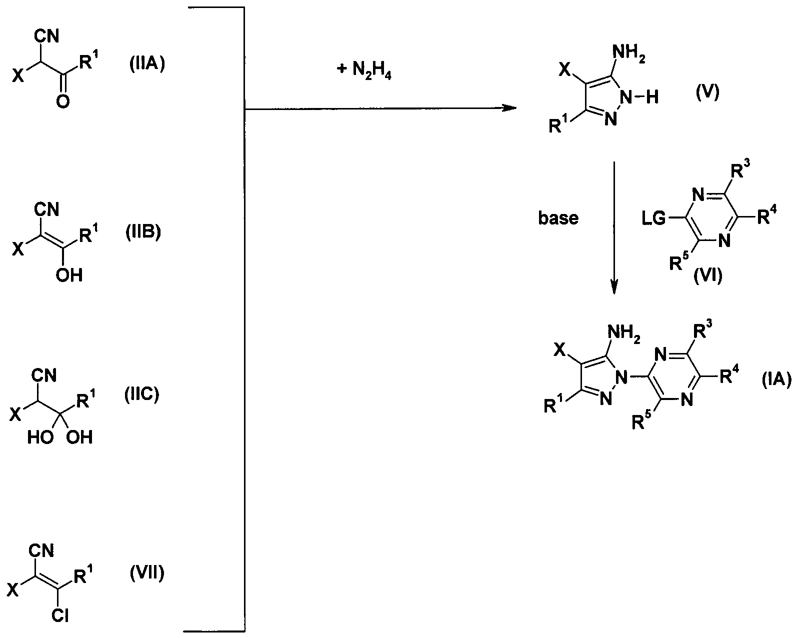 where X, R¹, R³, R⁴ and R⁵ are each as defined according to one of Claims 1 to 3 and LG is halogen or an alkylsulphonyl;
(c) reactions of keto nitriles or of the tautomers or hydrates thereof, of the general formulae (IIA), (IIB) or (IIC), with chlorinating agents, optionally diluted in an inert organic solvent or promoted by auxiliary bases such as nitrogen bases, to give chloroacrylonitriles (VII) 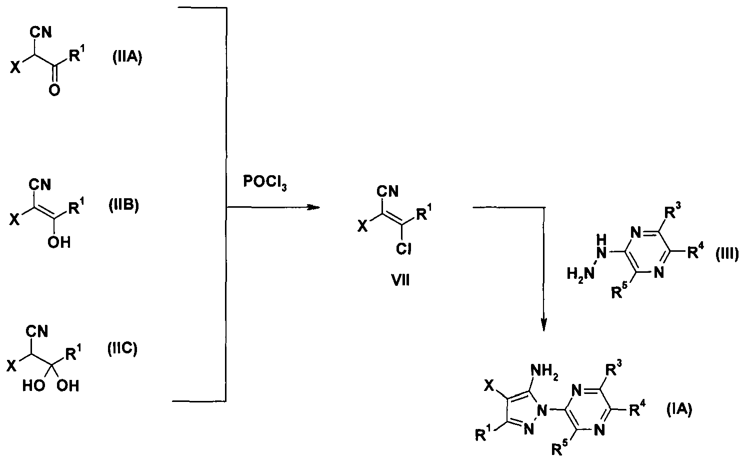 where X, R¹, R³, R⁴ and R⁵ are each as defined according to one of Claims 1 to 3;
(d) reaction of compounds of the general formula (IA) according to the following reaction scheme with one or two alkylating agents or acylating agents R⁶-LG and/or R⁷-LG to obtain compounds of the general formula (IB) and (IC) in which at least one radical of R⁶ and R⁷ is not hydrogen: 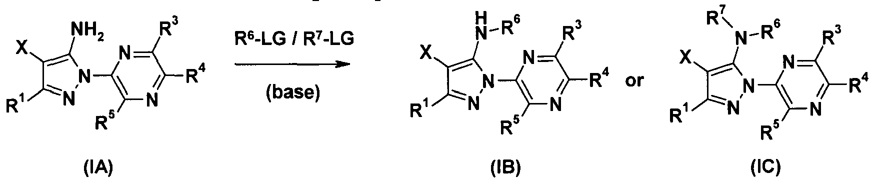 where
X, R¹, R³, R⁴ and R⁵ are each as defined according to one of Claims 1 to 3;
LG is halogen or alkylsulphonyl; and
R⁶ and R⁷ are each independently hydrogen, alkyl, haloalkyl, alkoxyalkyl, alkylsulphanylalkyl, alkylsulphinylalkyl, alkylsulphonylalkyl, alkylcarbonyl, cycloalkyl, cycloalkylalkyl, alkenyl, where the above radicals are optionally substituted by halogen, cyano, alkoxy, alkoxycarbonyl and phenyl, where the phenyl ring is optionally mono- or polysubstituted by one or more substituents selected independently from halogen, alkyl, haloalkyl and alkoxy; alkynyl, alkoxycarbonyl, alkenyloxycarbonyl, alkynyloxycarbonyl, alkoxycarbonylalkyl, alkoxycarbonylcarbonyl, heterocyclyl, heteroaryl, heterocyclylalkyl or heteroarylalkyl, where the heterocyclic or heteroaromatic ring may optionally be mono- or polysubstituted by one or more substituents selected independently from halogen, alkyl, haloalkyl and alkoxy; benzyl or phenylcarbonyl, where the phenyl ring in the benzyl and phenylcarbonyl is optionally mono- or polysubstituted by one or more substituents selected independently from halogen, alkyl, haloalkyl and alkoxy; and
with the proviso that at least one radical of R⁶ and R⁷ is not hydrogen; or
(e) reaction of bromides or iodides of the general formula (VII) with boronic acids or boronic esters of the formula (IX) in the presence of suitable palladium catalysts and bases within a temperature range from -20°C to 120°C according to the following reaction scheme: 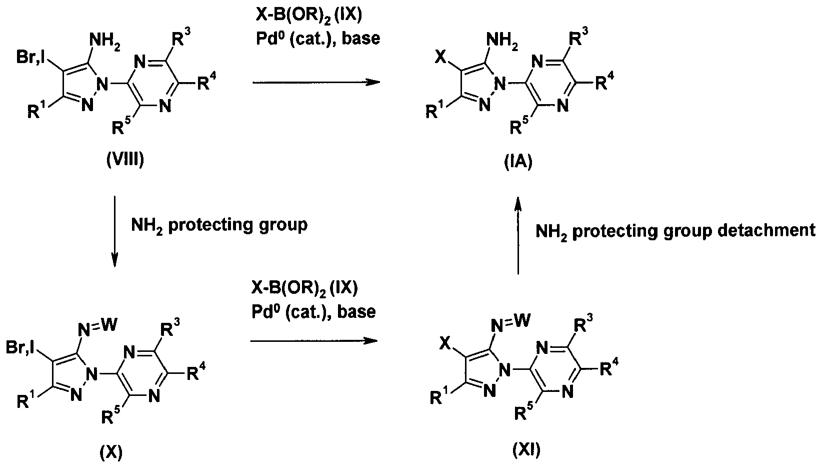
where X, R¹, R³, R⁴ and R⁵ are each as defined according to one of Claims 1 to 3 and W is a protecting group.

5. Compositions comprising at least one compound of the general formula (I) according to any of Claims 1 to 3.

6. Use of compounds of the formula (I) according to any of Claims 1 to 3 or of the composition according to Claim 5 for controlling animal pests in agriculture, in horticulture, in forests, in gardens and leisure facilities, in the protection of stored products and of materials.

7. Method for controlling animal pests, **characterized in that** compounds of the formula (I) according to any of Claims 1 to 3 or compositions according to Claim 5 are allowed to act on seed.

8. Process for producing compositions, **characterized in that** compounds of the formula (I) according to any of Claims 1 to 5 are mixed with extenders and/or surfactants.

9. Compounds of the formula (I) according to any of Claims 1 to 3 or compositions according to Claim 5 for use in animal husbandry or the hygiene sector.

10. Compounds of the formula (I) according to any of Claims 1 to 3 or compositions according to Claim 5 for use as a medicament.

## Revendications

1. Pyrazinylpyrazoles de formule générale (I) 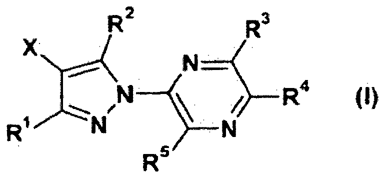 dans laquelle
X représente phényle, 2-pyridyle ou 3-pyridyle, qui sont à chaque fois substitués par un ou plusieurs substituants choisis dans le groupe constitué par halogène, alkyle, haloalkyle, alcoxy, alcoxyalkyle, alcoxyalcoxy, cycloalkyle, alcényloxy, alcynyloxy, benzyloxy, cycloalkylalcoxy, haloalcoxy, haloalcoxyalkyle, alkylsulfanyle, haloalkylsulfanyle, alkylsulfinyle, haloalkylsulfinyle, alkylsulfonyle, haloalkylsulfonyle, cyano, nitro, alkylcarbonyle, alcoxycarbonyle, alcoxycarbonylalkyle, carboxyle, carboxamide, dialkylcarboxamide, trialkylsilyle, amino, alkylamino, dialkylamino, alkylsulfonylamino, dialkylsulfonylamino, formyle, -CH=NO-H, -CH=NO-alkyle, -CH=NO-haloalkyle, - C(CH₃)=NO-H, -C(CH₃)=NO-alkyle, -C(CH₃)=NO-haloalkyle ; ou phényle, 2-pyridyle et 3-pyridyle éventuellement substitués avec un ou plusieurs atomes d'halogène, cyano, nitro, alkyle, alcoxy ou haloalkyle, des groupes alkyle, haloalkyle, alcoxy et/ou haloalcoxy vicinaux sur le substituant phényle, le substituant 2-pyridyle ou le substituant 3-pyridyle pouvant former ensemble avec les atomes de carbone auxquels ils sont reliés un système cyclique de cinq à sept éléments, qui contient 0 à 2 atomes d'oxygène ou d'azote, deux atomes d'oxygène n'étant pas reliés directement l'un à l'autre, et dont la partie alkyle peut éventuellement être substituée avec un ou plusieurs atomes d'halogène et/ou autres radicaux alkyle ;
R¹ représente hydrogène, alkyle, qui est éventuellement substitué une ou plusieurs fois indépendamment avec alcoxy, haloalcoxy, alkylsulfanyle, haloalkylsulfanyle, alkylsulfinyle, haloalkylsulfinyle, alkylsulfonyle, haloalkylsulfonyle, alkylcarbonyle, alcoxycarbonyle, hydroxy et/ou cycloalkyle ; alcényle, qui est éventuellement substitué une ou plusieurs fois indépendamment avec halogène, alcoxy, haloalcoxy, alkylsulfanyle, haloalkylsulfanyle, alkylsulfinyle, haloalkylsulfinyle, alkylsulfonyle, haloalkylsulfonyle, alkylcarbonyle, alcoxycarbonyle et/ou cycloalkyle ; cycloalkyle, qui est éventuellement substitué une ou plusieurs fois indépendamment avec alkyle, haloalkyle et/ou halogène ; haloalkyle, qui est éventuellement substitué une ou plusieurs fois indépendamment avec alcoxy, alkylsulfanyle, haloalkylsulfanyle, alkylsulfinyle, haloalkylsulfinyle, alkylsulfonyle, haloalkylsulfonyle et/ou phényle, éventuellement substitué une ou plusieurs fois indépendamment avec halogène, alkyle, haloalkyle et/ou alcoxy ; phényle, qui est éventuellement substitué une ou plusieurs fois indépendamment avec halogène, alkyle, haloalkyle et/ou alcoxy ; benzyle, qui est éventuellement substitué une ou plusieurs fois indépendamment avec halogène, alkyle, haloalkyle et/ou alcoxy ; cyano, formyle, alkylcarbonyle, -CH=NO-H, -CH=NO-alkyle, -CH=NO-haloalkyle, -C(CH₃)=NO-H, -C(CH₃)=NO-alkyle ou -C(CH₃)=NO-haloalkyle ;
R² représente amino éventuellement substitué, amino pouvant être substitué une ou deux fois indépendamment avec alkyle, haloalkyle, alcoxyalkyle, alkylsulfanylalkyle, alkylsulfinylalkyle, alkylsulfonylalkyle, alkylcarbonyle, cycloalkyle, cycloalkylalkyle, alcényle, les radicaux précédents étant éventuellement substitués par halogène, cyano, alcoxy, alcoxycarbonyle ou phényle, le cycle phényle étant éventuellement substitué une ou plusieurs fois avec un ou plusieurs substituants choisis indépendamment les uns des autres parmi halogène, alkyle, haloalkyle et alcoxy ; alcynyle, alcoxycarbonyle, alcényloxycarbonyle, alcynyloxycarbonyle, alcoxycarbonylalkyle, alcoxycarbonylcarbonyle, hétérocyclyle, hétéroaryle, hétérocyclylalkyle ou hétéroarylalkyle, le cycle hétérocyclique ou hétéroaromatique pouvant éventuellement être substitué une ou plusieurs fois avec un ou plusieurs substituants choisis parmi halogène, alkyle, haloalkyle et alcoxy ; benzyle ou phénylcarbonyle, le cycle phényle dans le benzyle et le phénylcarbonyle étant éventuellement substitué une ou plusieurs fois avec un ou plusieurs substituants choisis indépendamment les uns des autres parmi halogène, alkyle, haloalkyle et alcoxy ;
et
R³, R⁴ représentent indépendamment l'un de l'autre hydrogène, halogène, alkyle, cycloalkyle, haloalkyle, cyano, hydroxy, formyle, alkylcarbonyle, -CH=NO-H, -CH-NO-alkyle, -CH=NO-haloalkyle, -C(CH₃)=NO-H, -C(CH₃)=NO-alkyle, -C(CH₃)=NO-haloalkyle, nitro, hydroxy, SH, alcoxy, alkylsulfanyle, haloalkylsulfanyle, alkylsulfinyle, haloalkylsulfinyle, alkylsulfonyle ou haloalkylsulfonyle ;
R⁵ représente halogène, alkyle, haloalkyle, hydroxy, alcoxy, alcoxyalkyle, alcoxyalcoxy, cycloalkyle, alcényloxy, alcynyloxy, benzyloxy, cycloalkylalcoxy, haloalcoxy, haloalcoxyalkyle, -SH, alkylsulfanyle, haloalkylsulfanyle, alkylsulfinyle, haloalkylsulfinyle, alkylsulfonyle, haloalkylsulfonyle, cyano, nitro, alkylcarbonyle, alcoxycarbonyle, alcoxycarbonylalkyle, carboxyle, carboxamide, dialkylcarboxamide, trialkylsilyle, nitro, amino, alkylamino, dialkylamino, alkylsulfonylamino, dialkylsulfonylamino, formyle, - CH=NO-H, -CH=NO-alkyle, -CH=NO-haloalkyle, -C(CH₃)=NO-H, -C(CH₃)=NO-alkyle, -C(CH₃)=NO-haloalkyle ou hétéroaryle, le cycle hétéroaromatique pouvant éventuellement être substitué une ou plusieurs fois avec un ou plusieurs substituants choisis indépendamment les uns des autres parmi halogène, alkyle, haloalkyle et alcoxy ;
ainsi que les N-oxydes et les sels des composés de formule générale (I).

2. Pyrazinylpyrazoles de formule générale (I) selon la revendication 1, **caractérisés en ce que** le radical X représente phényle, 2-pyridyle ou 3-pyridyle, qui sont à chaque fois substitués par un ou plusieurs substituants choisis dans le groupe constitué par halogène, alkyle, haloalkyle, alcoxy, haloalcoxy, alkylsulfanyle, haloalkylsulfanyle, alkylsulfinyle, haloalkylsulfinyle, alkylsulfonyle, haloalkylsulfonyle, cyano, nitro et dialkylamino ; ou phényle, 2-pyridyle et 3-pyridyle éventuellement substitués avec un ou plusieurs atomes d'halogène, cyano, nitro, alkyle, alcoxy ou haloalkyle, des groupes alkyle, haloalkyle, et/ou alcoxy vicinaux sur le substituant phényle, le substituant 2-pyridyle ou le substituant 3-pyridyle pouvant former ensemble avec les atomes de carbone auxquels ils sont reliés un système cyclique de cinq à sept éléments, qui contient 0 à 2 atomes d'oxygène ou d'azote, deux atomes d'oxygène n'étant pas reliés directement l'un à l'autre, et dont la partie alkyle peut éventuellement être substituée avec un ou plusieurs atomes d'halogène et/ou autres radicaux alkyle ;
le radical R¹ représente hydrogène, alkyle, qui est éventuellement substitué une ou plusieurs fois indépendamment avec alcoxy, haloalcoxy, alkylsulfanyle, haloalkylsulfanyle, alkylsulfinyle, haloalkylsulfinyle, alkylsulfonyle, haloalkylsulfonyle, alkylcarbonyle, alcoxycarbonyle, hydroxy et/ou cycloalkyle ; alcényle, qui est éventuellement substitué une ou plusieurs fois indépendamment avec halogène, alcoxy, haloalcoxy, alkylsulfanyle, haloalkylsulfanyle, alkylsulfinyle, haloalkylsulfinyle, alkylsulfonyle, haloalkylsulfonyle, alkylcarbonyle, alcoxycarbonyle et/ou cycloalkyle ; cycloalkyle, qui est éventuellement substitué une ou plusieurs fois indépendamment avec alkyle, haloalkyle et/ou halogène ; haloalkyle, qui est éventuellement substitué une ou plusieurs fois indépendamment avec alcoxy, alkylsulfanyle, haloalkylsulfanyle, alkylsulfinyle, haloalkylsulfinyle, alkylsulfonyle, haloalkylsulfonyle et/ou phényle ; CH=NOH, CH=NOCH₃ ou CN,
le radical R² représente amino et amino substitué, l'amino substitué pouvant être substitué une ou deux fois indépendamment avec alkyle, haloalkyle, cycloalkylalkyle, alcényle éventuellement substitué par halogène ou phényle, alcynyle, hétérocyclylalkyle et/ou hétéroarylalkyle, le cycle hétéroaromatique pouvant éventuellement être substitué une ou plusieurs fois avec un ou plusieurs substituants choisis indépendamment les uns des autres parmi halogène, alkyle, haloalkyle et alcoxy ; benzyle, le cycle phényle dans le benzyle pouvant éventuellement être substitué une ou plusieurs fois avec un ou plusieurs substituants choisis indépendamment les uns des autres parmi halogène, alkyle, haloalkyle et alcoxy ;
les radicaux R³ et R⁴ représentent indépendamment l'un de l'autre hydrogène, halogène, alkyle, cycloalkyle, haloalkyle, cyano et/ou hydroxy et
le radical R⁵ représente halogène, alkyle, haloalkyle, hydroxy, alcoxy, haloalcoxy, alkylsulfanyle, haloalkylsulfanyle, alkylsulfinyle, haloalkylsulfinyle, alkylsulfonyle, haloalkylsulfonyle, cyano, amino, alkylamino, dialkylamino, alkylsulfonylamino, dialkylsulfonylamino ou hétéroaryle, le cycle hétéroaromatique pouvant éventuellement être substitué une ou plusieurs fois avec un ou plusieurs substituants choisis indépendamment les uns des autres parmi halogène, alkyle, haloalkyle et alcoxy.

3. Pyrazinylpyrazoles de formule générale (I) selon la revendication 1 ou 2, **caractérisé en ce que** le radical X représente phényle, 2-pyridyle ou 3-pyridyle, qui sont à chaque fois substitués par un ou plusieurs substituants choisis dans le groupe constitué par fluor, chlore, brome, iode, CF₃, méthoxy, éthoxy, propanyloxy, trifluoroéthoxy, méthylsulfanyle, 2,2,2-trifluoroéthylsulfanyle, méthylsulfinyle, 2,2,2-trifluoroéthylsulfinyle, méthylsulfonyle, 2,2,2-trifluoroéthylsulfonyle, cyano et diméthylamino ; ou phényle éventuellement substitué avec un ou plusieurs atomes d'halogène, cyano, nitro, méthyle, méthoxy ou CF₃, des groupes alkyle ou alcoxy vicinaux sur le substituant phényle pouvant former ensemble avec les atomes de carbone auxquels ils sont reliés un système cyclique de cinq à sept éléments, qui contient 1 ou 2 atomes d'oxygène, deux atomes d'oxygène n'étant pas reliés directement l'un à l'autre, et dont la partie alkyle peut être substituée avec un ou plusieurs autres radicaux alkyle ;
le radical R¹ représente CH₃, CH₂CH₃, CH(CH₃)₂, CH₂CH₃CH₃, C(CH₃)₃, C(OCH₃)HCH₂CH₃, CH(OCH₃)₂, CH=CH₂, prop-1-én-2-yle, cyclopropyle, CF₃, CHFCH₃, CHF₂, CF₂Cl, CF₂Br, CF₂CF₃, CF₂CH₃, CF₂CF₂CF₃, CF₂CF₂H, 2-fluoropropan-2-yle, 1,1,2,2-tétrafluoro-2-méthoxyéthyle, CH=NOH, CH=NOCH₃ ou CN,
le radical R² représente amino, méthylamino, diméthylamino, benzylamino, dibenzylamino, (4-chlorobenzyl)amino, bis(4-chlorobenzyl)amino, (4-méthoxybenzyl)amino, bis(4-méthoxybenzyl)amino, (2-méthylprop-2-én-1-yl)amino, prop-2-én-1-ylamino, diprop-2-én-1-ylamino, bis(2-méthylprop-2-én-1-yl)amino, prop-2-yn-l-ylamino, bis(prop-2-yn-1-yl)amino, (pyrazin-2-ylméthyl)amino, (6-méthyl-pyridin-2-ylméthyl)amino, bis(6-méthyl-pyridin-2-ylméthyl)amino ou (pyridin-2-ylméthyl)amino ;
les radicaux R³ et R⁴ représentent indépendamment l'un de l'autre hydrogène, chlore et/ou méthyle, et
le radical R⁵ représente chlore, brome, méthyle, CF₃, méthoxy, éthoxy, propoxy, propan-2-yloxy, diméthylamino, cyano, méthylsulfanyle, méthylsulfinyle, méthylsulfonyle, 1H-pyrazol-1-yl, 1H-imidazol-1-yle ou 4-fluoro-1H-pyrazol-1-yle.

4. Procédé de fabrication de pyrazinylpyrazoles de formule générale (I) selon l'une quelconque des revendications 1 à 3, **caractérisé par** les étapes de procédé suivantes :
(a) la condensation de cétonitriles, leurs tautomères ou leurs hydrates des formules (IIA), (IIB) et/ou (IIC) avec des pyrazinyl-hydrazines de formule générale (III) selon le schéma réactionnel suivant : 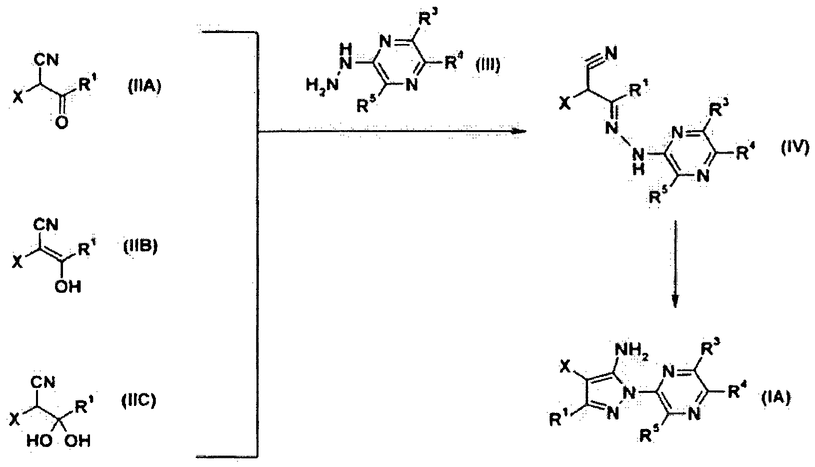 dans lequel X, R¹, R³, R⁴ et R⁵ ont les significations selon l'une quelconque des revendications 1 à 3 ;
(b) la mise en réaction de 1-H-aminopyrazoles de formule générale (V) avec des halogénures de pyrazinyle et/ou des pyrazinyl-alkylsulfones de formule générale (VI) en présence d'une base dans des solvants organiques selon le schéma réactionnel suivant : 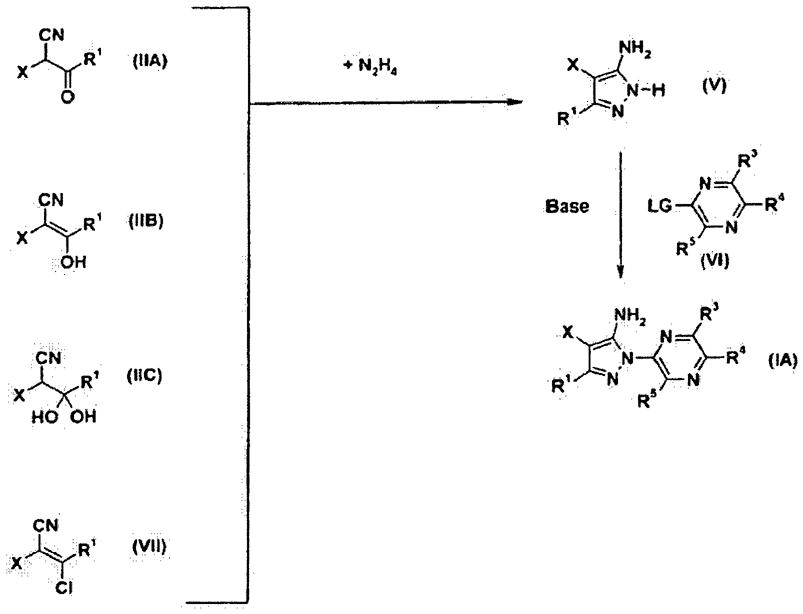 dans lequel X, R¹, R³, R⁴ et R⁵ ont les significations selon l'une quelconque des revendications 1 à 3 et LG représente halogène ou un alkylsulfonyle ;
(c) la mise en réaction de cétonitriles, leurs tautomères ou leurs hydrates des formules générales (IIA), (IIB) ou (IIC) avec des agents de chloration, éventuellement dilués dans un solvant organique inerte ou assistés par des bases auxiliaires telles que des bases azotées, pour former des chloroacrylonitriles (VII) 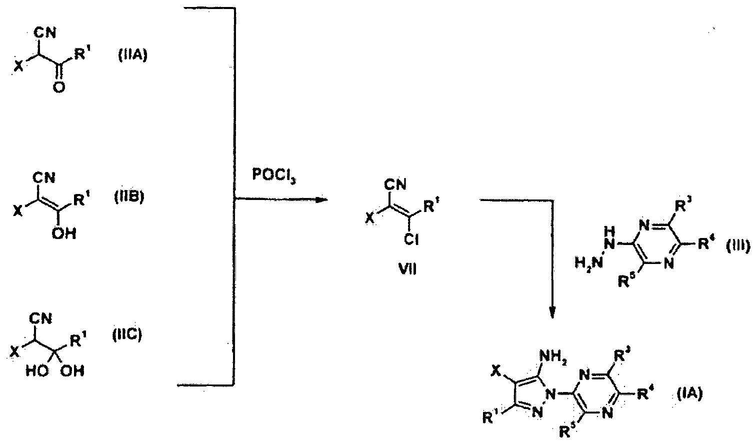 X, R¹, R³, R⁴ et R⁵ ayant les significations selon l'une quelconque des revendications 1 à 3 ;
(d) la mise en réaction de composés de formule générale (IA) selon le schéma de réaction suivant avec un ou deux agents d'alkylation ou agents d'acylation R⁶-LG et/ou R⁷-LG pour obtenir des composés de formule générale (IB) et (IC), dans lesquelles au moins un radical parmi R⁶ et R⁷ ne représente pas l'hydrogène ; 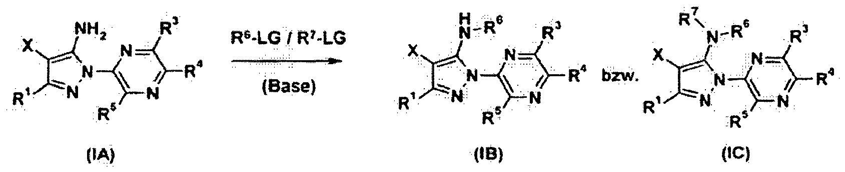 dans lequel
X, R¹, R³, R⁴ et R⁵ ont les significations selon l'une quelconque des revendications 1 à 3 ;
LG représente halogène ou alkylsulfonyle ; et
R⁶ et R⁷ représentent, à chaque fois indépendamment l'un de l'autre hydrogène, alkyle, haloalkyle, alcoxyalkyle, alkylsulfanylalkyle, alkylsulfinylalkyle, alkylsulfonylalkyle, alkylcarbonyle, cycloalkyle, cycloalkylalkyle, alcényle, les radicaux précédents étant éventuellement substitués par halogène, cyano, alcoxy, alcoxycarbonyle et phényle, le cycle phényle étant éventuellement substitué une ou plusieurs fois avec un ou plusieurs substituants choisis indépendamment les uns des autres parmi halogène, alkyle, haloalkyle et alcoxy ; alcynyle, alcoxycarbonyle, alcényloxycarbonyle, alcynyloxycarbonyle, alcoxycarbonylalkyle, alcoxycarbonylcarbonyle, hétérocyclyle, hétéroaryle, hétérocyclylalkyle ou hétéroarylalkyle, le cycle hétérocyclique ou hétéroaromatique pouvant éventuellement être substitué une ou plusieurs fois avec un ou plusieurs substituants choisis indépendamment les uns des autres parmi halogène, alkyle, haloalkyle et alcoxy ; benzyle ou phénylcarbonyle, le cycle phényle dans le benzyle et le phénylcarbonyle étant éventuellement substitué une ou plusieurs fois avec un ou plusieurs substituants choisis indépendamment les uns des autres parmi halogène, alkyle, haloalkyle et alcoxy ;
à condition qu'au moins un radical parmi R⁶ et R⁷ soit différent de l'hydrogène ; ou
(e) la mise en réaction de bromures ou d'iodures de formule générale (VII) avec des acides boroniques ou des esters d'acides boroniques de formule (IX) en présence de catalyseurs de palladium appropriés et de bases dans une plage de température allant de -20 °C à 120 °C selon le schéma réactionnel suivant : 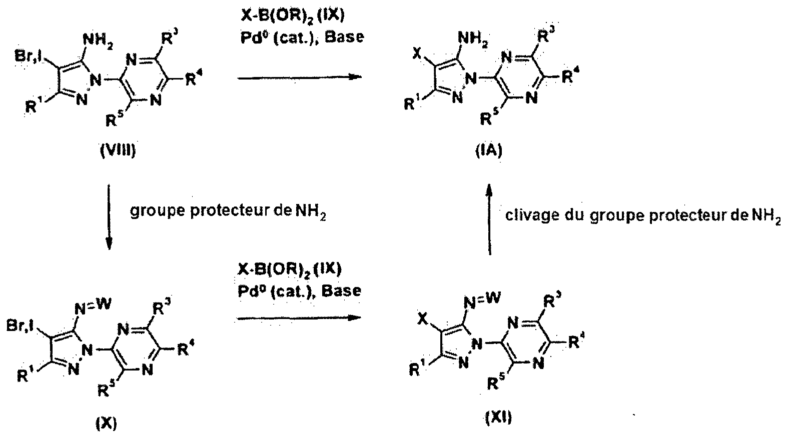
dans lequel X, R¹, R³, R⁴ et R⁵ ont les significations selon l'une quelconque des revendications 1 à 3 et W correspond à un groupe protecteur.

5. Agent contenant au moins un composé de formule générale (I) selon l'une quelconque des revendications 1 à 3.

6. Utilisation de composés de formule (I) selon l'une quelconque des revendications 1 à 3 ou de l'agent selon la revendication 5 pour lutter contre les animaux nuisibles dans l'agriculture, dans l'horticulture, dans les forêts, dans les jardins et dans les installations de loisirs, pour la protection des stocks et des matériaux.

7. Procédé de lutte contre les animaux nuisibles, **caractérisé en ce que** des composés de formule (I) selon l'une quelconque des revendications 1 à 3 ou des agents selon la revendication 5 sont laissés agir sur des semences.

8. Procédé de fabrication d'agents, **caractérisé en ce que** des composés de formule (I) selon l'une quelconque des revendications 1 à 5 sont mélangés avec des extendeurs et/ou des substances tensioactives.

9. Composés de formule (I) selon l'une quelconque des revendications 1 à 3 ou agent selon la revendication 5, destinés à être utilisés dans la production animale ou dans le secteur de l'hygiène.

10. Composés de formule (I) selon l'une quelconque des revendications 1 à 3 ou agent selon la revendication 5, destinés à être utilisés en tant que médicament.
